(19) Europäisches Patentamt
European Patent Office
Office européen des brevets

(11) Veröffentlichungsnummer: **0 640 562 A2**

# (12) EUROPÄISCHE PATENTANMELDUNG

(21) Anmeldenummer: **94113096.5**

(22) Anmeldetag: **23.08.94**

(51) Int. Cl.⁶: **C01B 31/00**, C07F 5/02, C07D 487/22

(30) Priorität: **25.08.93 DE 4328534**
**06.09.93 DE 4330028**
**28.06.94 DE 4422431**

(43) Veröffentlichungstag der Anmeldung:
**01.03.95 Patentblatt 95/09**

(84) Benannte Vertragsstaaten:
**CH DE FR GB LI**

(71) Anmelder: **Oeste, Franz-Dietrich**
**Tannenweg 2**
**D-35274 Kirchhain-Schönbach (DE)**

(72) Erfinder: **Oeste, Franz-Dietrich**
**Tannenweg 2**
**D-35274 Kirchhain-Schönbach (DE)**

(74) Vertreter: **Schlagwein, Udo, Dipl.-Ing.**
**Patentanwalt,**
**Frankfurter Strasse 34**
**D-61231 Bad Nauheim (DE)**

## (54) Spheren, Verfahren zu ihrer Herstellung und Anwendungen.

(57) Spheren genannte Fulleren- und Heterofullerenfragmente, die dadurch gekennzeichnet sind, daß sie in ihrem Molekül mittels Hexagonnetzwerk anelliert verbundene Funktionen triangularer Symmetrie und/oder quadratischer Symmetrie und/oder pentagonaler Symmetrie entsprechend Formel 55) enthalten, Verfahren zu ihrer Herstellung und Anwendungen.

EP 0 640 562 A2

55)

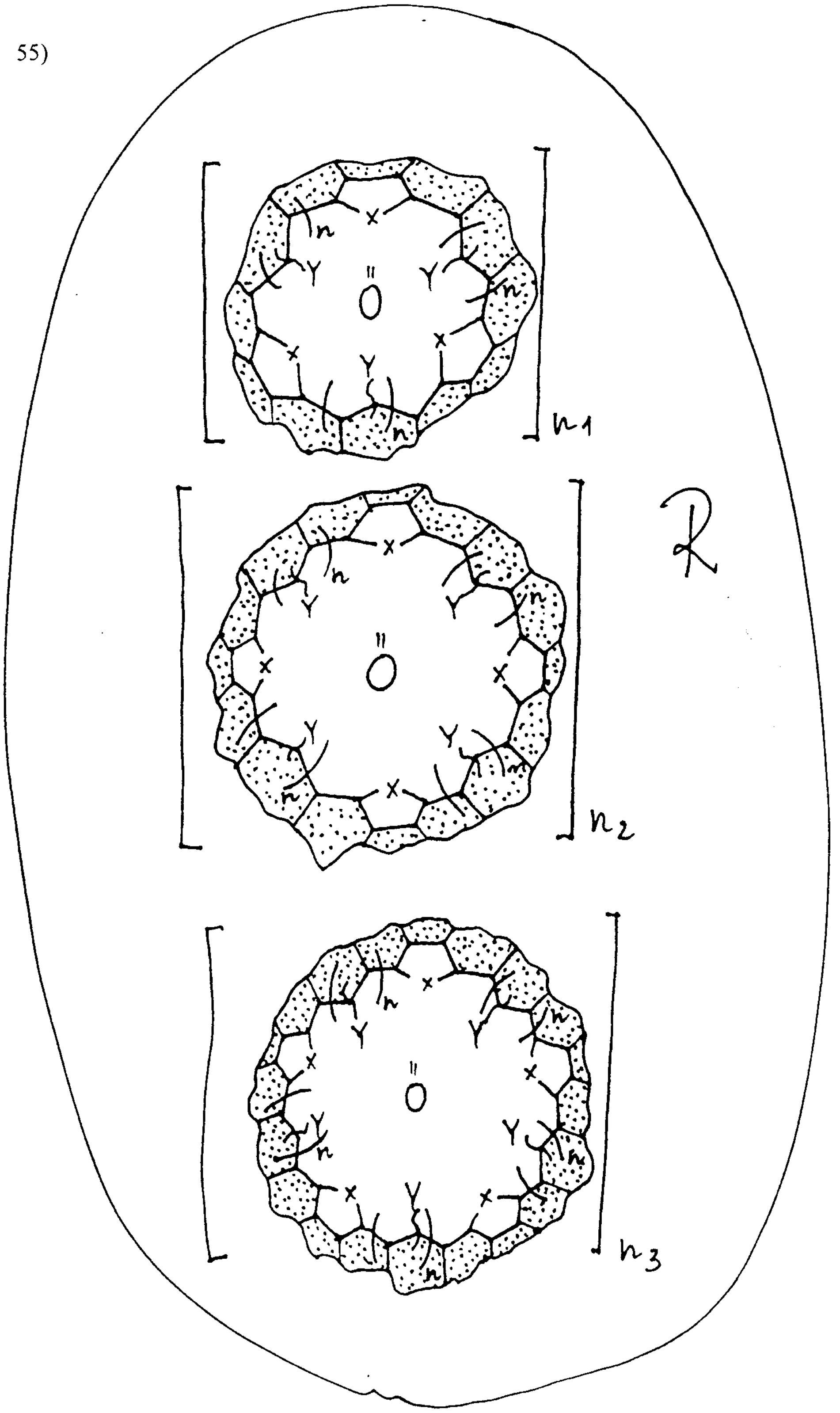

Durch Kondensation von Kohlenstoffdampf oder bei der unvollständigen Verbrennung von organischen Kohlenstoffverbindungen entstehen Fullerene, wie aus der Veröffentlichung in der Zeitschrift Nature Vol. 347 vom 27.9.1990, Seiten 354 ff und Vol. 352 vom 11.7.1991 Seiten, 139 ff hervorgeht.

Fullerene sind stabile sphärische Hohlmoleküle aus Kohlenstoffatomen, deren Schale von einem Netzwerk gebildet wird, das aus Hexagon- und Pentagonzyklen besteht, die aus dreifach koordinierten Kohlenstoffatomen aufgebaut sind.

In der DOS 41 28 357.0 und der DOS 41 14 536.4 werden überdies Heterofullerene beschrieben, die neben Kohlenstoff Bor und Stickstoff enthalten und wobei alle beteiligten Atome dreifach koordiniert in Hexagon- und Pentagonzyklen angeordnet sind.

Anfangs wurden hohe Erwartungen an die Möglichkeiten zur vielseitigen Anwendbarkeit von chemisch modifizierten Fullerenen gestellt. Es hat sich nun aber gezeigt, daß die Bindungen zwischen Fullerenen und den an sie gebundenen Resten in vielen Fällen sehr instabil sind. Das ist z.B. der Fall, wenn Fluor, Chlor, Brom, Phenyl oder Wasserstoff an das Fulleren gebunden sind. Diese Reste werden leicht wieder unter Rückbildung der Fullerene abgespalten, wie aus den Veröffentlichungen in der Zeitschrift Journal of the American Chemical Societey, Nr. 113, Jahrgang 1991, Seiten 9900 und 9901 sowie 9385 bis 9387 hervorgeht. In jedem Fall verliert das Atom des Fullerens, an dem eine Bindung geknüpft wird, seine elektrisch leitende Elektrokonfiguration, weil es dabei aus dem dreifach koordinierten Zustand in den vierfach koordinierten Zustand übergeht.

Nach einer neuerlich bekannt gewordenen Methode zur chemischen Modifikation der Fullerene wird die Fullerenstruktur aufgebrochen, um an den entstehenden Rändern organische Reste anzuknüpfen. Bislang ist es nach dieser Methode aber lediglich gelungen, vierfach koordinierte Kohlenstoffunktionen als Brückenfunktionen an dem Fulleren anzubinden, wie in der Zeitschrift C&EN vom 16.12.1991, Seiten 17 bis 20 berichtet wird. Mit dieser Methode ist es auch gelungen, zwei Fullerene miteinander mittels einer p-Xylyl-Brückenfunktion miteinander zu verknüpfen. Bislang ist die Herstellung von Öffnungen nur bei "leeren" Fullerenen gelungen. Kondensate aus metall- und kohlenstoffdampfhaltigen Atmosphären enthalten sog. endohedrale Metallofullerene. Diese enthalten in dem Hohlraum, den jedes Fullerenmolekül umschließt, ein oder zwei Metallatome. Bislang wurden endohedrale Yttrium- und Lanthanfullerene hergestellt. Hierüber wird ebenfalls in der Zeitschrift C&EN in der o.g. Ausgabe berichtet.

In der DOS 41 14 536.4 werden Spheren genannte Fulleren- und Heterofullerenfragmente beschrieben. Die Spheren haben analoge symmetrische Eigenschaften wie die betreffenden Fullerene. Im Gegensatz zu den Fullerenen enthalten sie symmetrisch verteilt Ausnehmungen in dem Hexagon-/Pentagonnetzwerk, wobei mindestens die Pentagone bzw. der Bereich des Netzwerkes um die Pentagone von den Ausnehmungen betroffen ist. Die Spheren sind außerdem dadurch gekennzeichnet, daß sie an mindestens 4 Ausnehmungen und höchstens 12 Ausnehmungen mit Pyrrolringen dekoriert sind, die dort eine geometrische Anordnung einnehmen, die derjenigen Anordnung der Pyrrolringe im Subphthalocyanin oder im Phthalocyanin oder im Superphthalocyanin entspricht.

Die in dieser Offenlegung beschriebenen Spheren lassen sich bezüglich ihrer Symmetrie und topographischen Eigenschaften wie Durchmesser und Inhalt auf 6 Fullerenklassen zurückfuhren:

1. ikosaedersymmetrische Fullerene mit isolierten Pentagonen mit Kohlenstoffzahlen x, die der Formel 0.1) entsprechen, wobei n auf die Zahlen 4, 5, 6, 7 und 8 beschränkt ist.

0.1) $x = 20 + 60n + 40 (n(n-1)/2)$

2. ikosaedersymmetrische Fullerene mit isolierten Pentagonen mit Kohlenstoffzahlen x, die der Formel 0.2) entsprechen, wobei n auf die Zahlen 2, 3 und 4 beschränkt ist.

0.2) $x = 60 + 180n + 120 (n(n - 1)/2)$

3. oktaedersymmetrische Fullerene mit isolierten Pentagonpaaren mit Kohlenstoffzahlen x, die der Formel 0.3) entsprechen, wobei n auf die Zahlen 2, 3, 4, 5 und 6 beschränkt ist.

0.3) $x = 60 + 56n + 16 (n(n - 1)/2)$

4. oktaedersymmetrische Fullerene mit isolierten Pentagonpaaren mit Kohlenstoffzahlen x, die der Formel 0.4) entsprechen, wobei n auf die Zahlen 1, 2 und 3 beschränkt ist.

0.4) $x = 84 + 120n + 48 (n(n - 1)/2)$

5. tetraedersymmetrische Fullerene mit isolierten Pentagontrimeren mit Kohlenstoffzahlen x, die der Formel 0.5), wobei n auf die Zahlen 0, 1, 2, 3 und 4 beschränkt ist.

0.5) $x = 68 + 44n + 8 (n(n - 1)/2)$

6. tetraedersymmetrische Fullerene mit isolierten Pentagontrimeron mit Kohlenstoffzahlen x, die der Formel 0.6) entsprechen, wobei n auf die Zahlen 0, 1 und 2 beschränkt ist.

0.6) $x = 76 + 84n + 24 (n(n - 1)/2)$

In der Offenlegungsschrift wird berichtet, daß die Spheren vielseitige chemische Modifikationsmöglichkeiten bieten, weitaus mehr, als bei den Fullerenen möglich ist. Davon ganz abgesehen haben die Spheren den Vorteil, chemisch weitaus stabiler bzw. beständiger zu sein, als die Fullerene, weil zumindest der Bindungsanteil bei den Fullerenen im Bereich der Pentagone überwiegend Doppelbindungscharakter und weniger aromatische Anteile enthält. Die Spheren bestehen jedoch vollständig aus aromatischer Struktur; sie sind daher z.B., bezüglich oxidativem Angriff weitaus stabiler als die Fullerene.

Anders als Fullerene werden die Spheren ausschließlich durch übliche organisch-chemische Syntheseverfahren hergestellt. Besonders die Herstellung von Spheren mit hoher Netzwerkdichte, bei denen die Flächen zwischen den pyrroldekorierten Öffnungen durch geschlossenes Hexagonnetzwerk gebildet wird, verlangen danach eine Vielzahl von Syntheseschritten. Weil aber gerade diese Spheren von besonders hoher Beständigkeit sind, besteht die Aufgabe, für diese Spheren eine wirtschaftlichere Darstellungsmethode zu finden. Weiterhin ist es von Interesse, Spheren mit einer beliebigen Symmetrie herzustellen und auch die Kohlenstoffzahl x der Fullerene, auf die die Spheren zurückgeführt werden können, nach oben unbegrenzt zu lassen.

Diese Aufgaben werden durch das erfindungsgemäße Verfahren zur Herstellung von Spheren gelöst. Darüber hinaus gelingt es mit dem erfindungsgemäßen Verfahren, Spheren herzustellen, deren Öffnung nicht nur mit anellierten Pyrrolringen dekoriert sind, also die Form von anellierten Porphinen, Subporphinen oder Superporphinen haben können, sondern auch anstelle der anellierten Pyrrolringe jeweils anellierte Furan-, Thiophen-, Selenophen-oder Cyclopentadienringe enthalten können. Darüber hinaus sind die erfindungsgemäßen Spheren nicht auf die Öffnungsgröße von Subporphin, Porphin oder Superporphin beschränkt, sondern können weitaus größer hergestellt werden. Diese großen Öffnungen in den Spheren enthalten außer den genannten anellierten Heteropentacyclen und anellierten Carbopentacyclen Funktionen, wie sie beispielhaft in den Formeln 0.1) bis 0.6) gezeigt werden.

Nach dem erfindungsgemäßen Verfahren gelingt die Herstellung der genannten Spheren in wenigen Schritten. Ausgangsstoffe sind Fullerene, Heteroffullerene oder Spheren. Dabei werden die folgenden chemischen Verfahrenschritte angewendet:

a) Fullerene oder Heterofullerene, also Spheren, die keine Öffnungen in ihrem geschlossenen 12 Pentacyclen enthaltenden Hexagonnetzwerk enthalten, werden einer oxidierenden Öffnung in der Gegenwart sauerstoffhaltiger oxidierend wirkender Fluide unterworfen. Sauerstoffhaltige Komponenten wie z. B. Sauerstoff Ozon, Stickoxide, Sauerstoffplasma, Wasserstoffperoxid, Fentons Reagens, Salpetersäure, Perchlorsäure, Peroxide, Chromat, Permanganat eignen sich für die oxidierende Öffnung. Durch die Gegenwart katalysierender Komponenten wie Bestrahlung, speziell im kurzwelligen sichtbaren Photonenspektrum bis in die ultravioletten Wellenlängenbereiche, ggf in der Gegenwart von Photokatalysatoren wie z. B. $TiO_2$ oder $ZrO_2$, lassen sich die Öffnungsreaktionen noch beschleunigen, oder auch in der Gegenwart schwacher

0.1)

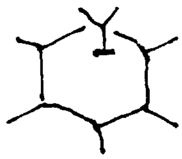

Y = O, S, Se, N, NH, CH, BH

0.1.1)

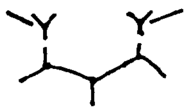

Y = O, S, Se, N, NH, CH, BH

0.2)

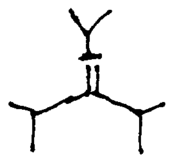

Y = O, S, Se, NH

0.3)

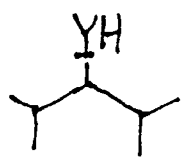

Y = O, S, Se, NH

0.4)

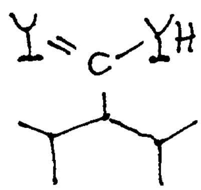

Y = O, S, Se, NH

In den Formeln 0.1) bis 0.6) bedeutet Y überwiegend dreifach koordiniertes C, B oder N.

0.5)

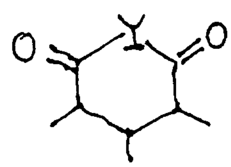

Y = NH, O, S, Se

0.6)

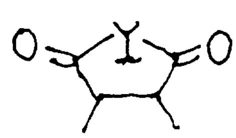

Y = NH, O, S, Se

Oxidationsmittel wie Wasser oder gar Von Reduktionsmitteln wie z. B. Schwefelwasser oder Ammoniak durchführen. Die photolytisch induzierte Öffnungsreaktion wird vorzugsweise unter 100 °C in der Gas- oder Flüssigphase durchgeführt. Auch die nicht photolytisch induzierte Öffnungsreaktion mit Oxidationsmitteln in der flüssigen Phase wird vorzugsweise bei Temperaturen unter 100 °C in der wässerigen Phase vorgenommen. Es ist auch möglich, die Fullerene bzw. Heterofullerene in fluiden Elektrolyten anodisch zu oxidieren. Auch hier liegen die bevorzugten Reaktionstemperaturen in flüssigen Elekrolyten unter 100 °C. Bei der Anwendung überkritischer Gase als Elektrolyt liegen die bevorzugten Reaktionstemperaturen unter 350 °C. Der Temperaturbereich unter 350 °C wird auch für die chemische Öffnungsreaktion mit sauerstoffhaltigen Oxidationsmitteln in überkritischen Reaktionsphasen vorgezogen. Als überkritische Reaktionsphase wird hier sowohl die überkritische Gasphase mit Dichten über 0,1 $g/cm^3$ als auch die mit überkritischer Gasphase gesättigte oder mit dieser koexistierende Flüssigphase bezeichnet. Die Gasphasenoxidation wird vorzugsweise bei Atmosphärendruck und bei Reaktionstemperaturen zwischen 300 °C und 450 °C durchgeführt. Die Reaktion mit sauerstoffhaltigen Plasmen wird vorzugsweise bei Temperaturen unter 200 °C vorgenommen. Die Dauer der Oxidationsreaktion wird vorzugsweise so bemessen, daß nicht mehr als der beabsichtigten Größe der Öffnungen in den Fullerenen bzw. Heterofullerenen entsprechende Molekularbestandteile aus den Fullerenen herausgelöst werden. Das kann durch quantitative Bestimmung der entstehenden Reaktionsprodukte wie $CO_2$ Carbonat bzw. Borsäure oder Borat gut kontrolliert werden.

b) Entsprechend a) geöffnete Fullerene oder Öffnungen enthaltende Spheren, deren Öffnungen erweitert werden sollen, können ebenfalls den unter a) genannten Reaktionsschritten unterzogen werden. Dabei ist allerdings zu berücksichtigen, daß die Reaktionsgeschwindigkeit der geöffneten Fullerene bzw. Heterofullerene bzw. Spheren geringer ist, als diejenigen betreffenden Fullerene bzw. Heterofullerene und daher geringfügig drastischere Reaktionsbedingungen notwendig sein können. Durch analytische Bestimmungen der Reaktionsprodukte ist auch hierbei eine gute Reaktionskontrolle möglich.

c) Abbauende, aufbauende oder aktivierende Konditionierung der durch die chemischen Verfahrensschritte a) bzw. b) mit Öffnungen versehenen Fullerene bzw. Heterofullerene bzw. Spheren durch Gase vorzugsweise im Temperaturbereich zwischen 700 °C und 1.000 °C. Unter abbauender Konditionierung wird hier die Beseitigung von Funktionen verstanden, die die nach den, Verfahrensschritt a) erzeugten Öffnungen in den Spheren dekorieren. Solche Funktionen sind z.B. Nitrogruppen, Sulfonsäuregruppen, Carboxylfunktionen, Carboxylsäureanhydridgruppen, Lactone, Chinone, Phenole, Ether. Durch aktivierende Konditionierung werden hochreaktive Öffnungen erzeugt, die nahezu oder ganz ausschließlich reaktive Kohlenstoffunktionen bei den geöffneten Fullerenen oder fullerenstämmigen Spheren oder reaktive Kohlenstoffunktionen neben reaktiven Bor- und/oder Stickstoffunktionen bei den geöffneten Heterofullerenen oder heterofullerenstämmigen Spheren enthalten. Die aufbauende Konditionierung ist ein Verfahrensschritt, um eine Dekoration der Öffnungen mit CH- Funktionen oder Stickstoffunktionen oder Gemischen davon zu erzielen, womit vielfach jedoch eine Verkleinerung der Öffnungen in Bezug auf die Öffnungsgröße nach der aktivierenden Konditionierung gegeben ist. Die abbauende Konditionierung ist ein Verfahrensschritt, um die Öffnungen in Bezug auf die Öffnungsgröße nach der aktivierenden Konditionierung zu vergrößern. Die Reihenfolge der Konditionierungsschritte ist in der Regel so, daß vor der auf- oder abbauenden Konditionierung die aktivierende Konditionierung liegt. Die aufbauende oder abbauende Konditionierung kann auch, je nach den verwendeten Reaktionsgasen mit der aktivierenden Konditionierung verbunden werden.

Für die aktivierende Konditionierung eignen sich die Edelgase und Stickstoff. Für die abbauende Konditionierung eignen sich Wasserdampf Ammoniak, Schwefelwasserstoff Selenwasserstoff Kohlendioxid, Sauerstoff Schwefeldioxid, Schwefel, Stickstoffoxide oder Gemische davon. Bei der abbauenden Konditionierung ist eine vorausgehende aktivierende Konditionierung nicht notwendig, weil die vorhandenen Funktionen unter den Reaktionstemperaturen instabil sind und vergast werden. Gleiches gilt im Fall der aufbauenden Konditionierung. Für die aufbauende Konditionierung eignen sich Gase, die unter den gegebenen Temperaturbedingungen eine Tendenz zur Abscheidung von Kohlenwasserstoffunktionen, Kohlenstoffstickstoffunktionen, Stickstoffunktionen, Stickstoftwasserstoffunktionen, Wasserstoffunktionen oder Gemischen davon haben. Das können Gase wie Methylamin, Methan, Methanol, Methylmercaptan, Methylselenol oder Gemische davon sein. Darüber hinaus eignen sich auch die entsprechenden $C_2$-Homologen oder noch höhere C-Verbindungen zu diesem Zweck. Von Nachteil ist eine zu schnelle Kondensation der genannten Funktionen in den Öffnungen, was z. B. bei den $C_2$ - oder höheren Verbindungen der Fall sein kann. In diesen Fällen ist es vorteilhaft für die aufbauende Konditionierung diese Gase im Gemisch mit abbaukonditionierenden Gasen wie z. B. Wasserdampf Wasserstoff oder/und Methylamin so zu mischen, daß noch ein aufbauendes Konditionspotential resultiert. Vorteilhafte Gase für die aufbauende Konditionierung sind auch Blausäure und Dicyan. Sowohl die aufbauende als auch die abbauende Konditionierung mit Stickstoff-Wasserstoff- sowie Stickstoff-Wasserstoff-Kohlenstoff-Verbindungn erzeugt neben der Kohlenwasserstoffdekoration in den Öffnungen zusätzlich Stickstoff- und/oder Stickstoff-Wasserstoffdekoration in den Öffnungen, während die übrigen Gase zur auf- oder abbauenden Konditionierung ausschließlich oder überwiegend Kohlenwasserstoffdekoration in den Öffnungen hinterläßt. Die von den Stickstoffverbindungen in den Öffnungen hinterlassene Dekoration besteht bei den kleinen Öffnungen überwiegend aus anellierten Pyrrolringen, die Öffnungen in der Form von anellierten Subporphinen, Porphinen oder Superporphinen bilden. Die anellierten Subporphine, Porphine und Superporphine bilden auch die kleinsten Öffnungen, die in den Fullerenen bzw. Spheren möglich sind. Auch bei den größeren Öffnungen kann die von den Stickstoffverbindungen hinterlassene Dekoration neben den anellierten Pyrrolringen anellierte Pyridinringe enthalten. Bei der auf-und abbauenden Konditionierung mit überwiegend stickstoffreien Konditioniermitteln werden die Öffnungen mit vorzugsweise anellierten Cyclopentadien- und anellierten Benzolringen dekoriert. Auch hier entspricht die Konfiguration der Ringdekoration bei den kleinst möglichen Öffnungen derjenigen der anellierten Subporphine, Porphine und Superporphine. Durch die aufbauende Konditionierung können auch anellierte Heptagonringe generiert werden.

d) Aufbauende Konditionierung der durch den Verfahrensschritt c) aktivierende Konditionierung der Öffnungen in Fullerenen oder Spheren vorbereiteten Reaktanten mit anellierten O-, S-, Se-, N-, oder C-haltigen Penta- und Hexacyclen durch Reaktion mit Sauerstoff, Schwefel, Selen, aktiviertem Stickstoff oder aktiviertem Methan. Diese Reaktion wird vorzugsweise bei Temperaturen unter 300 °C vorgenommen. Auch hierbei können die Öffnungen mit Dekorationen versehen werden, die bei den kleinsten Öffnungen den Pentacyclenarrangements der anellierten Porphine, Sub- oder Superporphine entsprechen. Dabei werden die anellierten Pentacyclen durch Furan-, Thiophen-, Selenophen, Pyrrol- oder Cyclopentadien-Ringe repräsentiert. Größere Öffnungen können auch hier neben anellierten Pentacyclen anellierten Hexacyclen vom Pyran-, Thiopyran-, Selenopyran-, Pyridin- oder Benzoltyp enthalten..

e) Substituierende Konditionierung der durch den Verfahrensschritt c) abbauende oder aufbauende Konditionierung der Öffnungen überwiegend mit Wasserstoffunktionen, ungepaarten oder gepaarten Elektronen dekorierten Öffnungen durch Reaktion mit $O_2$, Schwefel, Selen oder Sauerstoff-, Stickstoff- oder Methanplasma oder Schwefel-und Selenhalogeniden bei Temperaturen zwischen 50 °C und 500 °C oder durch lewissauer katalysierte oder schwermetallsalzkatalysierte Reaktion mit Sauerstoff, Schwefel oder Selen bei Temperaturen unter 350 °C. Auch hierbei lassen sich die Öffnungen mit anellierten O-, S-, Se-, und ggf. N-haltigen Heteropenta- und bei größeren Offnungen zusätzlich Heterohexacyclen dekorieren wie unter Verfahrensschritt d) beschrieben.

f) Sauerstoffsubstitution anellierter Furane des Öffnungsdekors durch Schwefel oder Selen durch säurekatalysierte Umsetzung von Schwefelwasserstoff oder Selenwasserstoff vorzugsweise in der Gegenwart von Halogenwasserstoff und bei Temperaturen unterhalb von 250 °C. Das Furandekor wird dadurch in ein Thiophen- oder Selenophendekor umgesetzt.

In den nachfolgenden Erläuterungen werden die folgenden Vereinfachungen in der Schreibweise gewählt:

- Anellierte Heteropentacyclen heißen AHPC
- Anellierte Carbopentacyclen heißen ACPC
- Anellierte Hetero- und Carbopentacyclen heißen APC
- Fullerene und Bor-, Stickstoff und Kohlenstoff enthaltenden Heterofullerene heißen Fullerene oder F
- Fullerenfragmente und Heterofullerenfragmente, die zwar topologisch eine sphärische Molekülgestalt haben, aber kein vollständig geschlossenes Netzwerk aus Hexa-und Pentacyclen besitzen, also Öffnungen in diesem Netzwerk enthalten, heißen Spheren oder S

Die Behandlung der F und S mit den reaktiven Gasen wird bei Reaktivgaspartialdrücken von vorzugsweise 0,1 bar bis 1 bar vorgenommen. Die Umsetzung der F und S wird bevorzugt in heterogener Phase vorgenommen. Dazu werden die F oder S auf einen Träger wie z. B. Kieselgelpellets verteilt und dann unter den genannten Bedingungen den reaktiven Gasphasen ausgesetzt. Eine besondere Variante des erfindungsgemäßen Verfahrens ist die Verwendung von photokatalytisch wirksamen Halbleitern als F- oder S-Träger. Solche Photokatalysatoren sind z. B. Titandioxid in der Anataskristallform, Zirkondioxid, Zinkoxid, Zinndioxid, Cadmiumsulfid, Nioboxid, Tantaloxid, Bariumtitanat, Berylliumzirkonat. In diesen Fällen ist es vorteilhaft, die F- oder S-beschichteten Photokatalysatoren mit Photonen der Wellenlängen zu bestrahlen, die die photokatalytische Umsetzung der F oder S mit den oxidierenden Gasen bewirkt. Bei dem vorzugsweise verwendeten Anatas liegen die wirksamen Photonenenergien bei Wellenlängen von kleiner 400 nm. Bei der photokatalytischen F- oder S-Umsetzung mit den reaktiven Gasen liegen die bevorzugten Reaktionstemperaturen bei der Behandlung mit sauerstoffhaltigen Oxidationsmitteln vorzugsweise zwischen 20 °C und 100 °C. Unter dem photokatalytischen Einfluß kann die Fullerenoxidation auch in flüssiger Phase mit oxidierenden Medien z. B. Wasserstoffperoxid, Sauerstoff, Ozon, Salpetersäure vorgenommen werden. Der Photokatalysator kann in diesen Fällen auch als Suspension zugegen werden, die vorteilhaft mit den umzusetzenden F oder S beschichtet ist. Zur weiteren Behandlung in der Gasphase können die getrockneten und gewaschenen mit oxidierten F oder S beschichteten Träger in der Gasphase dann entsprechend den Verfahrensschritten b) bis f) eingesetzt werden.

Die Beschichtung der Träger mit F oder S geschieht durch Einpudern oder Adsorption aus Lösung. Besonders vorteilhaft ist das Auftragen der F oder S als Langmuir-Blodget-Film auf den/die Träger. Dieses

Verfahren eignet sich vorzüglich zur gleichförmigen Beschichtung von Oberflächen mit S, die nach der Umsetzung aus der F- oder S-Schicht entstehen.

In den meisten Fällen lassen sich die umzusetzenden F oder S nicht in eine Lösung in organischen verdampfbaren Lösungsmitteln überführen, die eine Voraussetzung für die Erzeugung derartiger Filme ist. In den Fällen, in denen keine Löslichkeit gegeben ist, ist oftmals auch keine Sublimierbarkeit der Fullerene gegeben, so daß auch die Anwendung der Vakuum-Sublimiertechnik zur Oberflächenbeschichtung versagt. Hier eignen sich Methoden wie das Beschichten von Gegenständen mit feinstzerriebenen F- oder S-Pulvern, die zuvor auf Wasseroberflächen gespreitet wurden oder elektrochemische Beschichtungsmethoden, die auf der elektrochemischen Zersetzung von in Tensidmizellen eingeschlossenen F oder S beruhen, wie sie von T. Saji in dem Buch Phthalocyanines Vol. 2, VCH-Verlag, 1993, am Beispiel der Beschichtung mit Phthalocyaninpigmenten beschrieben wird.

In der nachfolgenden Aufstellung wird eine Übersicht über die molekulare Topographie der nach dem erfindungsgemäßen Verfahren erzeugten heteropentacyclen-dekorierten Öffnungen in den Spheren gegeben:

1.1. Subphthalocyaninartig angeordnete APC-Dekoration entsprechend Formel (1):

1)

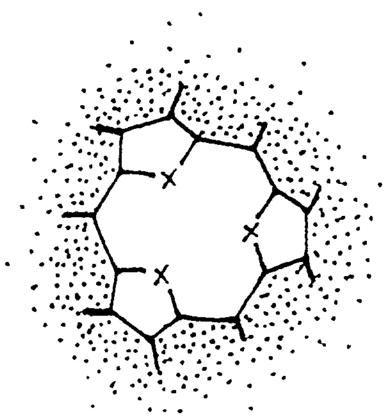

In der Formel (1) steht X für NH, N, O, $O^+$, S, $S^+$, Se, $Se^+$, $CH_2$ oder $CH^-$. Dabei ist X die einzige CH bzw. $CH_2$ bzw. Heterofunktion im jeweiligen Heteropentacyclus. Die punktiert dargestellte nahe Umgebung der Ausnehmung besteht aus lückenlosem Hexacyclennetzwerk aus Kohlenstoffatomen oder Kohlenstoff-, Stickstoff- und Boratomen oder einer weiteren APC-dekorierten Öffnung.

1.2. Subphthalocyaninartig angeordnete APC-Dekoration entsprechen Formel (1.1):

1.1)

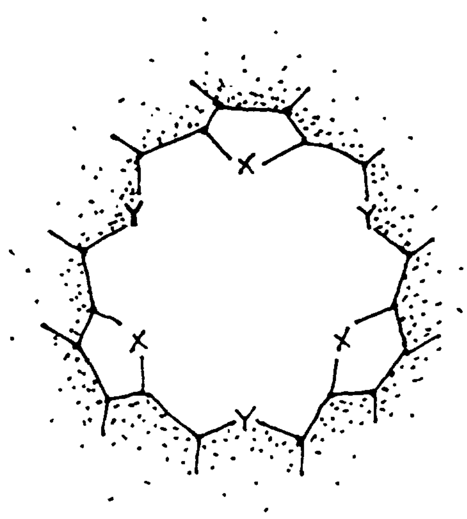

In der Formel (1.1) hat X die gleiche Bedeutung wie in der Formel (1). Das gilt auch für die Punktur. Y steht für $CH_2$, CH, N, NH, B, BH, CO, COH, BOH, O, S, Se.

1.3. Subphthalocyaninartig angeordnete APC-Dekoration entsprechend Formel (1.2):

1.2)

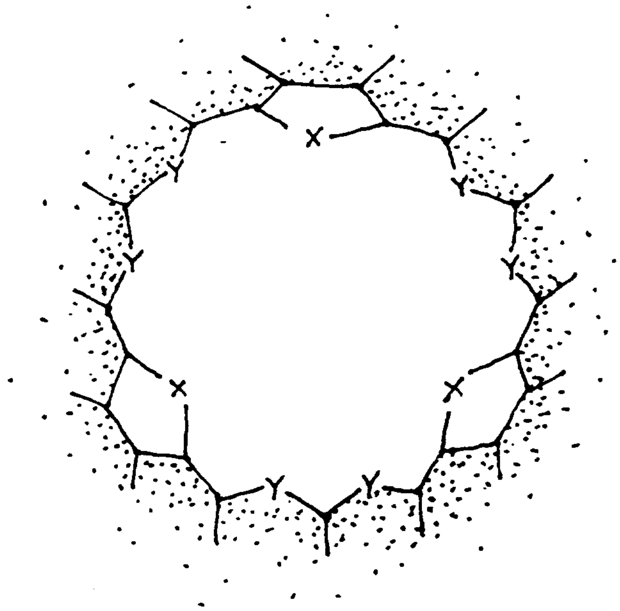

In der Formel (1.2) hat X die gleiche Bedeutung wie in der Formel (1). Das gilt auch für die Punktur. Y hat die gleiche Bedeutung wie in der Formel (1.1).

1.4. Subphthalocyaninartig angeordnete APC-Dekoration entsprechend Formel (1.3):

1.3)

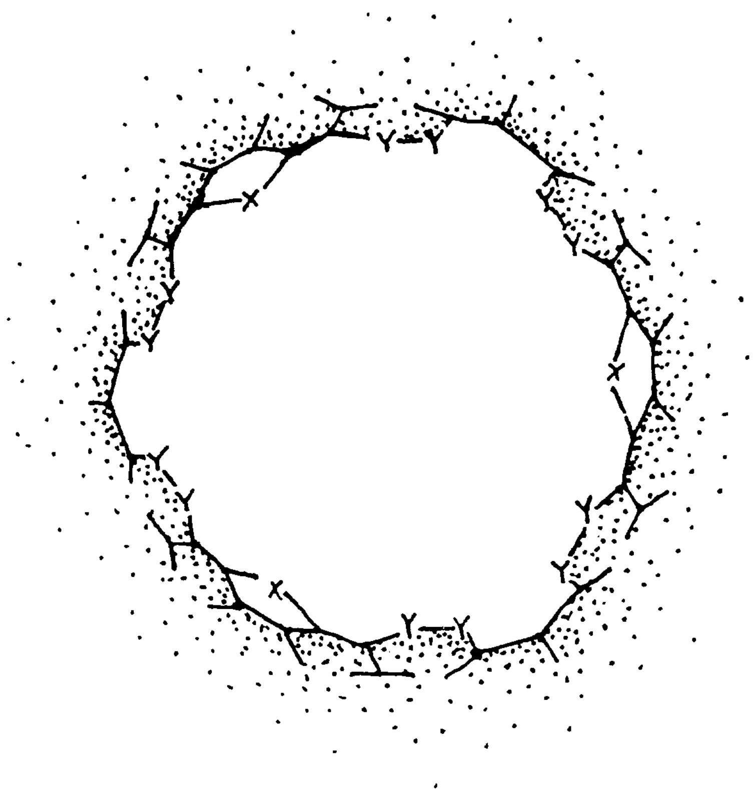

In der Formel (1.3) haben X, Y und die Punktur die gleiche Bedeutung wie in den Formeln (1) bis (1.2).

1.5. Subphthalocyaninähnlich aufgebaute asymmetrische APC-Dekorationen wie beispielhaft in der Formel (1.4) gezeigt:

1.4)

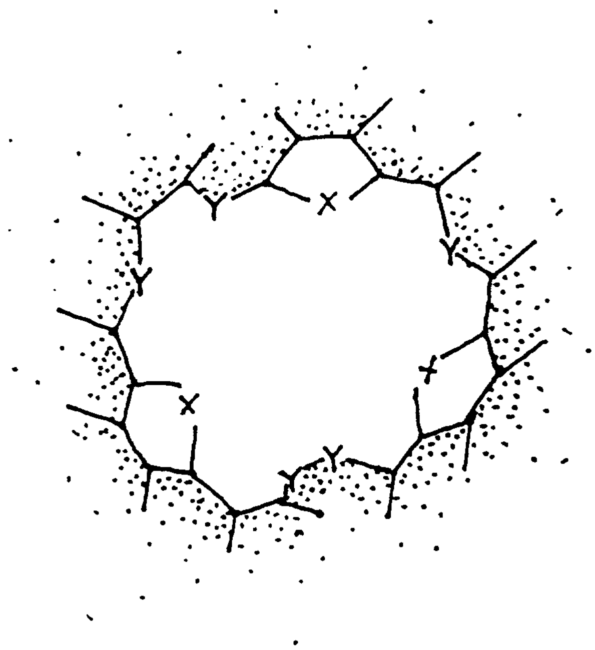

In der Formel (1.4) haben X, Y und die Punktur die gleiche Bedeutung wie in den Formeln (1) bis (1.3).
2.1. Phthalocyanin-/porphinartig angeordnete APC-Dekoration entsprechen Formel (2):

2)

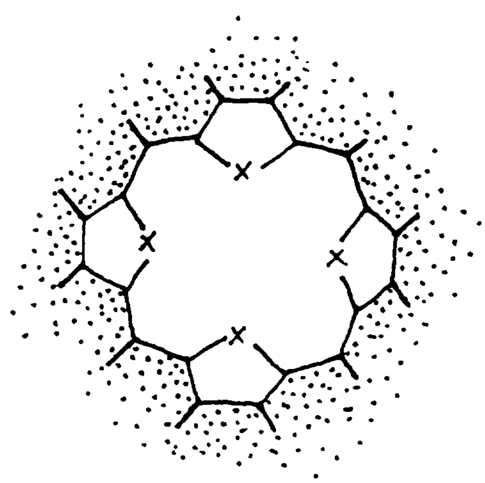

In der Formel (2) hat X die gleiche Bedeutung wie in der Formel (1). Das gilt auch für die Punktur.
2.2. Phthalocyanin-/porphinartig angeordnete APC- Dekoration entsprechend Formel (2.1):

2.1)

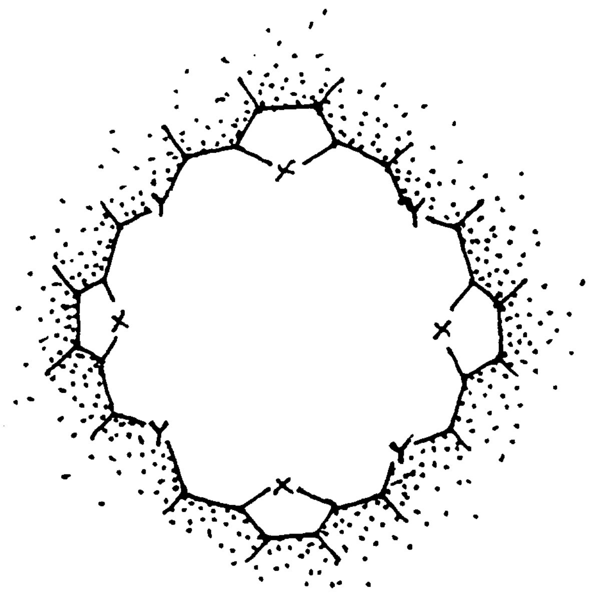

In der Formel (2.1) haben X, Y und die Punktur die gleiche Bedeutung wie in den Formeln (1) bis (2).

2.3. Phthalocyanin/porphinartig angeordnete APC-Dekoration entsprechend Formel (2.2):

2.2)

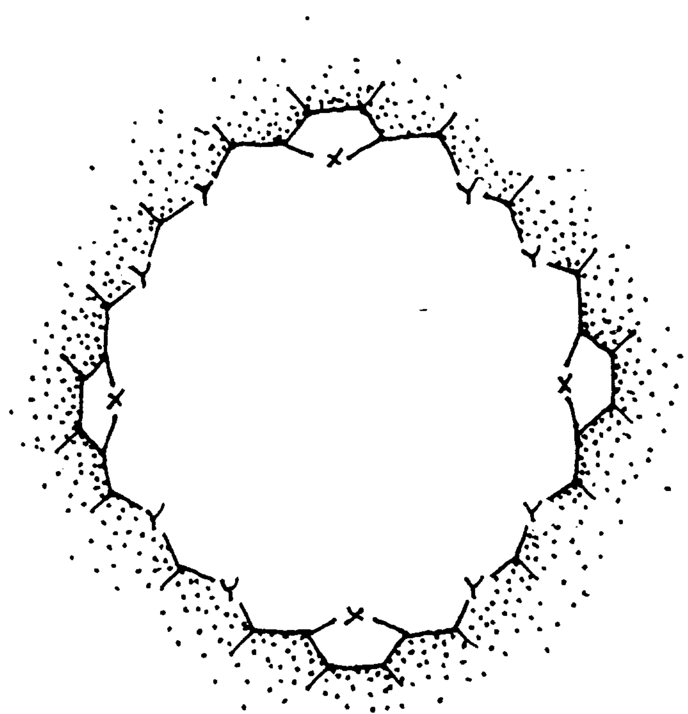

In der Formel (2.2) haben X, Y und die Punktur die gleiche Bedeutung wie in den Formeln (1) bis (2.1).

2.4. Phthalocyanin-/porphinartig angeordnete APC-Dekoration entsprechend Formel (2.3):

2.3)

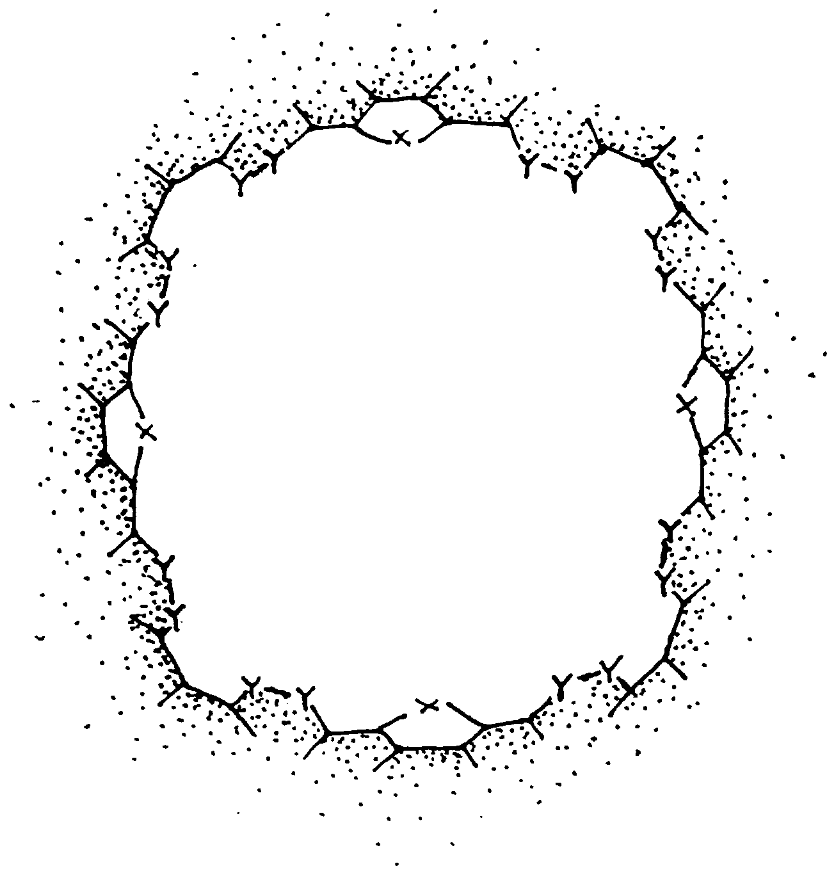

In der Formel (2.3) haben X, Y und die Punktur die gleiche Bedeutung wie in den Formeln (1) bis (2.2).

2.5. Phthalocyanin-/porphinähnlich aufgebaute asymmetrische APC-Dekorationen wie beispielhaft in den Formeln (2.4) und (2.5) gezeigt werden.

2.4)

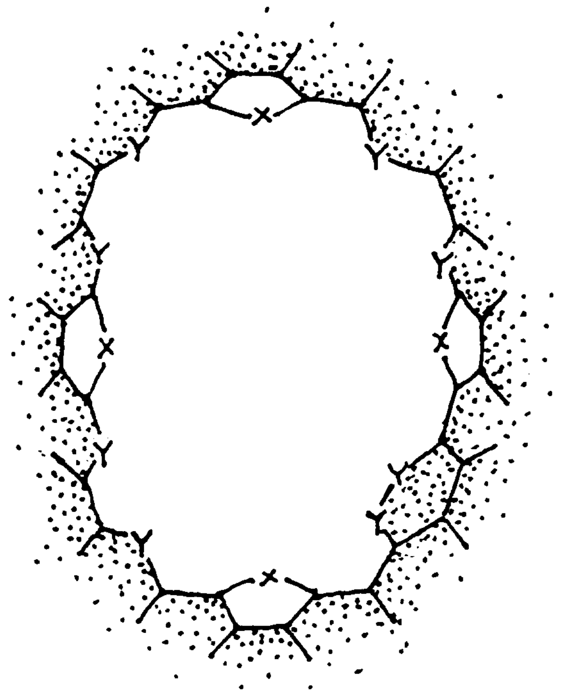

In den Formeln haben X, Y und die Punktur die gleiche Bedeutung wie in den Formeln (1) bis (2.3).

2.5)

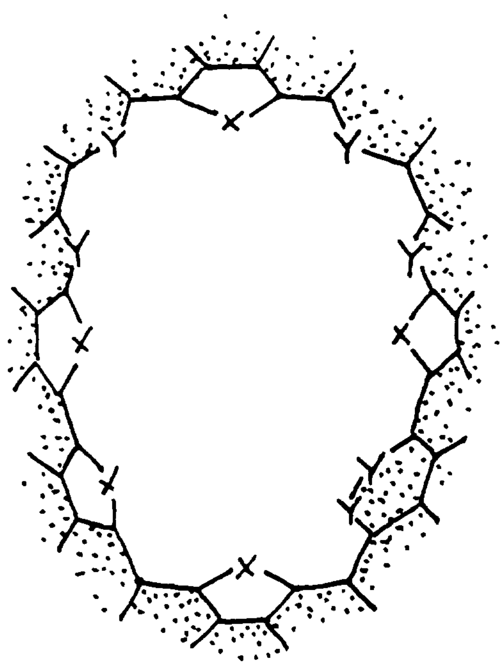

3.1. Superphthalocyaninartig angeordnete APC-Dekoration entsprechend Formel (3):

3)

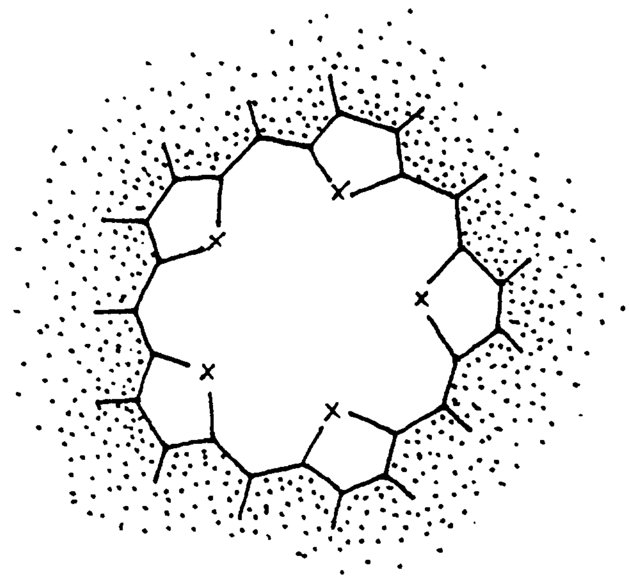

In der Formel (3) hat X die gleiche Bedeutung wie in den Formeln (1) und (2). Gleiches gilt für die Punktur.

3.2. Superphthalocyaninartig angeordnete APC-Dekoration entsprechend Formel (3.1):

3.1)

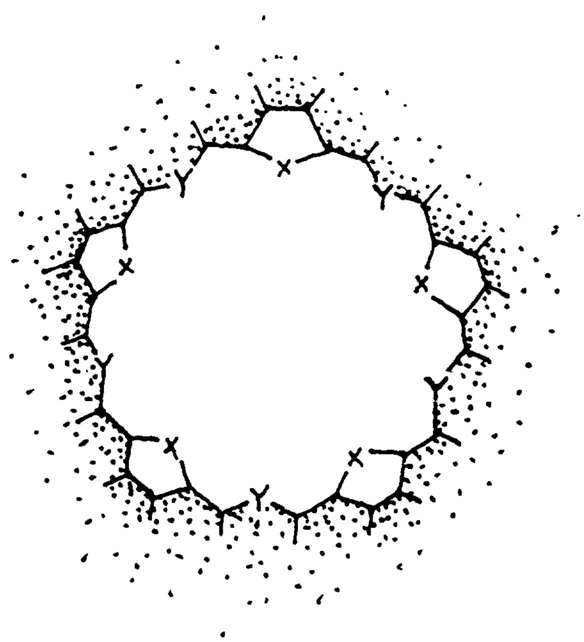

In der Formel (3.1) haben X, Y und die Punktur die gleiche Bedeutung wie in den Formeln (1) bis (3).

3.3. Superphthalocyaninartig angeordnete APC-Dekoration entsprechend Formel (3.2):

3.2)

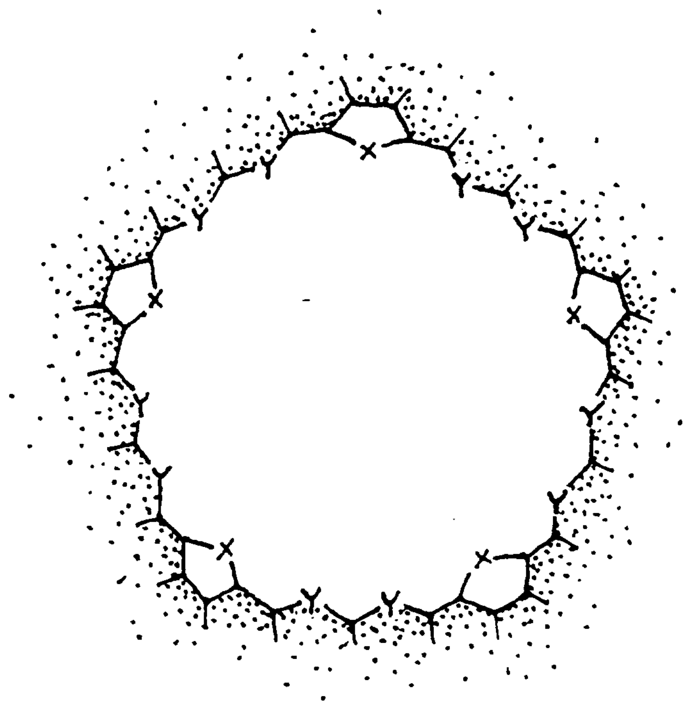

In der Formel (3.2) haben X, Y und die Punktur die gleiche Bedeutung wie in den Formeln (1) bis (3.1).

3.4. Superphthalocyaninartig angeordnete APC-Dekoration entsprechend Formel (3.3):

3.3)

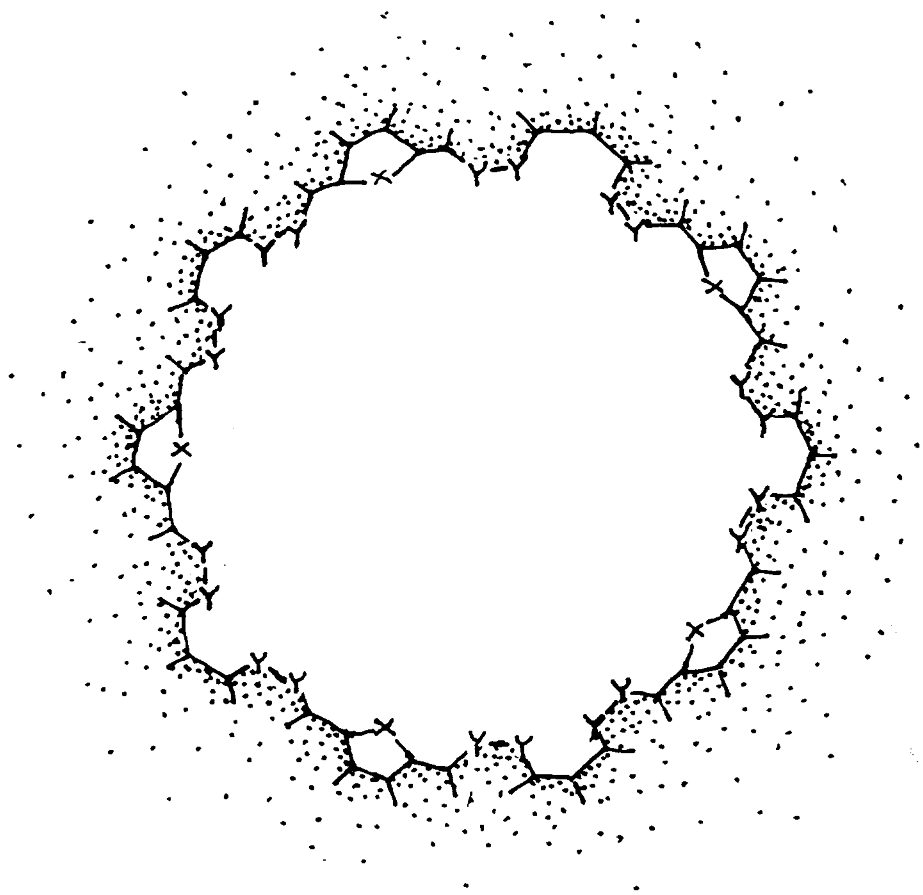

In der Formel (3.3) haben X, Y und die Punktur die gleiche Bedeutung wie in den Formeln (1) bis (3.2).

3.5. Superphthalocyaninähnlich aufgebaute asymmetrische APC- Dekorationen wie beispielhaft in den Formeln (3.4) und (3.5) gezeigt.

3.4)

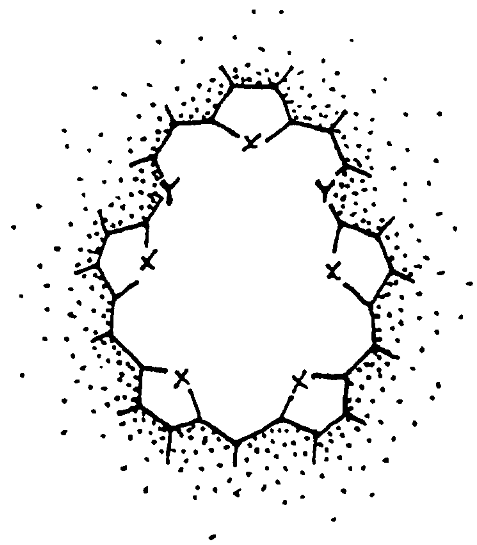

3.5)

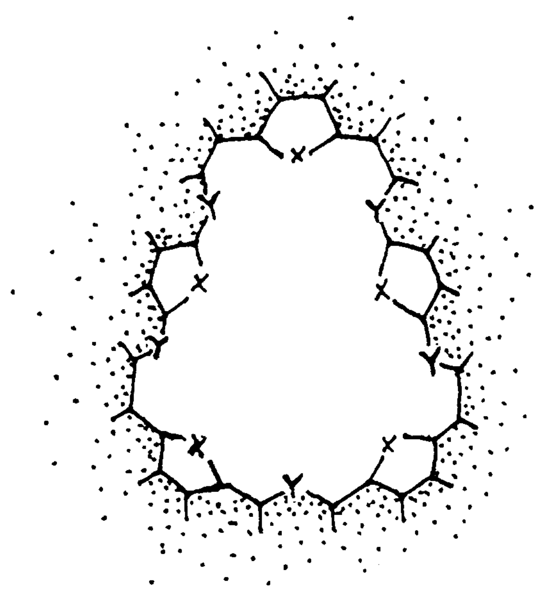

In den Formeln (3.4) und (3.5) haben X, Y und die Punktur die gleiche Bedeutung wie in den Formeln (1) bis (3.3).

Zur Kennzeichnung der in den Formeln 1) bis 3.5) beispielhaft gezeigten Öffnungen in den erfindungsgemäßen Spheren wird eine Nomenklatur verwendet, die nachfolgend erläutert wird.

Die APC-dekorierten Spherenöffnungen werden durch das Synonym "Porphin" gekennzeichnet. APC-dekorierte Spherenöffnungen werden durch die an die Bezeichnung Porphin angehängte Zahlen- und Symbolgruppen näher gekennzeichnet. Die Zahlen-bzw. Symbolgruppen lassen sich in vier Klassen einteilen, die hier mit den Buchstaben a bis d bezeichnet werden.

<u>Zahlengruppe a</u>

Porphine, die APC entsprechend Formel (3.6) enthalten, werden als Porphin-3-, diejenigen, die APC entsprechend Formel (3.7) enthalten, werden als Porphin-4- und diejenigen, die APC entsprechend Formel (3.8) enthalten als Porphin-5- bezeichnet. Die Zahlengruppe a umfaßt also nur drei mögliche Ziffern.

Zahlengruppe b

Eine Zahlengruppe von jeweils 3 bis 5 Zahlen, die untereinander durch Schrägstriche getrennt sind, kennzeichnet die Form und Größe der APC-dekorierten Spherenöffnungen. Jede Zahl gibt den Abstand zwischen zwei APC an, wobei sich die Zahl aus der Anzahl von Hexagonecken ergibt, die zwischen den APC liegen. Gezählt werden aber nur diejenigen Hexagonecken, die in der Projektionslinie sichtbar sind, die bei Projektion entsprechend Formel (3.9) in oder entgegen Pfeilrichtung auf den Öffnungsrand entsteht. Dabei beträgt der Winkel zwischen den beiden strichpunktierten Geraden 72 ° oder 90 ° oder 120 °. Ausgenommen von dieser Regel sind asymmetrische Spherenöffnungen, sowie diejenigen Öffnungen, die bei vollständiger Entfernung der Halbspheren nach dem erfindungsgemäßen Verfahren an den Enden der rohrförmigen Fullerene entstehen. Die letzteren Öffnungen enthalten nur in Ausnahmefällen APC-Dekorationen mit Thiophen oder Selenophen, die darüber hinaus völlig asymmetrisch angeordnet ist. Versuche, mit dem erfindungsgemäßen Verfahren Pyrrol-, Furan- oder Cyclopentadien-APC-Dekoration zu installieren, sind fehlgeschlagen.

Die Formeln (3.10) bis (3.13) zeigen jeweils beispielhaft die Struktur zwischen APC. Die Pfeile zeigen die jeweils zu zählenden Hexagonecken.
Die Tabelle 1 gibt beispielhaft die Nomenklatur der Formeln (1) bis (3.3) unter Beschränkung auf die Zahlengruppen a und b.

Tabelle 1

| Nomenklaturbeispiele für Spherenöffnungen unter Beschränkung auf die Zahlengruppen a und b | |
|---|---|
| Formel | Nomenklatur Zahlengruppe |
| | a b |
| (1) | Porphin-3-1/1/1 |
| (1.1) | Porphin-3-3/3/3 |
| (1.2) | Porphin-3-5/5/5 |
| (1.3) | Porphin-3-7/7/7 |
| (2) | Porphin-4-1/1/1/1 |
| (2.1) | Porphin-4-3/3/3/3 |
| (2.2) | Porphin-4-5/5/5/5 |
| (2.3) | Porphin-4-7/7/7/7 |
| (3) | Porphin-5-1/1/1/1 |
| (3.1) | Porphin-5-3/3/3/3 |
| (3.2) | Porphin-5-5/5/5/5 |
| (3.3) | Porphin-5-7/7/7/7 |

Asymmetrische Spherenöffnungzen mit APC-Dekoration sind dadurch gekennzeichnet, daß die Zahlengruppe b entweder ungleiche Zahlen enthält oder gerade Zahlen. Die Formel (3.13) zeigt ein Beispiel für die Struktur zwischen zwei APC in einer asymmetrischen Spherenöffnung. Der Abstand beträgt in diesem Beispiel 2. Die Tabelle 2 ergibt beispielhaft die Nomenklatur der asymmetrischen Spherenöffnungen, die in den Formeln (1.4) bis (3.4) gezeigt werden.
Die Asymmetrie der Spherenöffnungen bezieht sich ausschließlich auf die entsprechend den Formeln (3.6) bis (3.8) angeordneten APC.

Tabelle 2

| Nomenklaturbeispiele für asymmetrische Spherenöffnungen unter Beschränkungen auf die Zahlengruppen a und b. | |
|---|---|
| Formel | Nomenklatur Zahlengruppe |
| | a b |
| (1.4) | Porphin-3-3/4/4 |
| (2.4) | Porphin-4-4/4/4/4 |
| (3.4) | Porphin-5-1/1/1/2/2 |
| (3.5) | Porphin-5-2/2/2/2/3 |

Zahlengruppe c

Symmetrische und asymmetrische Spherenöffnungen - Asymmetrie wiederum nur bezogen auf die Anordnung der entsprechend Formel (3.6) bis (3.8) angeordneten APC - können zusätzliche APC enthalten. Sowohl Anzahl als auch Position der APC können durch hochgestellte Zahlen im Bereich der Zahlengruppe b gekennzeichnet werden. Dabei kennzeichnet die Zahl die Anzahl der in dem betreffenden Segment vorhandenen

3.6)

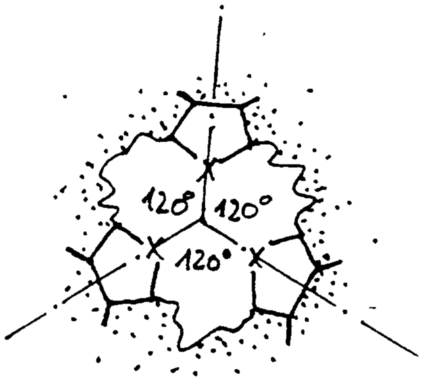


3.6.1)

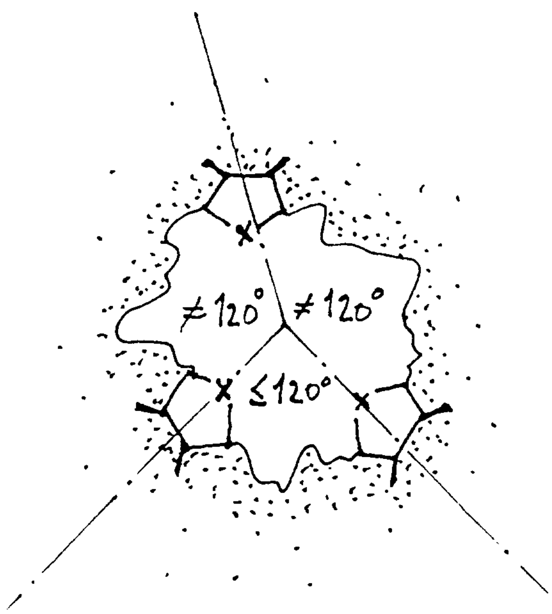

3.7)

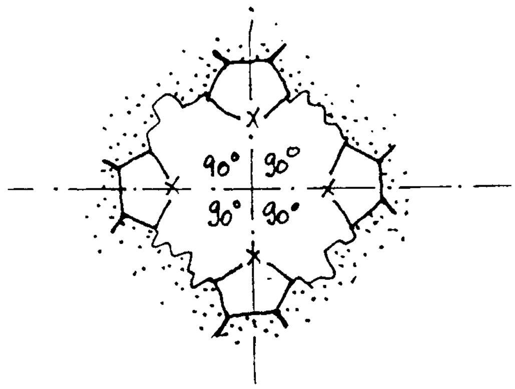


3.7.1)

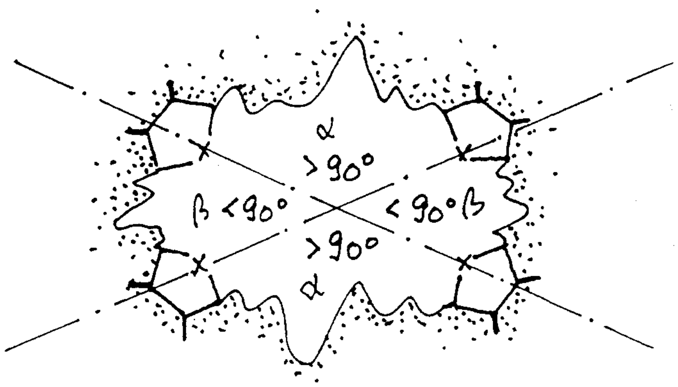


3.8)

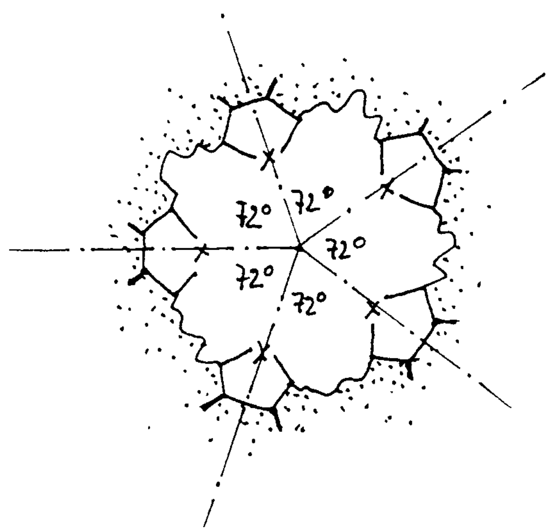

3.9)

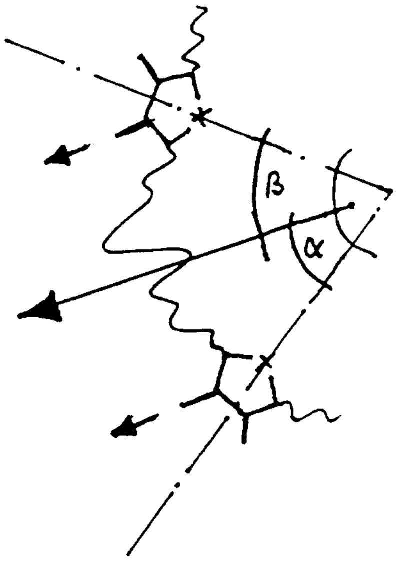


$\alpha = \beta = 36°$ oder $45°$ oder $60°$ bei porphinartigen Spherenöffnungen entsprechend Formeln 3.6), 3.7) und 3.8) sowie z. B. 1.3), 2.3) und 3.3) mit symmetrischen APC-Positionen

$\alpha = \beta \neq 45°$ bei porphin-4-ähnlichen Spherenöffnungen entsprechend Formel 3.7.1) mit nichtquadratischen APC-Positionen

$\alpha =$ oder $\neq \beta$ bei porphin-3-ähnlichen Spherenöffnungen entsprechend Formel 3.6.1) mit asymmetrischen APC-Positionen

3.10)

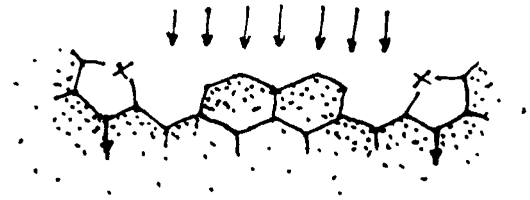

3.11)

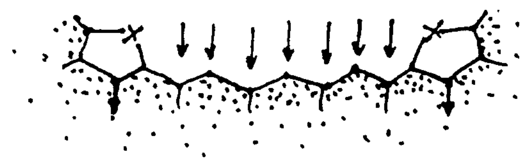

3.12)

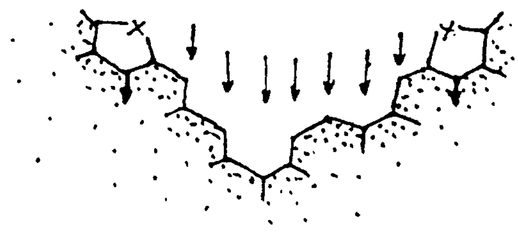

3.13)

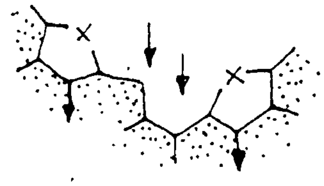

zusätzlichen APC. Die Zuordnung der hochgestellten Zahl zu einem bestimmten Abschnitt des Öffnungsrandes wird durch die Plazierung bei einem bestimmten Element der Zahlengruppe b erreicht.

Die in der Formel (2.5) gezeigte Spherenöffnung, die einen zusätzlichen APC enthält, ist daher als

Porphin-4-$4^1$/4/4/4

zu bezeichnen.

Zahlen-/Elementsymbolgruppe d

Die in den Porphinen (1) bis (3.5) mit X gekennzeichneten Heterofunktionen als Bestandteile der APC werden nach der Zahlengruppe b/c, von dieser durch einen Bindestrich getrennt, aufgezählt. In der Tabelle 3 wird die Gruppe d beispielhaft anhand einiger Formelbeispiele dargestellt; und zwar zusammen mit den übrigen Gruppen. Anhand der Nomenklatur lassen sich die erfindungsgemäßen Spheren hinreichend charakterisieren.

| Formel | Nomenklatur Zahlen-/Symbolgruppe |
|---|---|
| | a b/c d |
| (4) | Porphin-5-1/1/1/1/1-3N2NH |
| (4.1) | Porphin-5-3/3/3/3/3-5S |
| (4.2) | Porphin-4-4[1]/4/4/4-2N3NH |
| (5) | Porphin-4-1/1/1/1-4S |
| (6) | Porphin-3-0/0/0-3O |

4)

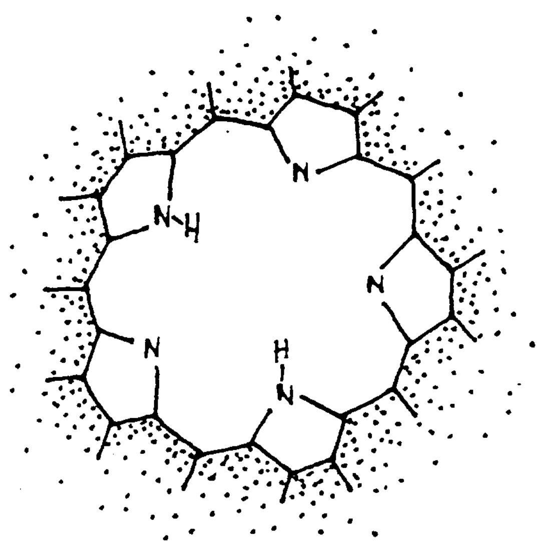

4.1)

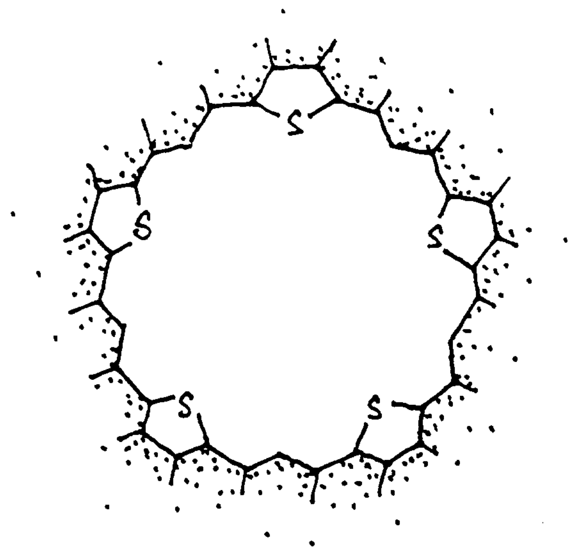

4.2)

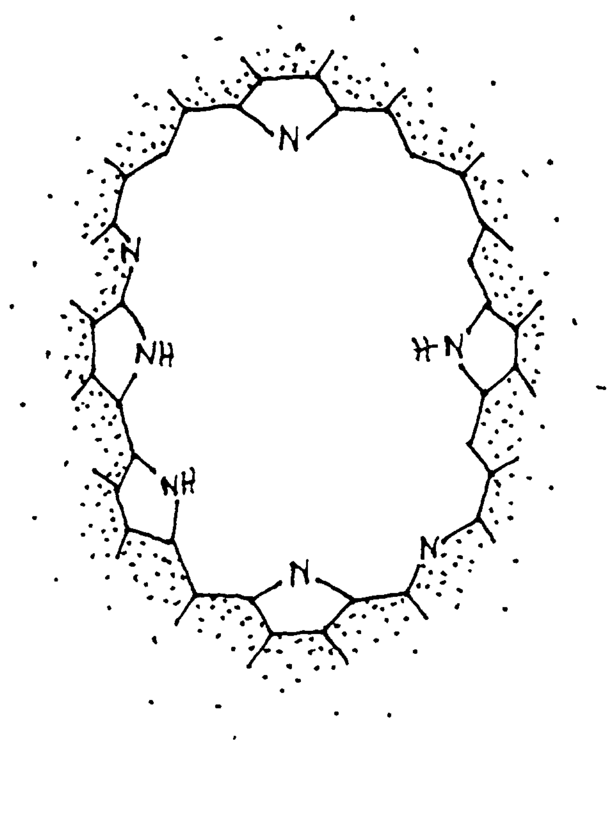

5)

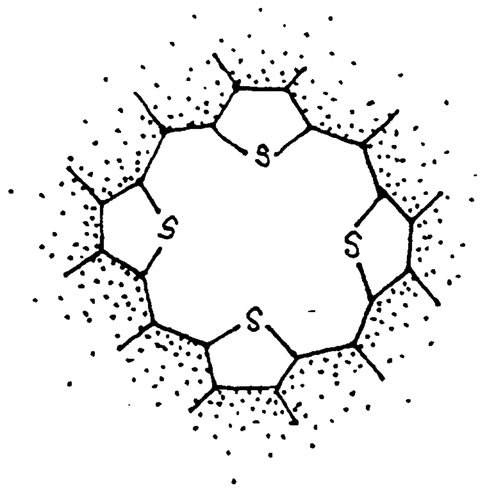

6)

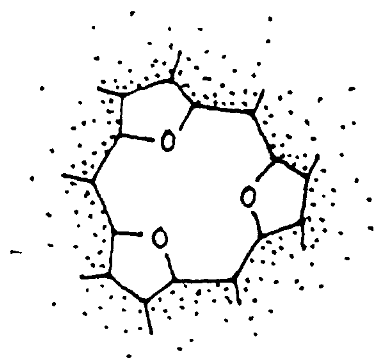

7)

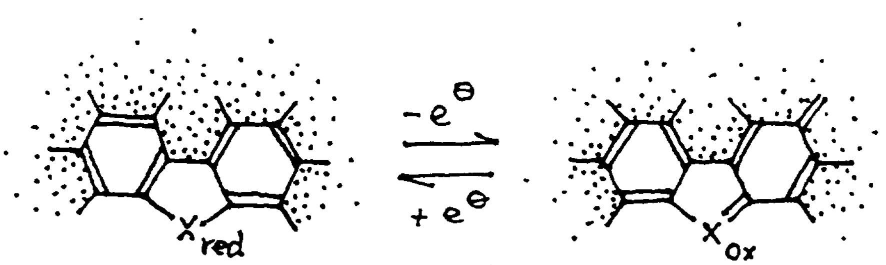


7.0.1) 7.0.2)

Es hat sich überraschend gezeigt, daß die Heteroatome der Heteropentacyclen, mit denen die Öffnungen dekoriert sind, reversibel aus der reduzierten Form (=NH bzw. =O bzw. =S bzw. =Se) in die oxidierte Form (=N- bzw. $=O^{+}$ bzw. $=S\text{-}^{+}$ bzw. $Se\text{-}^{+}$) durch chemische oder elektrochemische Oxidation bzw. Reduktion überführt werden können. Dabei können neben den aromatischen auch chinoide Strukturen auftreten, wie aus dem kennzeichnenden Ausschnitt aus dem Öffnungsrand der erfindungsgemäßen Spheren hervorgeht. Der kennzeichnende Ausschnitt der reduzierten Form entspricht einem anellierten Dibenzopyrrol oder Dibenzothiophen oder Dibenzofuran oder Dibenzoselenophen wie aus der Formel 7.0.1) hervorgeht. Diese besser als dreiseitig anellierte Pyrrole, Thiophene, Furane oder Selenophene bezeichneten Funktionen, in denen die Funktion $X_{red}$ eine der Funktionen - O, -S, -Se oder - NH bedeuten, lassen sich leicht durch oxidierende Gase oder Flüssigkeiten, wie Luft oder chinoide Stoffe enthaltende Lösungen als Beispiele, in die Porphyrine umwandeln, die ein oder mehrere der genannten Heteropentacyclen im oxidierten Zustand enthalten, wie er in der Formel (7.0.2) gezeigt wird. Dabei liegen die Heteroatome in den oxidierten Heteropentacyclen dreiwertig gebunden vor als $=O\text{-}^{\oplus}$, $=S\text{-}^{\oplus}$, $= Se\text{-}^{\oplus}$ oder =N-. Formelschema 7) zeigt vereinfacht und ausschnitthaft diesen reversiblen Oxidationsmechanismus wie er bei den erfindungsgemäßen Spheren bzw. deren Öffnungen durchgeführt werden kann.

Die Stickstoffunktionen der Pyrrolringe und die $CH_2$-Funktionen der Cyclopentadienringe neigen hingegen zur Elektronenaufnahme. Diese Funktionen neigen daher auch als Öffnungsdekoration in den Spheren dazu, Funktionen mit Elektronendonortendenz zu binden. Das gilt allerdings um so mehr für die Stickstoffunktion; weniger für die $CH_2$-Gruppe zu.

Speziell bei den größeren Öffnungen in den Spheren, die neben den Pyrrol-oder Furan- oder Thiophen- oder Selenophenfunktionen in der Regel auch weitere redoxaktive Heterofunktionen chinoiden oder hydrochinoiden Charakters enthalten, die bei den erfindungsgemäßen Umsetzungen der Fullerene und Heterofullerene entstehen können, kann die Bildung der Porphyrine auch ohne die Wirkung von Oxidationsmitteln geschehen, wie beispielhaft anhand der Formeln 7.1) und 7.2) gezeigt wird. Auch das Vorhandensein von pyronartigen Strukturen, wie es in den Formelausschnitten 7.5) und 7.6) gezeigt wird, kann die Porphinbildung forcieren. In den Spherenöffnungen vorhandene Carboxylfunktionen, die bevorzugt bei der oxidierenden Umsetzung und nachfolgenden Reaktion bei niedrigen Reaktionstemperaturen auftreten können, vermögen positive Ladungszustände der oxidierten Heteropentacyclen zu stabilisieren, wie anhand der Formelausschnitte 7.3) und 7.4) gezeigt wird.

7.1) + 7.2)

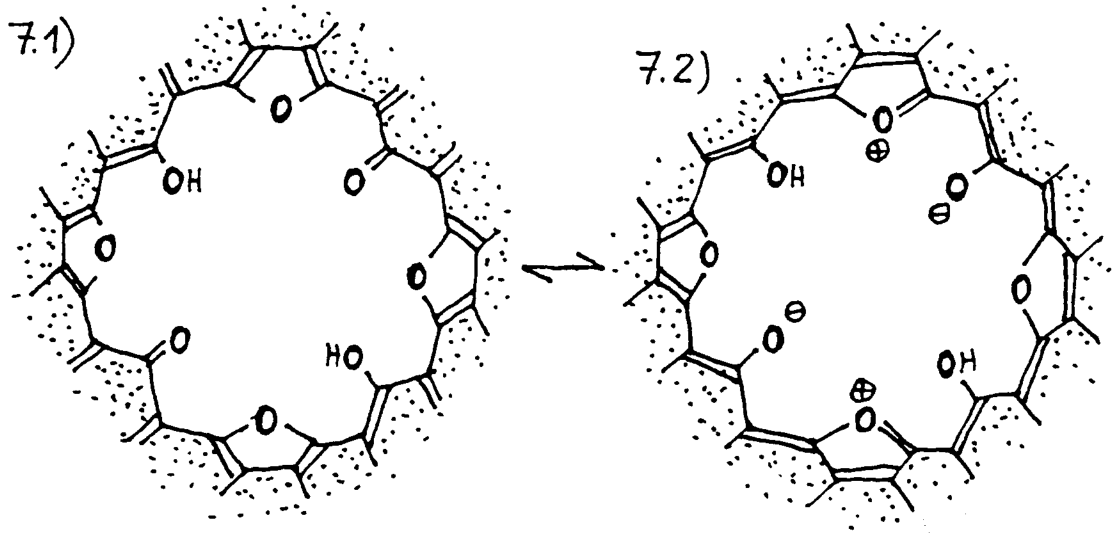

7.3) + 7.4)

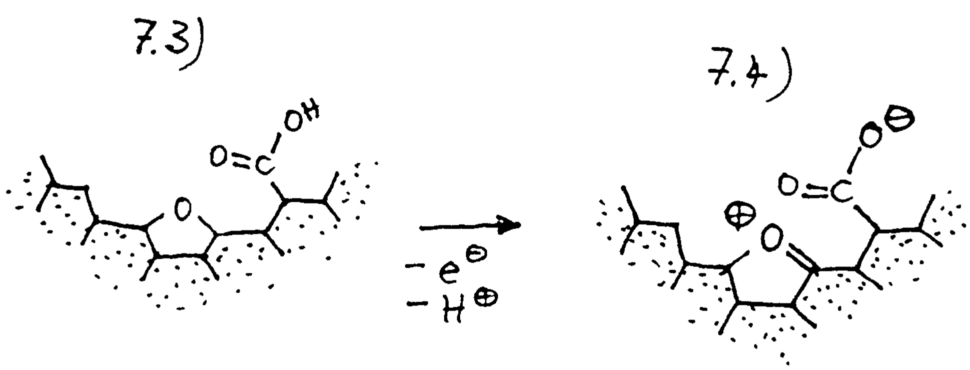


7.5) + 7.6)

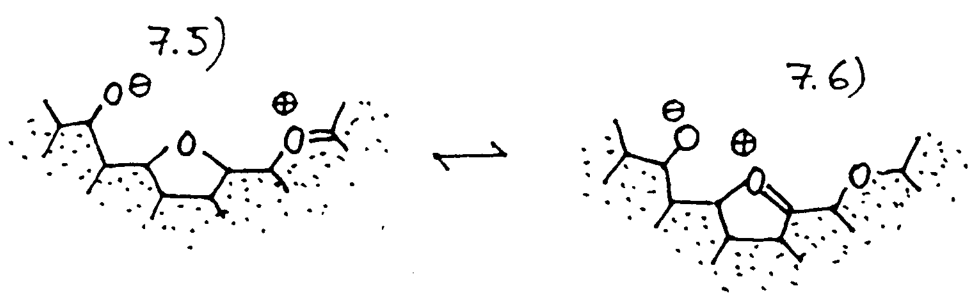

Nach dem erfindungsgemäßen Verfahren eignen sich nur solche Fullerene oder Heterofullerene zur Herstellung der Spheren, die eine bestimmte Mindestgröße haben. Die Mindestgröße der eingesetzten Fullerene ist abhängig von dem Verteilungsmuster der Pentacyclen auf der Oberfläche des Fullerens.

Hat ein einzelner Pentacyclus eines Fullerens einen Mindestabstand von dem zunächst benachbarten Pentacyclus des Fullerens entsprechend Formel (8) oder Formel (9), kann sich aus dem einzelnen Pentacyclus unter den erfindungsgemäßen Reaktionsbedingungen eine entsprechend Porphin-5 dekorierte Öffnung in dem Fulleren gemäß Formel 3.8) bilden.

8)

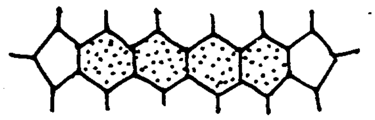

9)

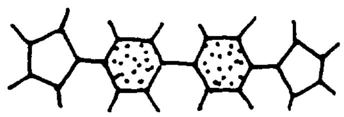

Diejenigen Heterofullerene, die neben Kohlenstoff Bor und Stickstoff enthalten und die einen oder mehrere Pentacyclen enthalten, die einen Mindestabstand entsprechend Formel (8) oder (9) haben, entwickeln je Pentacyclus ebenfalls eine Porphin-5-Öffnung.

Enthalten Fullerene oder CBN-Heterofullerene Pentacyclen, die in der in Formel (10) gezeigten Anordnung als Pentacyclenpaar vorkommen, und entspricht der Abstand zu den benachbarten Pentacyclenpaaren mindestens den Abständen, wie

10)

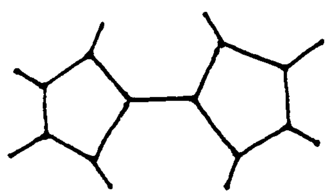

in den Formeln (11) bis (16) dargestellt ist, entsteht unter den erfindungsgemäßen Reaktionsbedingungen aus jedem Pentacyclenpaar des entsprechenden Fullerens/CBN-Heterofullerens eine Öffnung, die eine entsprechend Porphin-4 dekorierte Öffnung in dem Fulleren gemäß Formel 3.7) bilden.

11-16)

11)

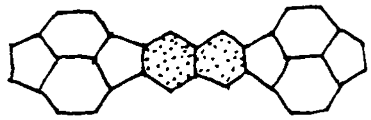

14)

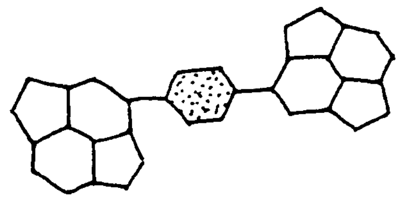

12)

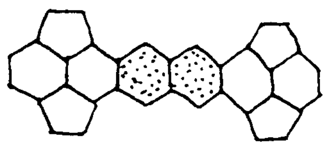

15)

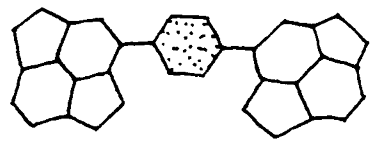

13)

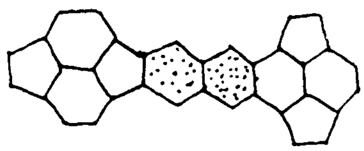

16)

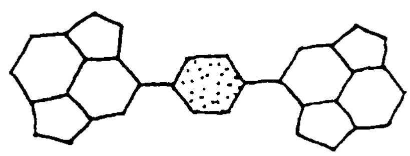

Enthalten Fullerene oder Heterofullerene Pentacyclenpaare in der in der Formel 16.1) gezeigten Anordnung, wobei n eine positive ganze Zahl größer oder gleich 1 bedeutet, können daraus bei hinreichendem Abstand der nächstliegenden Pentacyclenpaare unter den erfindungsgemäßen Reaktionsbedingungen Öffnungen mit quadratisch angeordneter APC-Dekoration entsprechend Porphin-4 gemäß Formel 3.7) entstehen. Der Abstand zwischen den Pentacyclenpaaren muß mindestens den Abstand der Pentacyclen im Paar überschreiten.

Enthalten die Fullerene oder Heterofullerene Pentacyclenpaare in einer Anordnung, wie sie in der Formel 16.2) gezeigt wird, entstehen Öffnungen mit asymmetrisch angeordneter APC-Dekoration entsprechend Porphin-4 in der in der Formel 3.7.1) gezeigten Variante. In der Formel 16.2) bedeutet n Null oder eine ganze positive Zahl. Je größer n ist, desto kleiner wird der Winkel $\beta$ bzw. desto größer wird der Winkel $\alpha$, deren Winkelposition in der Formel 3.7.1) gezeigt wird. Mit zunehmendem n, aber auch schon bei n = 0, erhalten die Öffnungen daher eine mehr rechteckige bzw. flacher elliptische Gestalt. APC-Dekorationen die bezüglich der Winkelsymmetrie von der höchsten Symmetrie abweichen, werden in der hier benutzten Nomenklatur durch das Synonym "Asym." gekennzeichnet, das vor dem Synonym "Porphin" genannt wird. Der Abstand der Pentacyclenpaare entsprechend Formel 16.2) muß mindestens den Abstand der Pentacyclen im Paar überschreiten und muß aber darüber hinaus mindestens 4 linear anellierte Hexagone betragen.

16.1)

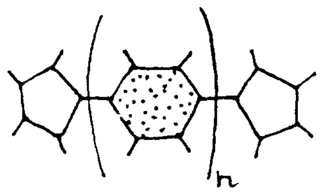

16.2)

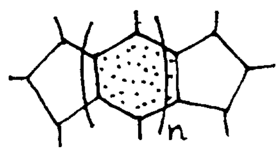

Enthalten Fullerene oder Heterofullerene Pentacyclen, die in der in der Formel (17) gezeigten Anordnung als Pentacyclentrimere vorkommen, und übersteigt der Abstand zu den

17)

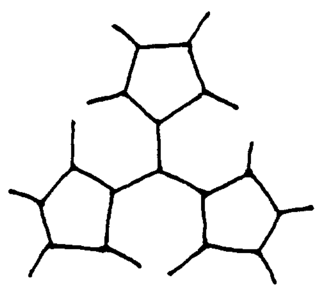

benachbarten Pentagontrimeren mindestens den Abständen, wie sie in den Formeln (18) und (19) dargestellt sind, entsteht unter den erfindungsgemäßen Reaktionsbedingungen aus jedem

18)

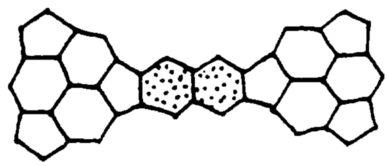

19)

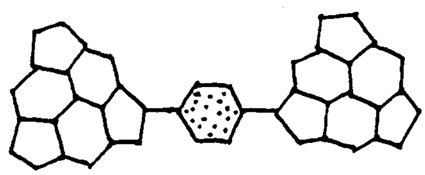

einzelnen Pentacyclentrimeren des betreffenden Fullerens bzw. Heterofullerens eine Öffnung, die eine entsprechend Porphin-3 dekorierte Öffnung in dem Fulleren gemäß Formel 3.6) bildet, in der also mindestens 3 Heteropentacyclen vorhanden sind.

Enthalten Fullerene oder Heterofullerene Pentacyclentrimere in der in Formel 20) gezeigten Anordnung, wobei n = 0 oder eine ganze positive Zahl bedeutet, können daraus bei hinreichendem Abstand der nächstliegenden Pentacyclentrimere unter den erfindungsgemäßen Reaktionsbedingungen Öffnungen mit symmetrisch angeordneter APC-Dekoration entsprechend Porphin-3 gemäß Formel 3.6) entstehen. Der Abstand zwischen den Pentacyclentrimeren muß mindestens den Abstand der Pentacyclen im Pentacyclentrimer überschreiten.

Enthalten Fullerene oder Heterofullerene Pentacyclentrimere in einer Anordnung, wie sie in der Formel 21) gezeigt wird, entstehen unter den erfindungsgemäßen Reaktionsbedingungen ebenfalls Öffnungen in den Fullerenen mit symmetrisch angeordneter APC-Dekoration entsprechend Porphin-3 gemäß Formel 3.6). In der Formel 21) hat n den Wert null oder eine ganze positive Zahl. Der Abstand zwischen den Pentacyclentrimeren muß dazu a) mindestens den Abstand der Pentacyclen im Pentacyclentrimer überschreiten und b) mindestens den Abstand zweier anellierter Hexagone entsprechen.

20)

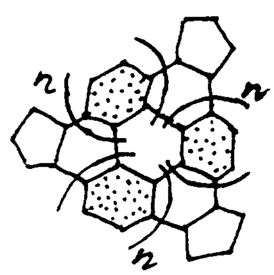

21)

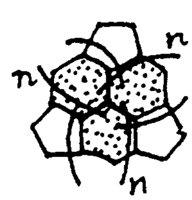

Sofern die Pentacyclentrimeren in asymmetrischer Anordnung vorliegen, wie beispielhaft anhand der Formel 22) gezeigt wird, bilden sich unter den erfindungsgemäßen Bedingungen ebenfalls Porphin-3-Öffnungen.

Dabei sind die APC der Porphine in ungleichen Winkeln zueinander angeordnet, wie die Formel (3.61) zeigt. Diese auch bezüglich der Winkel asymmetrische Anordnung der APC werden ebenfalls durch das Synonym "Asym. Porphin" gekennzeichnet. Damit sich aus diesen Pentacyclentrimeren ebenfalls isolierte Porphinöffnungen bilden können, muß der Mindestabstand zu dem nächstliegenden Pentacyclus bzw. Pentacyclengruppe größer sein als der größte Abstand der Pentacyclen voneinander innerhalb des Pentacyclentrimeren.

22)

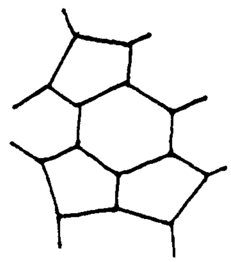

Die aus den Fullerenen oder Heterofullerenen erhaltenen Spheren behalten die Symmetrieeigenschaften der Fullerene. So bilden sich aus symmetrischen Fullerenen des ikosaedersymmetrischen Typs bzw. des oktaedersymmetrischen Typs bzw. des tetraedersymmetrischen Typs ikosaeder- bzw. oktaeder- bzw. tetraedersymmetrische Spheren. Die ikosaedersymmetrischen Spheren enthalten 12 Porphin-5-, die oktaedersymmetrischen Spheren 6 Porphin-4- oder 6 Asym. Porphin-4- und die tetraedersymmetrischen Spheren enthalten 4 Porphin-3-Öffnungen.

Durch die erfindungsgemäßen Umsetzungen der Fullerene werden jeweils die Ecken der Fullerene entfernt und durch die APC-dekorierten Öffnungen ersetzt. Als topographischer Effekt ergibt sich daraus auch eine der sphärischen Form angenäherte Gestalt als es bei den Fullerenen überhaupt möglich ist.

Neben den erwähnten hoch symmetrischen Fullerenen ist eine Reihe von Fullerenen bekannt, die z. B. einzelne Pentacyclen neben Pentacyclenpaaren oder Pentacyclentrimeren in symmetrischer Verteilung enthalten; aus diesen Fullerenen können Spheren hergestellt werden, die Porphin-5 dekorierte Öffnungen neben porphin-4- oder porphin-3-dekorierten Öffnungen enthalten. Ähnliches kann auch gelten für asymmetrische Fullerene und den daraus generierten Spheren, deren Öffnungen ebenfalls mit unterschiedlichen Porphinen dekoriert sein können.

Eine herausragende Rolle unter den Fullerenen wird den rohrförmigen Fullerenen zugesprochen. Diese bestehen aus einem langen zylindrischen Mittelteil aus reinem Hexagonnetzwerk, das auf beiden Seiten durch zwei mehr oder weniger symmetrische Halbspheren aus dichtem Hexagon- und Pentagonnetzwerk verschlossen sind. Bei der erfindungsgemäßen Umsetzung können die Halbspheren ebenfalls mit APC-dekorierten Öffnungen ausgestattet werden. Bei hinreichend lang andauernder Reaktionsführung können die Halbspheren völlig vergast werden, so daß die APC-dekorierten Öffnungen schließlich den Durchmesser des zylindrischen Mittelteils erhalten.

Obwohl die Frage noch ungeklärt ist, ob es sich bei den neuen kugelförmigen Kohlenstoffpartikeln uni Fullerene handelt, und ob nur Pentagone und Hexagone oder auch andere Strukturen an der molekularen Struktur dieser Partikel beteiligt sind, lassen sich auch diese Partikel mit APC-dekorierten Öffnungen versehen.

Sowohl die kugelförmigen Kohlenstoffpartikel, deren Herstellung in der Zeitschrift Nature, Vol. 359 vom 22.10.1992, Seiten 707 bis 709, beschrieben wird, als auch die rohrförmigen Fullerene zum Teil bestehen aus einer Mehrzahl ineinandergeschachtelter Kohlenstoffschichten. Auch derartige Partikel bzw. Moleküle lassen sich nach dem erfindungsgemäßen Verfahren mit Öffnungen versehen, die je nach Reaktionsführung und -dauer nicht nur die äußere Kohlenschicht betreffen, sondern auch beliebig viele darunter liegende Kohlenstoffschichten. Die Formel 23) zeigt den Ausschnitt eines erfindungsgemäß behandelten mehrschaligen Kohlenstoffpartikels im Schnitt, wobei die schwarzen Linien die Schichten aus unversehrtem Kohlenstoffnetzwerk im Schnitt zeigen, und wobei X die Position der an den Rändern der Öffnungen entstandenen APC kennzeichnen.

23)

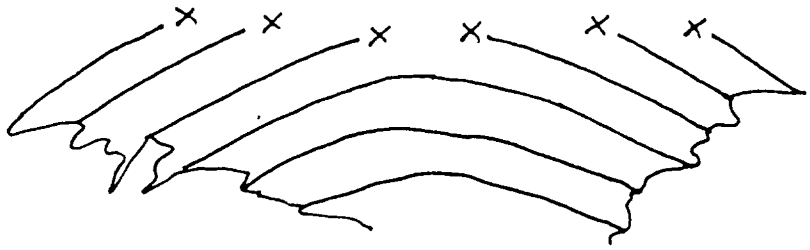

Wie aus dem bisher Ausgeführten hervorgeht, können die erfindungsgemäß erzeugten und dekorierten Öffnungen bei hinreichender Oxidations- bzw. Reaktionsdauer beliebig groß gestaltet werden. Besonders bei großen Molekülen der Fullerene, wie sie z. B. von den Kohlenstoffröhren dargestellt werden, wachsen die durch die erfindungsgemäße Reaktion verursachten Öffnungen im Reaktionsverlauf zu größeren symmetrischen oder asymmetrischen Öffnungen zusammen, deren APC-Dekoration sich schwerlich mit herkömmlicher Analysentechnik bezüglich der o. g. Nomenklatur klassifizieren läßt. Bestimmte Eigenschaften wie z. B. ihre katalytische Aktivität bezüglich Elektrodenkatalyse oder Katalyse von RedoxReaktionen sind ein Hinweis auf das Vorhandensein der für ihre Elektronenübertragungsfähigkeit bekannten APC-Öffnungsdekoration.

Kleine Fullerene werden nach dem erfindungsgemäßen Verfahren in der Regel zum größten Teil in gasförmige Bruchstücke zerlegt. Gelegentlich lassen sich auf den Trägern aspherische Fullerenfragmente in Form von Derivaten polycyclischer Aromaten, wenn kleinere Fullerene wie $C_{60}$ oder $C_{70}$ der erfindungsgemäßen Behandlung unterworfen werden, ermitteln.

Die erfindungsgemäße Reaktion läßt sich auch ohne Schwierigkeiten mit den sogenannten endohedralen Fullerenkomplexen durchführen, die dadurch in die entsprechenden endohedralen Spherenkomplexe überführt werden.

Die endohedralen Fullerenkomplexe enthalten innerhalb des Hohlraumes, den jedes Fulleren umschließt, eine Funktion. Gleiches gilt für die endohedralen Spherenkomplexe.

Anstelle der Fullerene bzw. Heterofullerene lassen sich auch deren Derivate als Edukte zur Herstellung von Spheren nach dem erfindungsgemäßen Verfahren einsetzen. Unter den erfindungsgemäßen Reaktionsbedingungen werden nämlich aus diesen Derivaten die betreffenden Fullerene bzw. Heterofullerene wieder zurückgebildet, die dann in die beschriebenen Spheren umgesetzt werden. Das gilt auch für die entsprechenden Fulleroide; das sind Fullerene mit aufgeweiteter Hohlraumstruktur, die ebenfalls unter diesen Reaktionsbedingungen die betreffenden Fullerene zurückbilden.

Es ist zusammenfassend anzumerken, daß die gemachten Aussagen Fullerene, Fulleroide, Spheren, endohedrale Fullerenkomplexe, Fullerenderivate und mehrschichtige Fullerene und spherische mehrschichtiger Kohlenstoffpartikel als geeignete Edukte für die erfindungsgemäße Spherensynthese einzusetzen uneingeschränkt auch für die Eignung der entsprechenden Heteroanaloga der genannten Stoffe, soweit sie die Atome Bor, Stickstoff oder Kohlenstoff enthalten, gelten.

Ergänzend zu der erfindungsgemäßen Reaktionsführung sind noch einige Ergänzungen nachzutragen. Als Träger für die Fullerenedukte eignen sich auch feuerfeste Materialien wie gebrannter Ton, Porzellan, pyrogenes Aluminiumoxid, Siliziumdi- und Titandioxid, Magnesiumoxid, Quarzglas, Siliziumcarbid, Asbest, Bornitrid, Borid-, Nitrid-und Carbidkeramiken, Bor- Kohlenstoffverbindungen, die eine gute Resistenz gegen oxidierende Medien sowie die in Frage kommenden Stickstoff- und Chalkogenverbindungen haben.

Die verwendeten Reaktionsgase, mit denen die Fullerene umgesetzt werden, werden rein oder mit Inertgasen wie Stickstoff oder Edelgasen verdünnt zur Anwendung gebracht.

Die notwendige Kontaktzeit der Gase mit den fullerenbeschichteten Trägern muß von Fall zu Fall ermittelt werden, da die Fullerene je nach Größe und Pentagonverteilung unterschiedliche Reaktivität gegenüber dem Oxidationsmittel zeigen. Die Bor- und/oder stickstoffhaltigen Heterofullerene zeigen generell eine geringere Reaktionsfähigkeit als die Fullerene. Die Zuführ des sauerstoffhaltigen oxidierenden Gases wird vorzugsweise abgebrochen, wenn die hinreichende Menge Von Oxidationsprodukten, also z. B. $CO_2$, CO, COS, freigesetzt worden sind. Anschließend wird bei erhöhter Temperatur das Gas oder Gasgemisch eingeleitet, das reaktive Stickstoff- oder Chalkogenverbindungen einzeln oder im Gemisch enthält. Außer den Wasserstoff enthaltenden Verbindungen von Kohlenstoff Stickstoff und Chalkogenen eignen sich auch Blausäure, Amine, Dicyan oder Harnstoff und sonstige stickstoffreiche niedermolekulare Verbindungen, die reaktiven Stickstoff in der Hitze abspalten bzw. bilden können.

Als schwefelabgehende Gase eignen sich auch Schwefelkohlenstoff Mercaptane, Elementarschwefel und sonstige schwefelreiche Verbindungen, wie auch Schwefelhalogenide.

Als selenabgebende Verbindungen können auch elementares Seien oder selenabspaltende selenreiche Verbindungen zur Anwendung kommen.

Als sauerstoffliefernde Verbindungen für den Reaktionsschritt der Dekoration der Öffnungen mit APC kann neben Sauerstoff auch Stickoxide oder andere sauerstoffreiche Substanzen zur Anwendung gebracht werden. Elementarer Sauerstoff aber auch andere Oxidationsmittel, können zur Redox-Konditionierung bei niedriger Temperatur der Dekoration der Öffnungen angewendet werden, daneben natürlich auch Reduktionsmittel sonstiger Art.

Gemische der genannten Verbindungen oder Verbindungen, die die Elemente im Gemisch enthalten wie z. B. Schwefeldioxid, Selendioxid, können eingesetzt werden.

Gelangen die Elemente C, O, S, N, Se in Gestalt der o. g. Gase nicht für sich allein, sondern im Gemisch zur Reaktion unter den erfindungsgemäßen Reaktionsbedingungen, so bilden sich Spheren, deren Öffnungen mit APC-Funktionen dekoriert sind, bei denen die Funktion X gemäß den Formeln (1) bis (3) innerhalb einer Öffnung nicht nur auf eine Atomsorte beschränkt ist, sondern für mehr als eine der genannten Atomsorten stehen kann.

Die Dauer der Behandlung mit Wasserdampf Kohlendioxid, Methan, Ammoniak, Schwefelwasserstoff Selenwasserstoff oder einem anderen der reaktiven C, O, N, S oder Se enthaltenden Gase oder Gasgemisch muß ebenfalls von Fall zu Fall ermittelt werden, mit dem Ziel, eine optimale Spherenausbeute zu erhalten. Dabei ist zu berücksichtigen, daß eine zu lange Behandlung mit einigen Gasen zu einem Ausbeuteverlust führen kann, weil die entstandenen Spheren durch weitere Reaktion mit den genannten Gasen durch Abbaureaktionen unter Abspaltung von CO oder $CH_4$ oder $B_2O_3$ bis zum Verlust ihrer sphärischen Molekültopographie abgebaut werden können.

F oder S, die auf diese Weise abgebaut wurden, wobei der Abbau aber noch nicht bis zum Verlust der sphärischen Molekülgestalt geführt hat, das heißt daß deren Öffnungen eine unerwünschte Größe erhalten haben, können unter Anwendung des erfindungsgemäßen Verfahrens derart mit reaktiven Gasen umgesetzt werden, bis die Spheren die gewünschte Öffnungsgröße erhalten haben. Es ist aber nicht möglich, die Öffnungen der Spheren derart wieder völlig zu Fullerenen zu schließen.

Bei der Verwendung von Gasen oder Gasgemischen nach dem erfindungsgemäßen Verfahren zur Herstellung aus Fullerenen oder Spheren, deren Öffnungen mit APC-Dekoration versehen werden sollen, muß auf das geeignete Verhältnis von den Gasbestandteilen H, O, N. S, oder Se zu Kohlenstoff geachtet werden: Enthält das Gas einen hohen C- und/oder N-Anteil, verkleinert sich die Öffnung durch Kondensation C-und/oder N-haltigen Hexagon-und APC-Netzwerks im Minimum auf die Größe von Subporphin, Porphin oder Superporphin.

Enthält das Gas einen hohen Sauerstoffanteil, vergrößert sich die Öffnung durch Verflüchtigung der Kohlenstoffbestandteile des Öffnungsrandes als Kohlenstoffoxide. Enthält das Gas einen hohen Schwefelanteil, vergrößert sich die Öffnung durch Verflüchtigung der Kohlenstoffanteile des Öffnungsrandes als Schwefelkohlenstoff.

Auch bei Verzicht auf den Ausgasungsschritt gelingt die Behandlung mit Stickstoff-und Chalkogenverbindungen z. B. dann mit gutem Erfolg, wenn die Verbindungen Methylamin, Methan, Methanol, Methylmercaptan oder Methylselenol bei Temperaturen von 700 °C bis 1000 °C und anschließender Abkühlung in der Gegenwart der betreffenden genannten Gasphase geschieht. Wirtschaftlich kann auch sein die Einstellung geeigneter Verhältnisse der Elemente zueinander in der reaktiven Gasphase durch Mischung geeigneter Gase wie dies beispielhaft hier gezeigt wird.

0) 1 Mol Methan + 2 Mol Wasserstoff
1) 1 Mol Ethen + 2 Mol Wasser oder
2) 1 Mol Ethen + 2 Mol Schwefelwasserstoff oder
3) 1 Mol Ethen + 2 Mol Selenwasserstoff oder
4) 1 Mol Ethen + 2 Mol Ammoniakgas oder
5) 1 Mol Ethan + 1 Mol Wasser oder
6) 1 Mol Ethan + 1 Mol Schwefelwasserstoff oder
7) 1 Mol Ethan + 1 Mol Selenwasserstoff oder
8) 1 Mol Ethan + 1 Mol Ammoniak oder
9) 1 Mol Cyanwasserstoff + 2 Mol Wasser oder
10) 1 Mol Cyanwasserstoff + 2 Mol Ammoniak oder
11) 1 Mol Cyanwasserstoff + 2 Mol Schwefelwasserstoff.

Die jeweils gewählten Gasgemische werden vorzugsweise in Verdünnung mit einem Inertgas wie Argon oder Stickstoff angewendet oder auch Wasserstoff.

Anstelle der Gasgemischkomponenten Wasserdampf Schwefelwasserstoff Selenwasserstoff oder Ammoniak können auch andere vergasungsaktive Komponenten wie Kohlendioxid, Distickstoffoxid oder Schwefeldioxid verwendet werden. Auch können alle der beispielhaft genannten Vergasungsmittel im Gemisch eingesetzt werden.

Bei den beispielhaft genannten Mischungen der reaktiven Gase 1) bis 11) oder dem Ersatz der Chalkogen- oder Stickstoffwasserstoffkomponenten in diesen Gemischen durch entsprechende Anteile Wasserstoff oder weitere Chalkogenverbindungen können vorhandene unerwünschte Sauerstoffunktionen aus den Spherenöffnungen abgebaut werden unter Hinterlassung von N-haltigen APC und C-haltigen APC. N-haltige APC werden nur bei N-haltigen Reaktivgasen erhalten oder bei N-haltigen Spheren. Die Dekoration mit S- oder Se-haltigen APC ist unter den hohen Behandlungstemperaturen unvollständig. Fast überhaupt nicht entstehen O-haltige APC.

Die Reaktivität der bei 700 °C bis 1000 °C ausgegasten und unter Inertgas bis unter 700 °C abgekühlten undekorierten Spheren ist allerdings gegenüber den weniger reaktiven Gasen wie Wasser, Schwefelwasserstoff Selenwasserstoff und Ammoniak bereits so stark eingeschränkt, daß es vorteilhafter ist, reaktivere Gase einzusetzen wie Schwefel, Dischwefeldichlorid, Selen, Diselendichlorid, Stickstoffplasma, Sauerstoff ($O_2$). Die genannten Reaktanten erfordern ganz unterschiedliche Reaktionstemperaturen zur Erzielung der gewünschten Reaktion mit den undekorierten Spheren, um die APC-Dekoration zu erzielen:

| | |
|---|---|
| Schwefel: | 200 °C bis 450 °C |
| Dischwefeldichlorid: | 100 °C bis 300 °C |
| Selen: | 220 °C bis 500 °C |
| Diselendichlorid: | 100 °C bis 300 °C |
| Stickstoffplasma: | 200 °C bis 500 °C |
| Sauerstoff: | 50 °C bis 150 °C |

Die genannten Temperaturwerte bedeuten nicht, daß außerhalb der genannten Temperaturbereiche keine APC-Dekoration entsteht; sie bezeichnen jedoch den Temperaturbereich, innerhalb dessen eine möglichst geringe Nebenreaktionsrate bei hinreichender Reaktionsgeschwindigkeit vorkommt. Die Reaktion mit Stickstoffplasma führt zu einer APC-Dekoration im maximal oxidierten Zustand, d. h., alle vorhandenen Pyrrolringe in den Öffnungen liegen nicht als =NH -Funktionen vor, sondern in der wasserstoffreien Form als =N- -Funktionen. Die einfachen Porphinformen, in der also Pyrrole mit =NH - Funktionen neben Pyrrolen mit =N- -Funktionen vorkommen, erreicht man, wenn das Stickstoffplasma geringe Mengen Ammoniak enthält.

Mit Furan dekorierte Öffnungen lassen sich in der reduzierten Form in fluiden Medien säurekatalysiert z. B. durch Chlorwasserstoff in der Gegenwart von Schwefelwasserstoff bzw. Selenwasserstoff in die betreffenden Thiophen- bzw. Selenophendekorierten Öffnungen umsetzen. Auf diese Weise lassen sich auch leicht gemischte APC-dekorierte Öffnungen erzeugen. Die Umwandlung der Spheren mit furandekorierten Öffnungen in solche mit thiophen- oder selenophendekorierten Öffnungen bzw. nur teilweise mit Furan dekorierten Öffnungen, also auch gemischt APC-dekorierten Öffnungen gelingt auch in der Gasphase durch Umsetzung der Spheren mit Selen- oder Schwefelwasserstoff oder Ammoniak einzeln oder im Gemisch durch Erwärmen auf Temperaturen bis 500 °C.

Die Vielfalt der neuartigen mit APC dekorierten Öffnungen enthalten Spheren, die mit dem erfindungsgemäßen Verfahren hergestellt werden kann, läßt sich durch die Formeln 24) bis 26) beschreiben.

24)

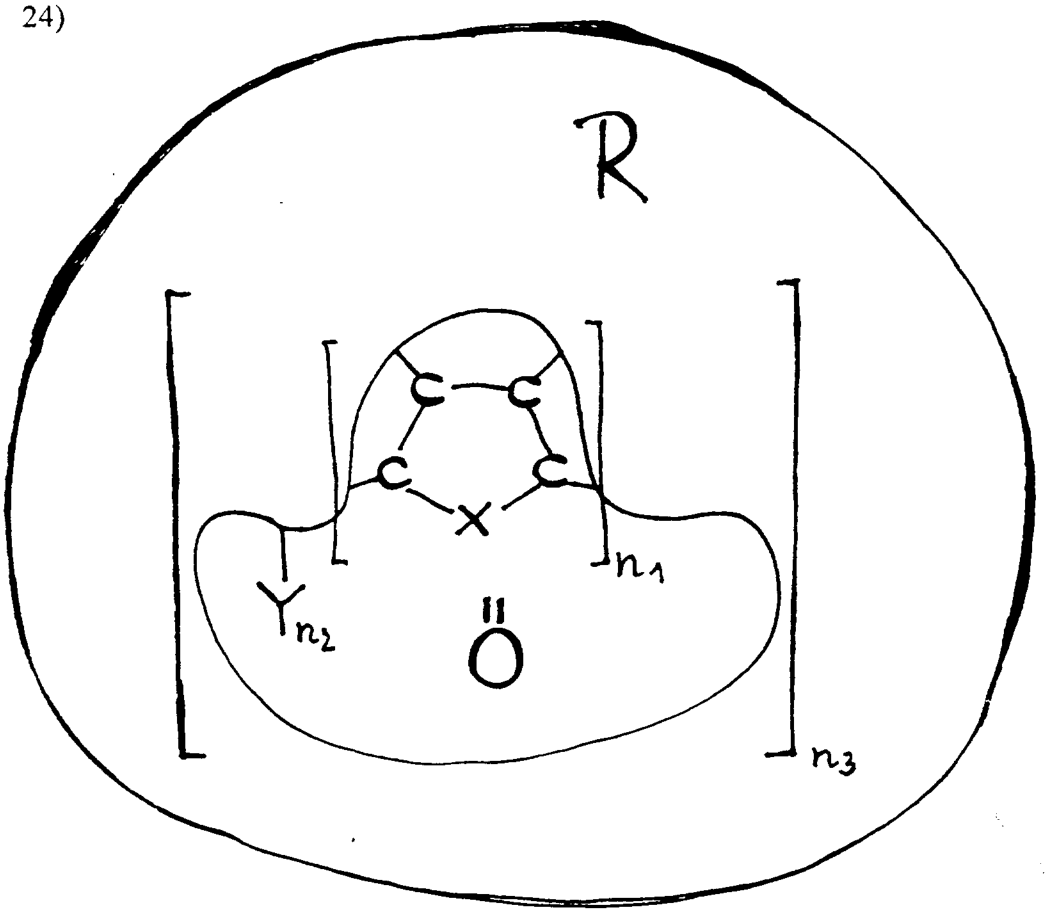

Die Formel 24) zeigt die erfindungsgemäßen Spheren als Derivate spherischer Porphin-n-Oligomerer, wobei n eine ganze Zahl größer gleich 3 bedeutet. In der Formel 24) haben die Symbole folgende Bedeutung:

R bedeutet Hexagonnetzwerk aus dreifach koordiniertem Kohlenstoff oder aus dreifach koordiniertem Kohlenstoff dreifach koordiniertem Stickstoff und dreifach koordiniertem Bor, das $n_3$ Öffnungen Ö enthält.

Ö bedeutet eine Öffnung in dem Hexagonnetzwerk von R.

X bedeutet O, $O^{\oplus}$, S, $S^{\oplus}$, Se, $Se^{\oplus}$, NH, N, $CH_2$, CH, CO.

Y hat die in den Formeln 0.1) bis 0.6) beschriebene Bedeutung.

$n_1$ bedeutet eine ganze Zahl größer oder gleich 3.

$n_2$ bedeutet null oder eine ganze Zahl größer null.

$n_3$ bedeutet eine ganze Zahl größer oder gleich 2.

Die Formel 25) zeigt die erfindungsgemäßen Spheren als Derivate spherischer Porphin-n-Oligomerer, wobei n eine ganze Zahl größer gleich 3 bedeutet, und in den Porphin-n-Funktionen jeweils mindestens eine Funktion komplex gebunden ist. In der Formel 25) haben die Symbole folgende Bedeutung:

Die Symbole R, Ö, X, Y, $n_1$, $n_2$, $n_3$ haben die gleiche Bedeutung wie in der Formel 24).

M bedeutet eine von den Funktionen X und/oder Y komplex gebundene anorganische oder organische oder gemischt organisch-anorganische Komponente.

$n_4$ bedeutet eine ganze Zahl größer oder gleich 1.

25)

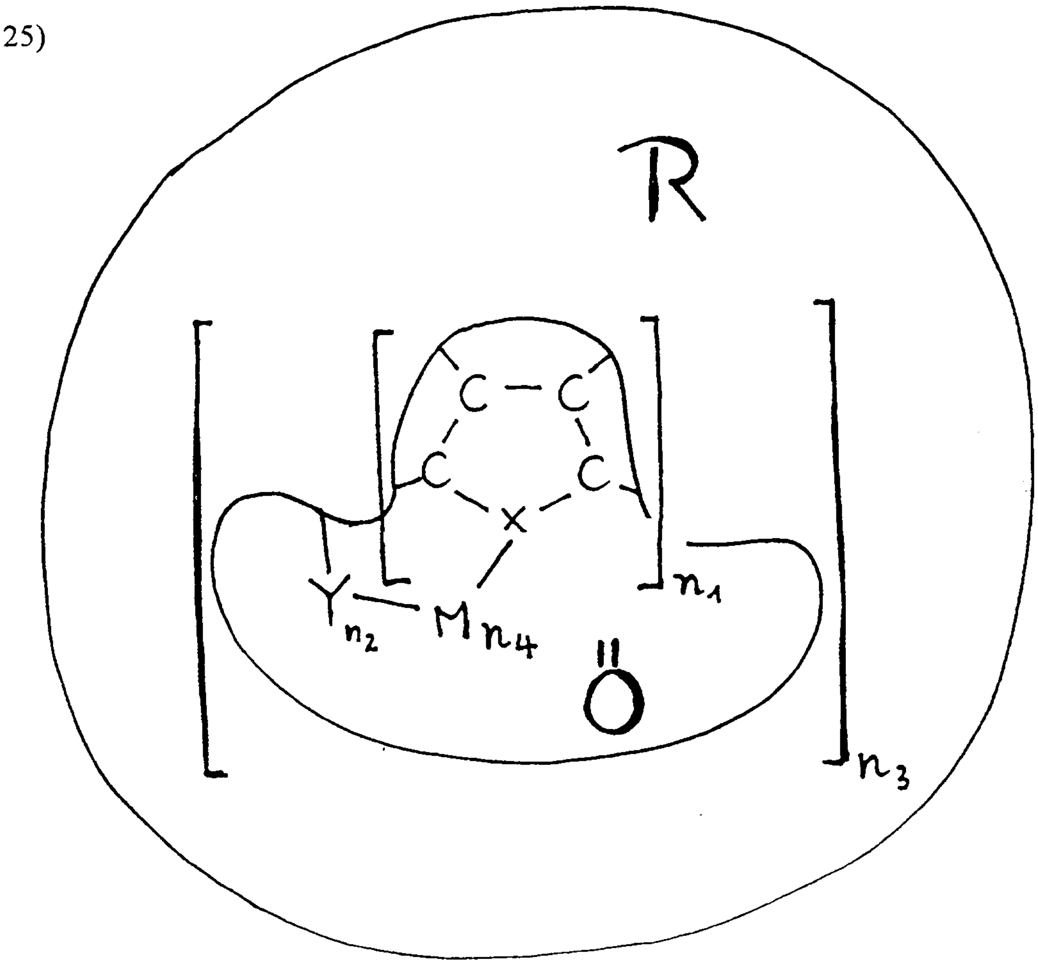

Die Formel 26) zeigt die erfindungsgemäßen Spheren als Derivate spherischer Porphin-n-Oligomerer, wobei n eine ganze Zahl größer gleich 3 bedeutet, und in den Spheren sowohl komplexierte als auch nicht komplexierte Porphin-n-Funktionen vorkommen. In der Formel 26) haben die Symbole folgende Bedeutung: Die Symbole R, Ö, X, Y $n_1$, $n_2$, $n_4$ haben die gleiche Bedeutung wie in den Formeln 24) und 25).

$n_5$ bedeutet eine ganze Zahl größer oder gleich 1.

Nochmals sei darauf hingewiesen, daß unter dem Synonym Porphin hier alle diejenigen in das aromatische Hexagonnetzwerk eingebetteten Öffnungen verstanden werden, die dreiseitig mit dem aromatischen Netzwerk anellierte APC aus vier 3-fach koordinierten Kohlenstoffatomen und einer $CH_2$- oder CH- oder einer Heterofunktion enthalten. Dabei hat die Heterofunktion oder die $CH_2$- oder CH-Funktion die in den Formeln 24) bis 26) mit X gekennzeichnete und erläuterte Bedeutung.

26)

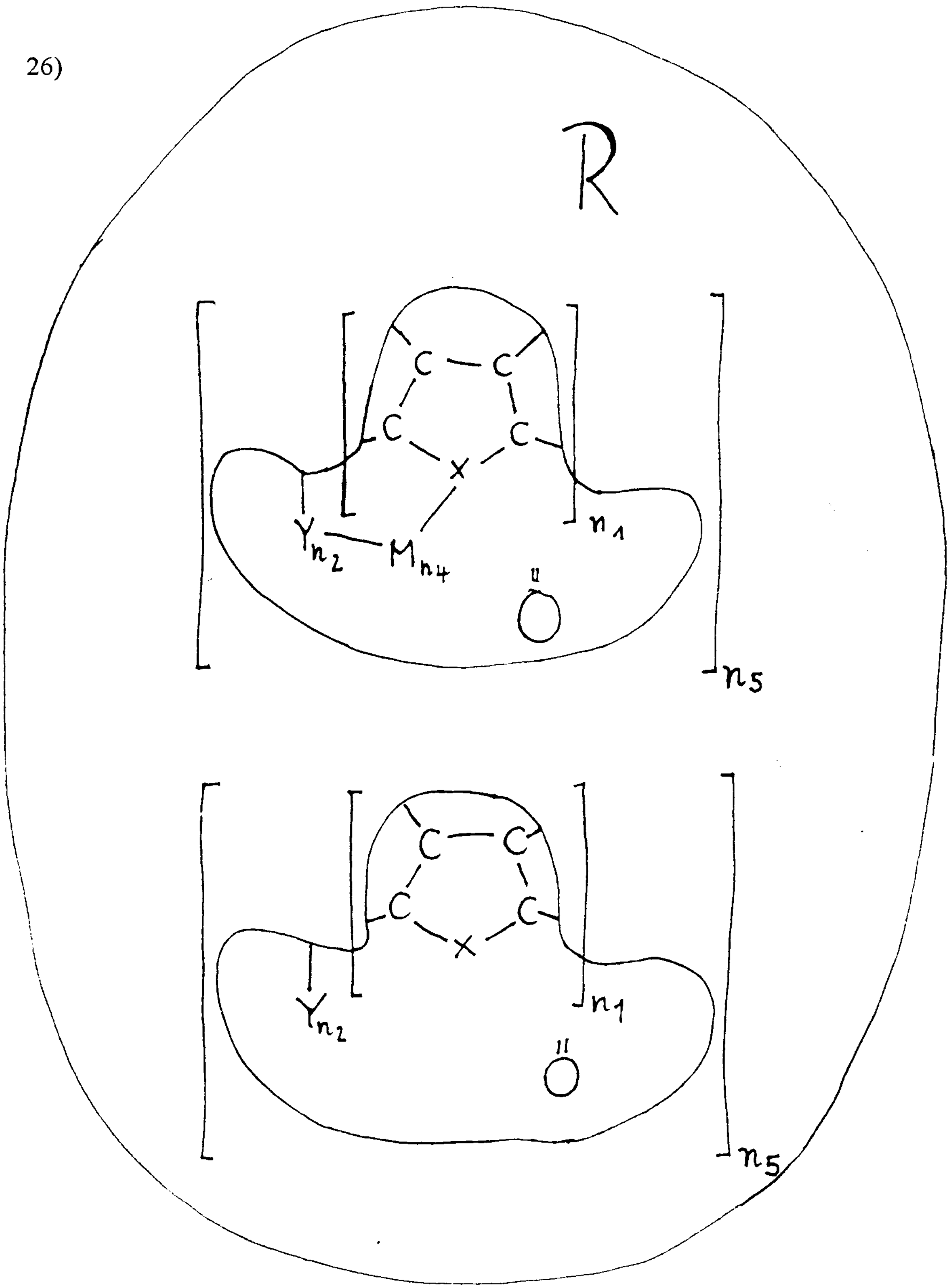

Die Formeln 1) bis 7) sowie 24) bis 26) stellen bezüglich der Anzahl der dargestellten APC, die in den Formeln 24) bis 26) durch das Symbol $n_1$ repräsentiert wird, einen Idealzustand dar. In einigen Fällen fehlt den anellierten Porphin-3- oder Porphin-4- oder Porphin-5-ähnliche dekorierten bzw. symmetrieanalogen Öffnungen bezüglich der Anellierung ein APC wie beispielhaft anhand der Formeln 27) und 27.1) anhand der Porphin-5- analog anellierten Öffnungen gezeigt wird. Auch diese Verbindungen werden aufgrund ihrer porphinanalogen Anellierung mit dem Synonym Porphin-3, Prophin-4 oder Porphin-5 bezeichnet.

27)

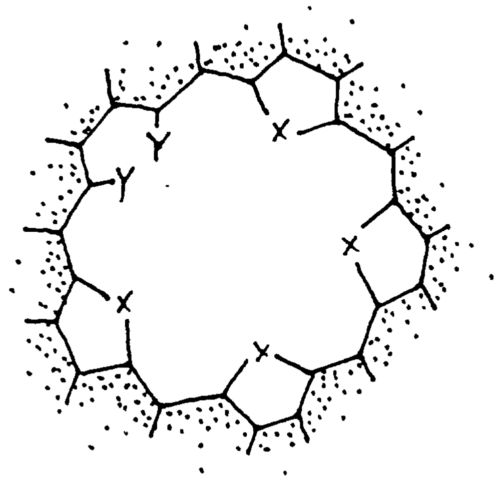

27.1)

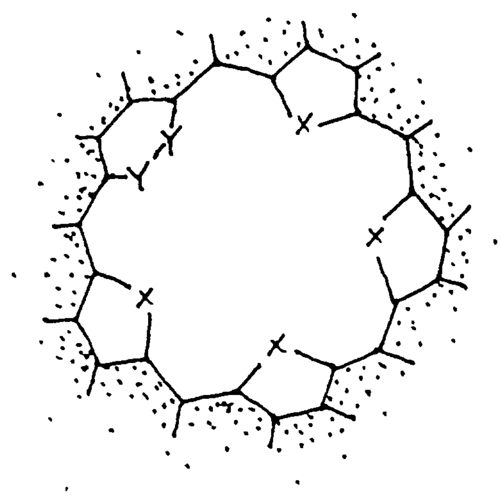

In der hier verwendeten Nomenklatur wird die fehlende APC-Funktion oder die fehlenden APC-Funktionen dadurch gekennzeichnet, daß die fehlende oder fehlenden APC durch mit Schrägstrich durchgestrichene betreffende Ziffern in der Zahlengruppe b gekennzeichnet sind. Die in der Formel 27.2) beispielhaft gezeigte APC-dekorierte Spherenöffnung ist dementsprechend als

Porphin-5-1/1/1-3S

zu bezeichnen.

27.2)

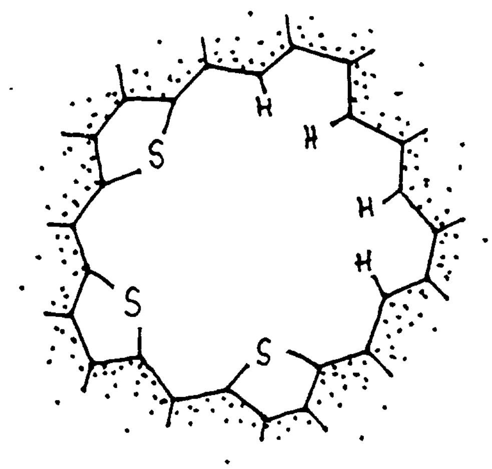

Die Gaszusammensetzung, um die Öffnungen der Spheren zu verkleinern, sollte vorzugsweise so gewählt werden, daß die Kondensation der Kohlenstoff- und/oder Stickstoff-und/oder Borfunktionen hinreichend langsam und damit geordnet in der beabsichtigten Weise geschieht. Das geschieht vorzugsweise durch die Einstellung geringer Reaktivgasgehalte von vorzugsweise kleiner gleich 2 Vol% und möglichst konstanter Reaktionstemperaturhaltung vorzugsweise bei 900 °C. Setzt man unter diesen Bedingungen die Behandlung der Spheren fort, bilden sich die Öffnungen zu zylindrischen Fortsätzen aus Hexagonnetzwerk aus, die lückenlos unter Beteiligung von 3 bis 5 Heptagonen mit dem Hexagonnetzwerk der Spheren verknüpft sind. An entsprechend Porphin-5 anellierter Öffnungsymmetrie bilden sich zylindrische Fortsätze mit einem Umfang entsprechend 10 in Reihe anellierten Hexagonen, wie in der Formel 27.3) ausschnitthaft mit Blickrichtung in den zylindrischen Fortsatz gezeigt wird. An entsprechend Porphin-4 anellierter Öffnungssymmetrie bilden sich zylindrische Fortsätze mit einem Umfang entsprechend 8 in Reihe anellierten Hexagonen, wie in der Formel 27.4) ausschnitthaft mit Blickrichtung in den zylindrischen Fortsatz gezeigt wird. An entsprechend Porphin-3 anellierter Öffnungssymmetrie bilden sich - ausgehend von Porphin-3-1/1/1-anellierung - zylindrische Fortsätze mit einem Umfang entsprechend 6 in Reihe anellierten Hexagonen; und - ausgehend von Porphin-3-3/3/3- anellierung - zylindrische Fortsätze mit einem Umfang entsprechend 9 in Reihe anellierten Hexagonen, wie in den Formeln 27.5) und 27.6) ausschnitthaft mit Blickrichtung in die zylindrischen Fortsätze gezeigt wird. Es ist bemerkenswert, daß die Heptagone am Übergang zwischen Spherenhexagonnetzwerk und Zylinderfortsatznetzwerk genau diejenigen Positionen besetzen, die die APC in den jeweiligen porphindekorierten Öffnungen einnehmen.

Die erfindungsgemäße Methode zur Umbildung der Spherenöffnungen in zylindrische hohle Fortsätze kann sogar dazu eingesetzt werden, die Hohlräume einer Vielzahl von Spheren miteinander kommunizierend zu verbinden. Hierzu eignen sich besonders die tetraedersymmetrischen Spheren und die oktaedersymmetrischen Spheren. Zu diesem Zweck werden die betreffenden Spheren als Langmuir-Blodget-Filme auf einem Träger ein-oder mehrfach aufgetragen, wie im Beispiel 5 gezeigt wird. Anschließend werden die derart beschichteten Träger bei den oben angegebenen Bedingungen, Reaktivgasbehandlung bei 900 °C, konditioniert. Die Behandlungsdauer ergibt sich aus der Kohlenstoffaufnahme der Spherenfilme; die Behandlung wird abgebrochen, wenn die Kohlenstoffaufnahme hinreichend ist, uni nicht beabsichtigte Kohlenstoffablagerung zu vermeiden.

Jeder tetraedersymmetrische Spherushohlraum ist dabei jeweils mit 4 Zylinderfortsätzen mit 4 Nachbarspheren kommunizierend verbunden. Jeder Spherus nimmt dabei die Position eines Kohlenstoffatoms im Diamantgitter ein, wobei die Hohlzylinderfortsätze zwischen den Spheren die Positionen der kovalenten Bindungen zwischen den Kohlenstoffatomen einnehmen. Diese makroskopische Ähnlichkeit dieses Spherengitters mit dem Diamantgitter macht sich auch durch die bemerkenswerte mechanische Stabilität dieses fraktalen Filmes bemerkbar.

Im Fall der oktaedersymmetrischen Splieren sind die Spherenhohlräume jeweils mit 6 Zylinderfortsätzen mit 6 Nachbarspheren kommunizierend verbunden. Auch die Filme aus diesem Material zeichnen sich durch hohe Stabilität aus.

Bei Anwendung von Temperaturen über 1000 °C können auch bei sehr geringen Reaktivgasgehalten und Verwendung von Gasen, die vorzugsweise Kohlenstoff abscheiden, Reaktionsbedingungen erzielt werden, bei denen sich auch aus ikosaeder- und oktaedersymmetrischen Spheren Fortsätze bilden, die größere Durchmesser haben, als die in den Formeln 27.3) und 27.4) gezeigten und die auch von der zylindrischen Form abweichen können. Voraussetzung dafür ist jedoch, daß Spheren mit großen Öffnungen eingesetzt werden, wie sie z. B. in den Formeln 4.1), 4.2), 1.2), 1.3), 2.1), 2.2), 2.3), 3.1), 3.2), 3.3) oder 3.5) gezeigt werden. Mehrschichtige Spheren, die in der Formel 23) gezeigt werden, können dabei auch mehrschichtige Fortsätze bilden.

In den Formeln 27.3) bis 27.6) sind die jeweiligen Positionen der Heptagone durch Punktierung hervorgehoben.

27.3)

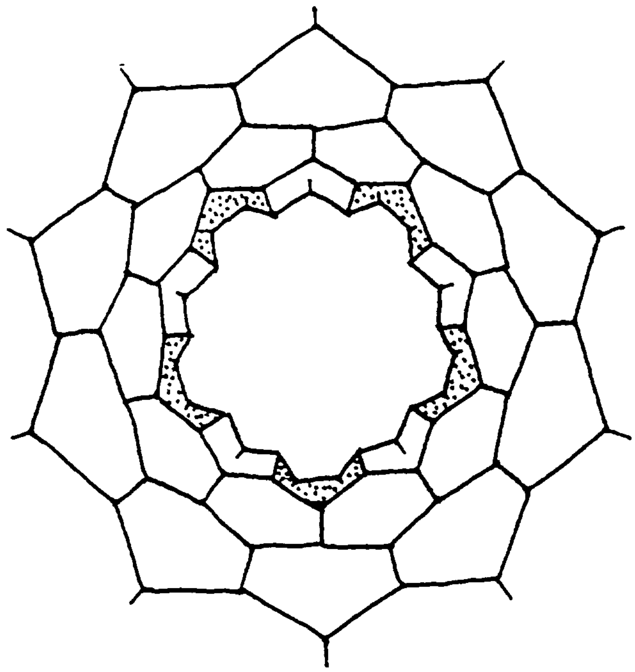

27.4)

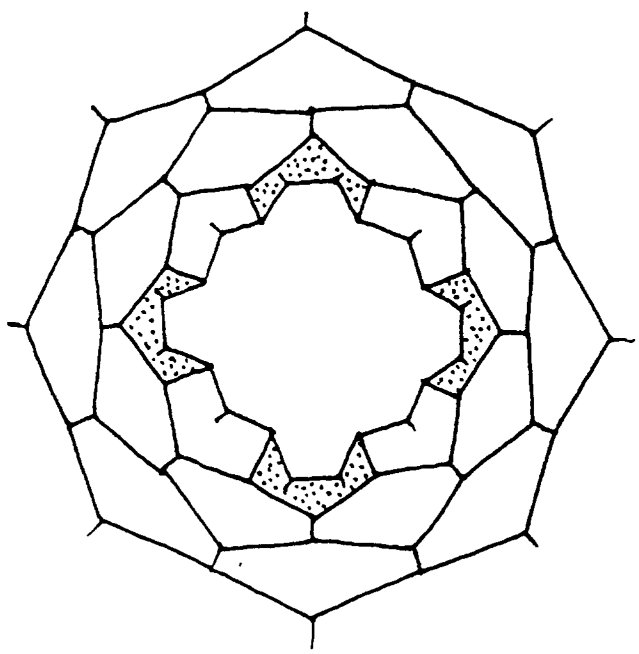

27.5)

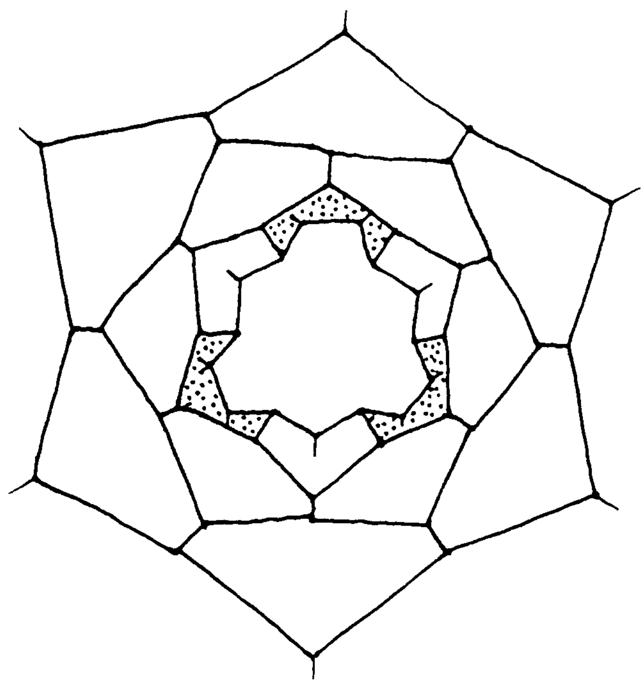

27.6)

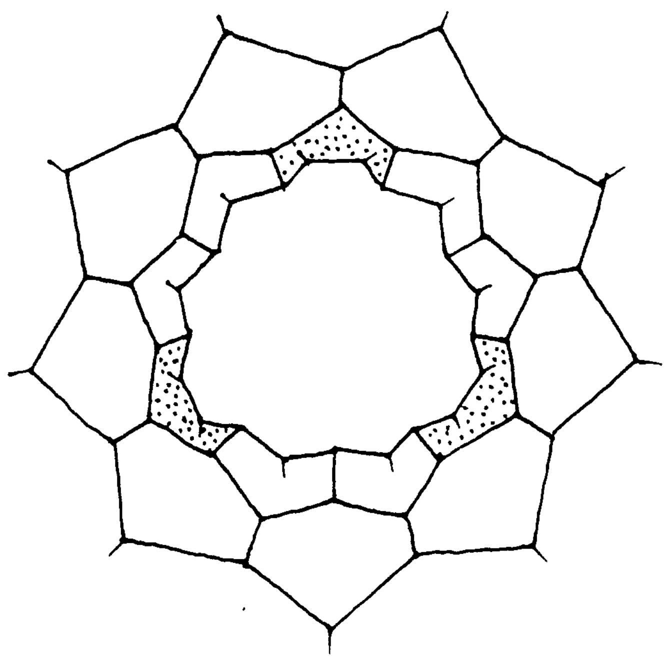

Durch die reaktiven zur Kohlenstoff- und/oder Stickstoff- und/oder Borabscheidung fähigen Gasphasen gelingt es nicht nur, die APC-dekorierten Öffnungen der Spheren zu verkleinern oder sie gar in zylindrische Fortsätze umzusetzen. Darüber hinaus werden auch Öffnungen im Hexagonnetzwerk, die sich vollständig durch Hexagonnetzwerk schließen lassen, mit dieser Methode geschlossen. Bei Verwendung kalter Plasmen als reaktive Gasphase läßt sich die Behandlungstemperatur sogar bis unter 500 °C absenken. Aus

Öffnungen im Hexagonnetzwerk, die sich vollständig durch Hexagonnetzwerk verschließen lassen, lassen sich allerdings keine zylindrischen Fortsätze generieren.

Zusammenfassend lassen sich die erfindungsgemäßen Spheren durch die Formel 27.7) kennzeichnen. Darin haben die Symbole die folgenden Bedeutungen:

R bedeutet Hexagonnetzwerk aus dreifach koordiniertem Kohlenstoff oder aus dreifach koordiniertem Stickstoff dreifach koordiniertem Bor und dreifach koordiniertem Kohlenstoff oder aus dreifach koordiniertem Kohlenstoff und zwei-und/oder dreifach koordiniertem Stickstoff das auch lückenlos mit Hexagonnetzwerk auffüllbare Ausnehmungen enthalten kann und das darüber hinaus $n_1 + n_2 + n_3$ Funktionen Ö enthält.

Ö bedeutet eine Funktion in dem Hexagonnetzwerk des Spherus, die nicht lückenlos mit gleichseitigem kohlenstoff-, bor- und/oder stickstoffhaltigem Hexagonnetzwerk ausgefüllt werden kann, und die gegebenenfalls an den Funktionen X und Y komplexierteFunktionen enthalten kann.

X bedeutet O, $O^{\oplus}$, S, $S^{\oplus}$, Se, $Se^{\oplus}$, NH, N, $CH_2$, CO als Brückenfunktion oder zwei getrennte Funktionen H H oder H O oder O O oder eine $C_3$-Gruppe, die Bestandteil anellierten dreifach koordinierten Hexagonnetzwerkes ist, wobei im letzten Fall Y ebenfalls anelliertes Hexagonnetzwerk bedeutet, das mit X anelliert ist.

Y hat die in den Formeln 01) bis 06) beschriebene Bedeutung, kann aber auch anelliertes Hexagonnetzwerk bedeuten, das auch mit X anelliert verbunden sein kann.

n bedeutet Null oder eine ganze positive Zahl

$n_1$ bedeutet Null oder eine ganze positive Zahl kleiner gleich vier

$n_2$ bedeutet Null oder eine ganze positive Zahl kleiner gleich Sechs.

$n_3$ bedeutet Null oder eine ganze positive Zahl kleiner gleich Zwölf.

In der Formel 27.7) ausgenommen sind die bereits bekannten Spheren, die durch die Konstellation X = N oder NH bei n gleich Null und bei $n_1$ gleich 4, wenn $n_2$ und $n_3$ gleich Null sind oder bei $n_2$ gleich 6, wenn $n_1$ und $n_3$ gleich Null sind oder bei $n_3$ gleich 12, wenn $n_1$ und $n_2$ gleich Null sind, gekennzeichnet sind.

27.7)

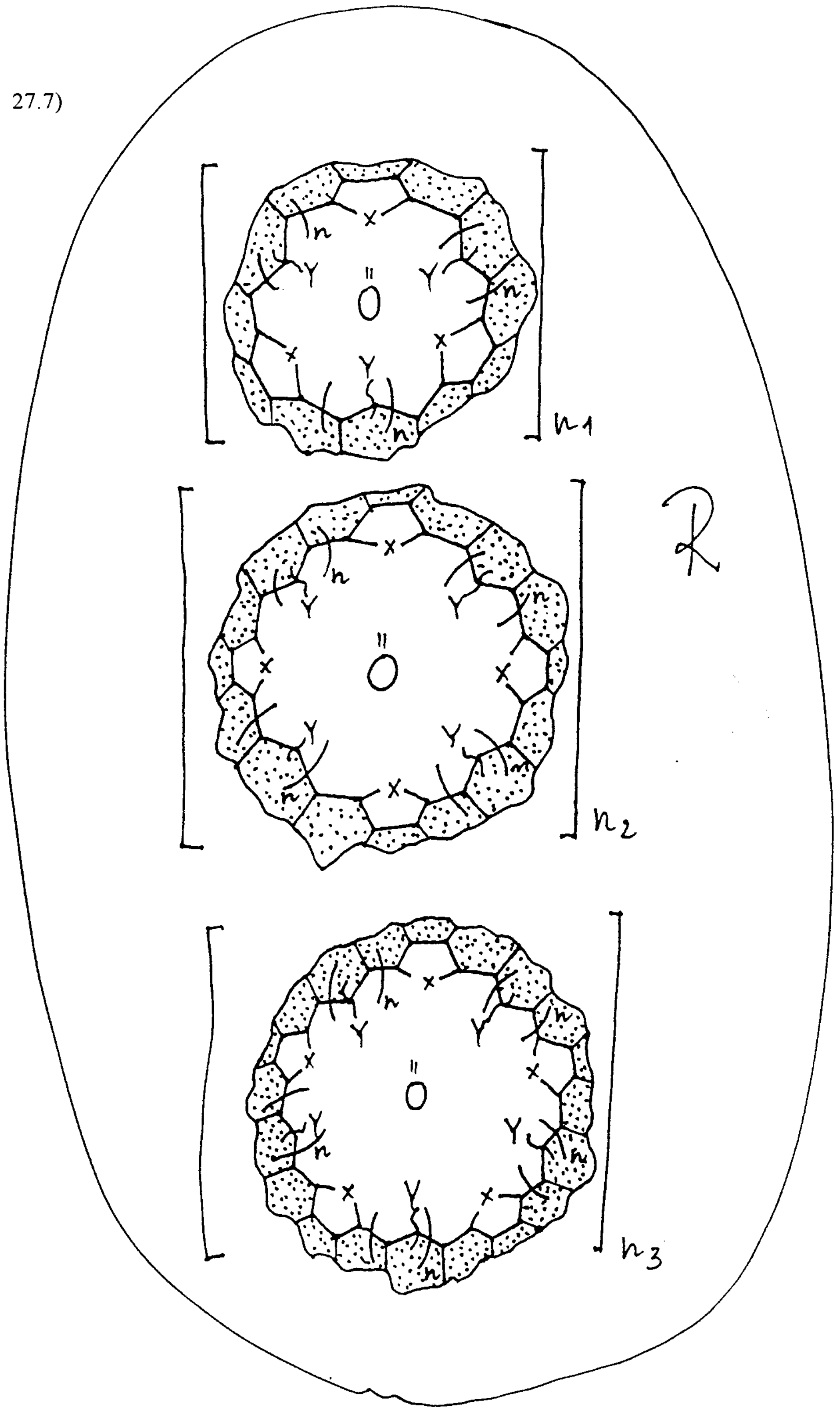

Durch die Vielseitigkeit der erfindungsgemäßen Spheren bezüglich Öffnungsdekoration, Öffnungsgröße, Öffnungsform, Spherengröße gegenüber den bisher bekannten Spheren, deren Öffnungsdekoration auf diejenige mit anellierten Pyrrolen beschränkt war, deren Öffnungsgröße auf diejenige von Subporphinen, Porphinen und Superporphinen beschränkt war, deren Öffnungsform ebenfalls auf diejenige dieser Porphinfunktionen beschränkt war und deren Größe diejenigen der symmetriegleichen Fullerene $C_{268}$ bzw. $C_{292}$ -

(Tetraeder) und $C_{588}$ bzw. $C_{636}$ (Oktaeder) und $C_{1500}$ bzw. $C_{1620}$ (Ikosaeder) nicht überschreitet, ist die Anwendbarkeit dieser neuen Spheren weitaus vielfältiger. Die hier verglichene Öffnungsdekoration bezieht sich allerdings nur auf diejenigen Spherenöffnungen, die das fehlende Fullerenpentagonnetzwerk betreffen.

Der Anwendungsbereich der erfindungsgemäßen Spheren umfaßt daher den der bekannten Spheren, der in der DOS 4114536.4 vom 03.05.1991 sowie in der DOS 4242216.7 vom 15.12.1992 beschrieben wird, und darüber hinaus die folgenden Anwendungsmöglichkeiten:

- Gewinnung von Fullerenen aus den fullerenhaltigen Reaktionsprodukten der Fullerensynthesen;
- Gewinnung von Spheren aus den spherenhaltigen Reaktionsprodukten der Spherensynthesen;
- Herstellung von Beugungsgittern;

Für die genannten Zwecke können die Spheren vorteilhaft ein- oder mehrschichtig als Langmuir Blodget-Filme auf geeignete Oberflächen wie Keramik, Glas oder Metall aufgetragen werden - entweder vor oder nach der erfindungsgemäßen Umsetzung; geschieht das vor der Umsetzung, können auch Fullerene bzw. Heterofullerene eingesetzt werden - anschließend wird das Lückenvolumen vollständig oder teilweise mit einem Bindemittel gefüllt. Als Bindemittel eignen sich reaktive Stoffe wie Metall- oder Halbmetallalkoxide, Schmelzen aus Salzen oder Gläsern und viele andere als Bindemittel wirksame Stoffe. Es kann auch so vorgegangen werden, daß als z. B. die Glasscheibe oder der Salzkristall oder der Kunststoff oder welches Substrat auch verwendet wurde, nach der Beschichtung einem schmelzenden oder sinternden Prozeß unterworfen wird und dann durch das Eigengewicht oder durch mechanische Einwirkung die Penetration bzw. intensive Benetzung der Spheren mit dem nun als Bindemittel wirksamen Substrat gefördert wird. Der Penetrationsvorgang durch sinterndes oder schmelzendes Substrat kann ggf. zusätzlich durch benetzungsfordernde Maßnahmen, wie z. B. zwischen den Spheren ausgefällte schmelzpunkterniedrigende Gele, gefördert werden. Nachdem die innige und möglichst vollständige Benetzung der Spheren durch Substrat bzw. Bindemittel geschehen ist, und der Spheren-Komposit-Werkstoff erstarrt bzw. formfest geworden ist, werden diese Kompositwerkstoffe einer oxidierenden Ätzung in der Flüssig-oder Gasphase unterzogen, und dadurch die Spheren entfernt. Zurück bleiben die Hohlformen der Spheren im Substrat. Für den Zweck der Gewinnung von Fullerenen oder Spheren aus den Reaktionsprodukten ist es nicht notwendig, zweidimensional hochgeordnete Spheren-oder Fullerenfilme auf den Substraten durch die Langmuir-Blodget-Technik (LB) zu erzeugen. Hier reicht es aus, vorzugsweise oberflächenreiche Substrate zu verwenden, an denen die Spheren oder Fullerene adsorbiert werden, dann die spherenhaltigen Substrate mit Bindemitteln umhüllt und gehärtet werden, anschließend die Spheren durch Oxidation entfernt. Anschließend wird das vorzugsweise feinpartikuläre Substrat mit den oberflächlichen Spherennegativformen als chromatographisches Trennsäulenmaterial für die Spheren/Fulleren-Gewinnung eingesetzt. Idealerweise wird die Bindemittelumhüllung der spherenbedeckten Substratpartikel so dosiert, daß die Spherennegativformen nur Spherenhalbschalen wiedergeben. Vorzugsweise werden spherenhaltige Lösungen bzw. fullerenhaltige Lösungen an dem Trennsäulenmaterial chromatographiert nach dem Verfahren der SFC oder HPLC. Das Trennmaterial wirkt außerordentlich selektiv, indem kleinere Fullerene oder Spheren oder größere Spheren oder Spheren einer anderen Symmetrieklasse von der Säulenfüllung nicht adsorbiert werden. Ausschließlich Spheren oder Fullerene der Größe und Symmetrie entsprechend den Negativformen im Trennsäulenmaterial werden stark zurückgehalten und können dadurch abgetrennt werden.

Durch die regelmäßige Anordnung der nach der LB-Technik hergestellten Negativgitterformen haben diese die Eigenschaft von Beugungsgittern.

Speziell aufgrund der möglichen größeren Öffnungen der erfindungsgemäßen Spheren ist die Möglichkeit gegeben, höhermolekulare Stoffe in den Spheren zu speichern. Durch die Dekorationsmöglichkeit der Spherenöffnungen mit positiv geladenen APC ist es möglich, die Öffnungen mit Anionen zu verschließen. Die größeren Öffnungen lassen sich zudem einfacher öffnen und schließen als die kleinen pyrroldekorierten Öffnungen, die ein außerordentlich hohes Bindungspotential zu Schwermetallen und Halbmetallen haben, das in diesem Ausmaß für die reversible Schließung der Öffnungen hinderlich ist.

Das in den erfindungsgemäßen Spheren vorhandene aromatische Kohlenstoff-und/oder Stickstoff-und/oder Boratome enthaltende Netzwerk kann durch bekannte chemische Manipulation, wie z. B. Hydrierung oder Fluorierung, auch in nichtaromatisches Netzwerk überführt werden. Es ist auch möglich, die Umwandlung des aromatischen Netzwerks in nichtaromatisches Netzwerk partiell durchzuführen, so daß einige Bereiche in den Spheren ihre aromatischen Funktionen behalten.

Beispiel 1

100 mg Hyperfullerene, die entsprechend der Methode gewonnen werden, die in der Zeitschrift Nature, Vol. 354 vom 07.11.1991, Seiten 56 bis 58, veröffentlicht ist, werden mit 50 ml Wasser und 0,5 g Perfluoroctancarbonsäure 1 h lang mit Ultraschall behandelt. Die erhaltene Suspension wird mit 20 g

Asbestfasern gemischt, die zuvor mit 20%iger Salzsäure gewaschen werden, getrocknet und bei 700 °C geglüht werden. Nach der intensiven Verteilung der Asbestfasern in der Hyperfullerendispersion wird die Aufschlämmung zur Trockenen eingedampft. Die so mit Hyperfullerenen beladenen Asbestfasern werden dann in ein Quarzglasrohr mit 2 cm Innendurchmesser eingebracht. Das so gefüllte Quarzglasrohr wird im elektrischen Ofen im Stickstoffstrom von 1 l/h mit einer Aufheizrate von 10 °C/min auf 450 °C erwärmt und dann bei dieser Temperatur ca. 30 min belassen. Unter Beibehaltung der Gasströmungsrate wird dann dem Stickstoffstrom 10 Vol% Sauerstoff und 5 Vol% Wasserdampf zugemischt. Die Zumischung von Wasserdampf und Sauerstoff wird nach ca. 7 min abgebrochen und nach Temperaturerhöhung auf 900 °C und Behandlung mit Stickstoff bei dieser Temperatur während 20 min mit Stickstoff versetzt, der 2 Vol% Methylamin enthält. Danach wird noch 30 min unter diesen Bedingungen belassen und dann bis auf 500 °C abgekühlt und die Methylaminzumischung abgestellt und durch eine Zumischung von 2 Vol% Ammoniak ersetzt. Danach wird innerhalb von 30 min auf 200 °C abgekühlt und unter Hinzufügung von 2 Vol% Sauerstoff innerhalb von 30 min auf Zimmertemperatur abgekühlt. Die auf den Asbestfasern adhärierten Hyperfullerene sind durch diese Behandlung in Hyperspheren umgewandelt worden, die zumindest in der äußeren Molekülschale pyrrolringdekorierte Öffnungen enthalten. Die Öffnungen lassen sich überwiegend als Porphin-5-1/1/1/1/1-3N2NH charakterisieren. Durch Ultraschallbehandlung in Chlorbenzol lassen sich die Hyperspheren von den Asbestfasern separieren. Der Ausdruck Hyperspheren wird deshalb gewählt, weil die erhaltenen Moleküle jeweils aus mehreren ineinandergeschachtelten Molekülen bestehen, von denen zumindest das äußere jeweils ein Spherenmolekül ist. Die erhaltenen Hyperspheren haben im Vergleich zu den Hyperfullerenen andere Eigenschaften. So vermögen sie im Unterschied zu den Hyperfullerenen Schwermetalle reversibel zu komplexieren: Bei der Umsetzung der Hyperspheren-Chlorbenzolsuspension mit Schwermetallverbindungen wie beispielsweise Uranylchlorid, Certrichlorid, KupferIIchlorid oder NickelIIchlorid in der Siedehitze werden die Metalle unter Abspaltung von Chlorwasserstoff von den Porphin-dekorierten Hypersphereneöffnungen gebunden. Durch Behandlung der Hyperspheren-Metallkomplexe mit konzentrierter Schwefelsäure in der Wärme lassen sich die Metalle wieder dekomplexieren.

Beispiel 2

10 g des octaedersymmetrischen Fullerens $C_{372}$ dessen Molekülstruktur ausschnitthaft in der Formel (28) gezeigt wird und das entsprechend der Methode synthetisiert wird, die in der DOS P 41 28 357.0 beschrieben wird, wird mit 2 g Perfluoroctancarbonsäure in 400 ml Wasser unter Ultraschalleinwirkung dispergiert bis eine homogene Suspension entstanden ist. Danach wird zu der Suspension unter heftigem Rühren langsam 40 g pyrogene Kieselsäure

28)

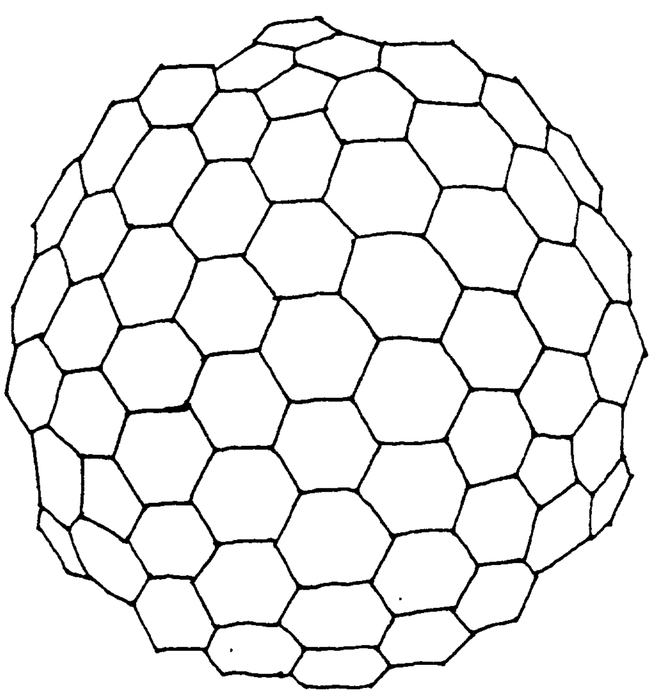

(Aerosil von der Fa. Degussa) hinzugegeben. Nach beendeter Zugabe wird noch 2 h weitergerührt. Anschließend wird die strukturviskose Masse auf einem Blech dünn ausgestrichen und getrocknet bis diese plastisch formbar wird. Dann wird die Masse zu dünnen Nudeln extrudiert und getrocknet. Die getrockneten Nudeln haben einen Durchmesser von etwa 1 mm. 10 g der bei 70 °C getrockneten Nudeln werden gebrochen und in dem in Beispiel 1 beschriebenen Quarzrohr mit der dort genannten Aufheizrate auf 400 °C erwärmt. Während der Aufheizung und fortwährend bis Versuchsende wird mit 1 l/h Stickstoff gespült. Bei der Temperatur von 400 °C wird 1 h lang belassen. Dann wird dem Stickstoffstrom 5 Vol% Wasserdampf und 7 Vol% Sauerstoff beigemischt. Unter diesen Bedingungen wird 15 min belassen, und dann die Wasserdampf- und Sauerstoffzufuhr abgebrochen und auf 900 °C im Stickstoffstrom erwärmt. Unter fortgesetzter Stickstoffspülung wird unter diesen Bedingungen 1 h belassen. Die Richtung der Gasströmung durch den Reaktor wird dabei jeweils nach 2 min umgekehrt, um Fulleren- bzw. Spherenverluste zu minimieren. Danach wird unter Stickstoff auf 400 °C abgekühlt und bei dieser Temperatur 1 h lang mit Stickstoffplasma behandelt, das mittels Koronaentladung zuvor erzeugt worden ist. Mit 10 °C/min wird dann unter $N_2$ auf Zimmertemperatur abgekühlt. Mit Flußsäure im Überschuß wird anschließend der Kieselsäureträger aufgelöst und danach die organische Substanz mit einem Gemisch von 200 ml Dichlormethan und 300 ml Wasser kräftig gemischt und dann die schwere organische Phase durch Aceotropdestillation getrocknet. Danach wird die organische Phase zusammen mit 20 g Kupfer-II-chlorid in einem Rührautoklaven 2 h lang auf 160 °C erwärmt und danach abgekühlt und entspannt. Aus dem erhaltenen Reaktionsgemisch wird Dichlormethan abgedampft und der Rückstand solange mit Wasser gewaschen, bis das ablaufende Wasser chloridfrei ist. Der verbleibende Rückstand wird getrocknet. Daraus kann der reine Spheren-Kupferkomplex durch Sublimation unter Argon als schwarzblaue mikrokristalline Substanz gewonnen werden. Die Sublimation gelingt bei Temperaturen über 500 °C. Der Spheren-Kupferkomplex wird in den Formeln 29) und 30) beschrieben. Danach besteht der Spheren-Kupferkomplex, der eine Summenformel von $C_{288}N_{24}Cu_6$ aufweist, aus einem Arrangement aus 6 Porphin-4-1/1/1/1-4NCu-Funktionen, die symmetrisch in ein spherisches Netzwerk aus dreifach koordiniertem Kohlenstoff eingebettet sind. Der Spherus ist oktaedersymmetrisch, wobei die Porphin-4-1/1/1/1-4NCu-Funktionen jeweils die Ecken eines gedachten Oktaeders einnehmen. Die Symmetrie des Spherus entspricht damit derjenigen des Fullereneduktes, in dem die 6 Pentacyclenpaare jeweils die Ecke eines gedachten Oktaeders einnehmen. Die Formeln 29) und 30) zeigen verschiedene Ansichten des Spherus ausschnitthaft.

29) + 30)

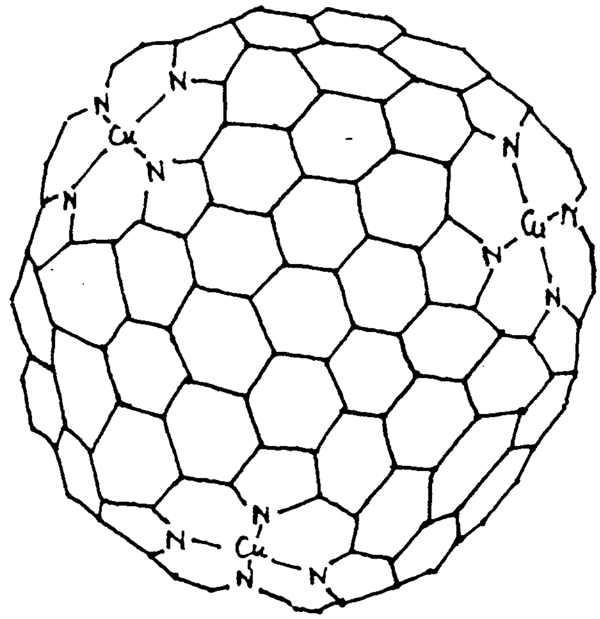

29)

30)

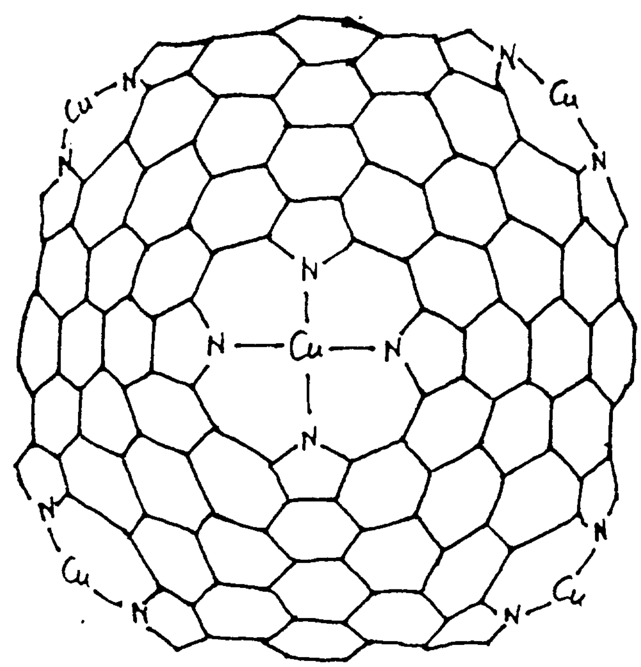

In einem weiteren Ansatz werden die entsprechend dem ersten Ansatz auf pyrogener Kieselsäure aufgetragenen Fullerene zunächst in der gleichen Weise oxidiert, bis etwa 1/3 des Kohlenstoffs vergast ist. Danach wird mit reinem Ammoniakgas bei 900 °C behandelt. Der Kohlenstoffgehalt in dem Abgas wird gemessen und die Ammoniakbehandlung abgebrochen, wenn von dem nach der Oxidationsbehandlung verbliebenen Kohlenstoffanteil weitere 2/3 vergast sind. Danach wird die Ammoniakgasbehandlung abgebrochen und auf 120 °C unter Stickstoff abgekühlt und danach bei dieser Temperatur noch 1 h lang Luft hinzutreten lassen. Danach wird auf Zimmertemperatur abgekühlt und die spherenhaltigen Kieselsäureträger mehrfach mit n-Hexan gewaschen und danach getrocknet. Danach werden die Kieselsäureträger in Flußsäure aufgelöst und die mit Ammoniak neutralisierte Lösung filtriert. Der erhaltene mit Wasser gewaschene Filterrückstand ergibt getrocknet ein blauviolettes Pulver mit der Summenformel $C_{84}H_{36}N_{24}$, bei dem es sich um oktaedersymmetrische Spheren handelt, die durch die Formelausschnitte 30.1) und 30.2) charakterisiert sind. Die Öffnungen in den Spheren lassen sich als Porphin-4-3/3/3/3-2N2NH bezeichnen.

30.1) + 30.2)

30.1)

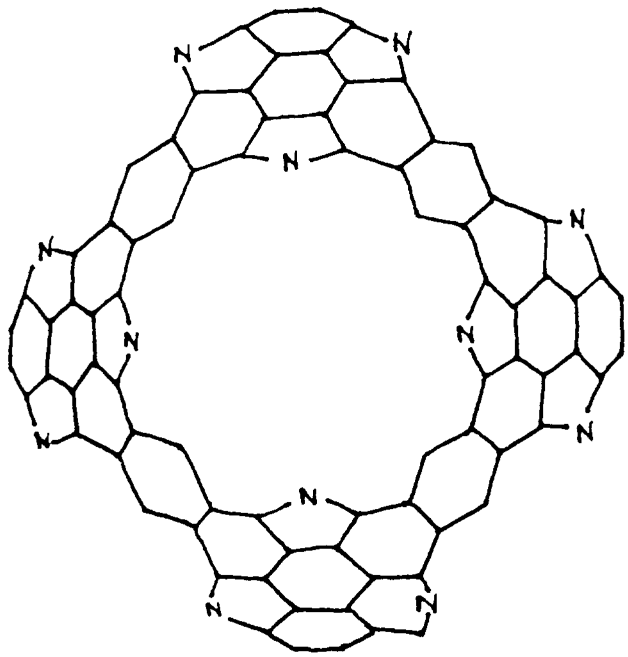

30.2)

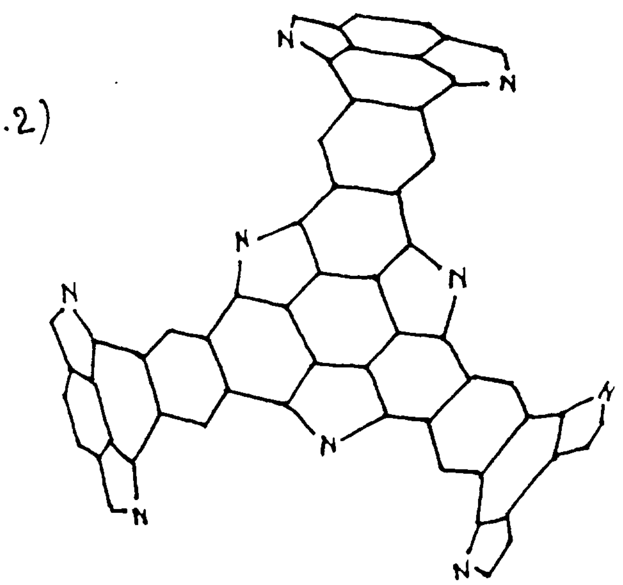

Beispiel 3

Beispiel 2 wird bis zu dem Schritt der Wasserdampf- und Sauerstoffbehandlung bei 400 °C wiederholt. Nachdem diese Behandlung entsprechend Beispiel 2 durchgeführt ist, wird im Stickstoffstrom mit einer

Aufheizrate von 15 °C/min auf 900 °C erwärmt. Dabei wird die Strömungsausrichtung des Inertgases im Rhythmus 1/min umgekehrt. Die Stickstoffbehandlung bei 900 °C wird nach 45 min abgebrochen und unter Stickstoff auf 110 °C abgekühlt. Bei dieser Temperatur werden die Spheren 1 h lang einem Luftstrom ausgesetzt. Die derart entstandenen Spheren werden anschließend isoliert, indem der Kieselsäureträger mit Flußsäure aufgelöst wird, und die lipophile organische Substanz in 200 ml Toluol aufgenommen wird. Die Toluolphase wird danach mit 200 g 2,3-Dichlor-5,6-dicyan-1,4-benzochinon unter Rühren versetzt. Die Reaktion wird bei Zimmertemperatur durchgeführt. Nach 2 h Reaktionszeit wird filtriert und der getrocknete Filterrückstand mit 100 ml konzentrierter Schwefelsäure unter Rühren auf 80 °C erwärmt. Dabei gehen die durch das Chinon oxidierten Spheren als Polykationen in Lösung. Nicht umgesetzte Fullerene bleiben ungelöst und können von der Spheren-Schwefelsäurelösung abgetrennt werden. Durch Behandlung der Schwefelsäure-Spherenlösung mit Zinkmetall unter Zugabe von Wasser können die gelösten Spheren-Polykationen reduziert werden, wobei die neutralen Spheren wieder ausfallen und abfiltriert werden können. Mit Methanol sulfatfrei gewaschen und getrocknet können die neutralen Spheren rein erhalten werden. Die Spherenpolykationen sind beispielhaft im Ausschnitt in der Formel 31) dargestellt; die neutralen reduzierten Spheren sind ausschnitthaft in der Formel 32) dargestellt. Danach enthalten die Spheren je Spherus 6 Porphin-4-1/1/1/1-4O-Funktionen im reduzierten Zustand und mindestens je Spherus 1 Porphin-4-1/1/1/1-2O$^{\oplus}$2O-Funktion und höchstens je Spherus 6 Porphin-4-1/1/1/1-2O$^{\oplus}$2O-Funktionen im oxidierten Zustand. Die Elementaranalyse der reduzierten Spheren steht im Einklang mit der Summenformel $C_{288}O_{24}$. Die Symmetrie des Spherus entspricht der eines regulären Oktaeders, wobei die Porphin-4-Funktionen jeweils eine Ecke des Oktaeders bilden. Die Symmetrie des Spherus entspricht damit derjenigen des Fullereneduktes.

31) + 32)

31)

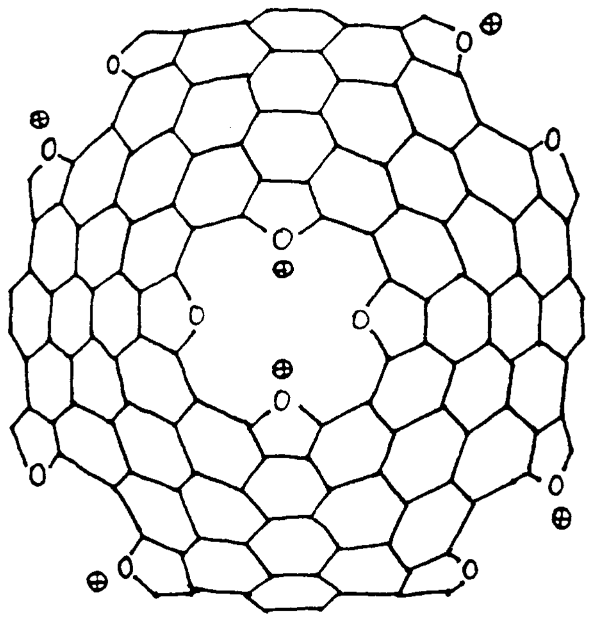

32)

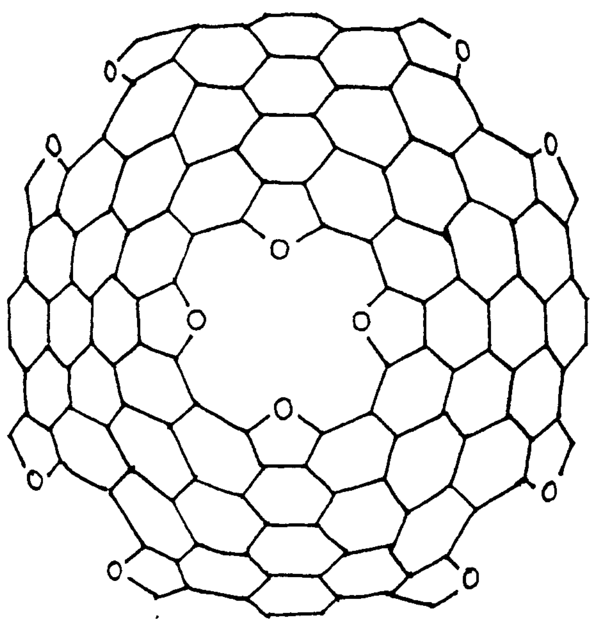

Beispiel 4

Beispiel 3 wird mit der Einschränkung wiederholt, daß nach der Stickstoffbehandlung bei 900 °C auf 400 °C abgekühlt wird, und dann dem Stickstoffstrom bei dieser Temperatur 5 Vol% Schwefeldampf beigemischt wird. Nach 20 min wird die Schwefelbehandlung abgebrochen und unter Stickstoff abgekühlt

und weiterhin wie in Beispiel 3 weiterverfahren. Durch Oxidation zu Spherenkationen, Lösung in Schwefelsäure und Fällung der reduzierten Spheren aus schwefelsaurer Lösung können die Spheren rein erhalten werden. Die Spherenpolykationen sind beispielhaft in einem Ausschnitt, der auf eine Porphin-4-Funktion beschränkt ist, in der Formel 33) dargestellt. In einer analogen Darstellung wird die Porphin-4-Funktion eines reduzierten neutralen Spherus dargestellt und zwar in der Formel 34). Danach enthalten die Spheren 6 Porphin-4-1/1/1/1-4S-Funktionen je Spherus im reduzierten Zustand und mindestens eine und höchstens sechs Porphin-4-1/1/1/12S2S$^{\oplus}$-Funktionen je Spherus im oxidierten Zustand. Die Elementaranalyse der reduzierten Spheren steht im Einklang mit der Summenformel $C_{288}S_{24}$. Die Symmetrie der Spheren entspricht derjenigen von regulären Oktaedern, wobei die Porphin-Funktionen jeweils die Ecken der Oktaeder einnehmen. Die Symmetrie der Spheren entspricht damit derjenigen der Eduktfullerene, die anstelle der Porphinfunktionen jeweils Pentacyclenpaare enthalten, von denen jedes Paar eine Oktaederecke einnimmt.

33) + 34)

33)

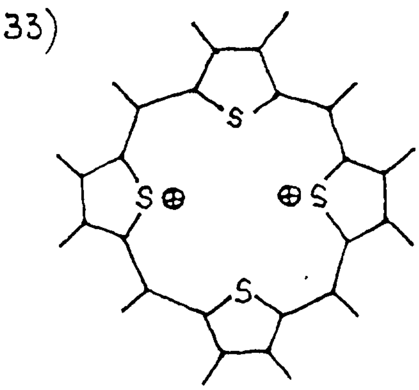

34)

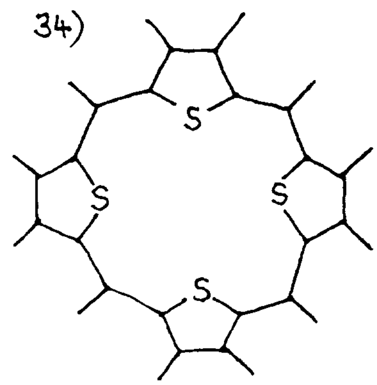

Beispiel 5

In Anlehnung an die Vorschrift zur Herstellung von Spheren, die in der DOS 41 14 536 gegeben wird, werden 250 g wasserfreies Uranylchlorid in einer Lösung von 500 ml Chinolin und 1000 ml N,N-Dimethylformamid im Rührautoklaven auf 200 °C und 100 bar erwärmt bzw. gedrückt. Dazu wird eine Lösung von 100 g Ovalen-3,4,10,11-tetranitril (35) in 2000 g Chinolin langsam zulaufen lassen. Die Herstellung des Tetranitrils 35) wird entsprechend der DOS 41 14 536 durchgeführt.

35)

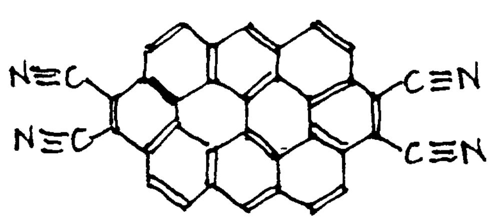

Danach wird 1 h unter diesen Bedingungen noch belassen und dann 15 min lang auf 300°C erwärmt, dann absitzen lassen und dann die überstehende fluide Phase abgezogen und entspannt. Aus dem entspannten Fluid lassen sich die ikosaedersymmetrischen Spheren erhalten, die sich durch die Formeln 36) und 37) ausschnitthaft beschreiben lassen, und die die Summenformel $C_{1080}H_{300}N_{120}O_{24}U_{12}$ besitzen. Diese Spheren werden entsprechend der DOS 41 28 357.0 in das ikosaedersymmetrische CBN-Heterofulleren $C_{1380}B_{120}N_{120}$ umgesetzt, das durch den Formelausschnitt 38) im Ausschnitt beschrieben wird. 0,1 g des CBN-Heterofullerens 38) wird mit 100 ml n-Hexan unter Ultraschalleinwirkung dispergiert. Danach wird die Dispersion auf eine Wasserfläche von 50 mal 50 $cm^2$ gegossen, und das n-Hexan verdampfen lassen. Danach wird eine durch Behandlung mit Dimethyldichlorsilan hydrophobierte trockene Glasscheibe mit einer Fläche von 10 cm mal 10 cm durch langsames Eintauchen in die CBN-heterofullerenbeschichtete Wasserfläche mit einer optisch gleichmäßigen CBN-Heterofullerenschicht beidseitig bedeckt. Mehrere derart beschichtete Glasscheiben werden, in ein Gestell aus Siliziumcarbid eingestellt, entsprechend Beispiel 2 oxidiert und bei 900°C mit Stickstoff behandelt. Die danach unter Stickstoff auf 450 °C abgekühlten Spheren werden dann bei dieser Temperatur 20 min lang mit bei 400°C mit

36) + 37)

36)

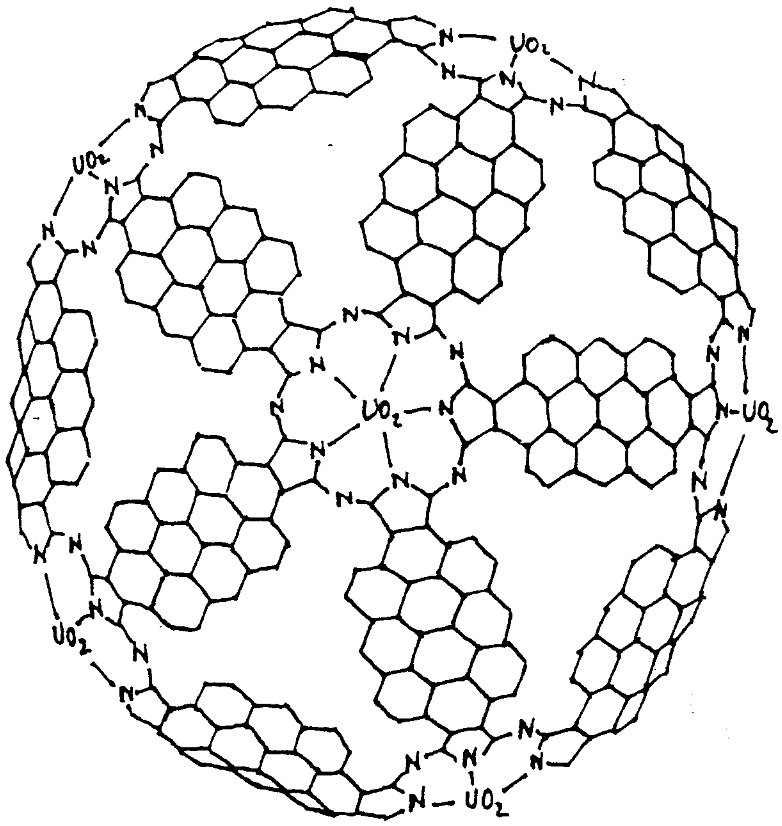

37)

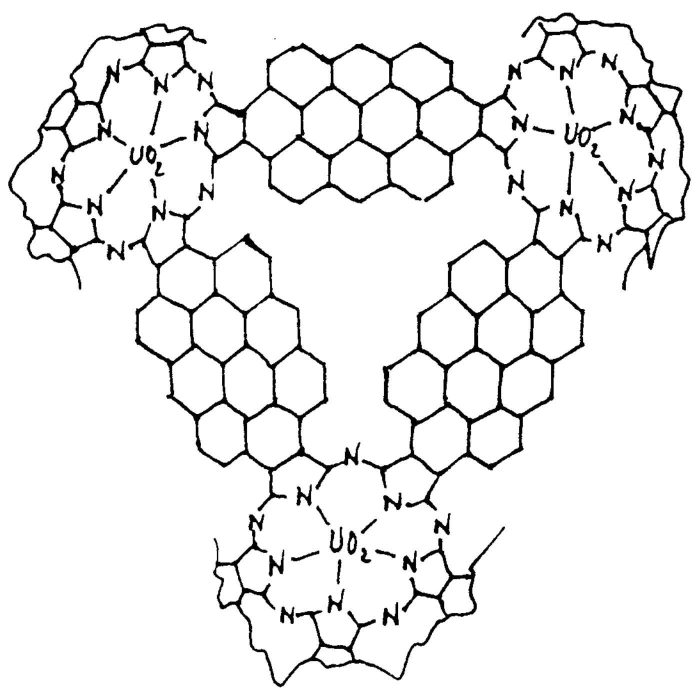

38)

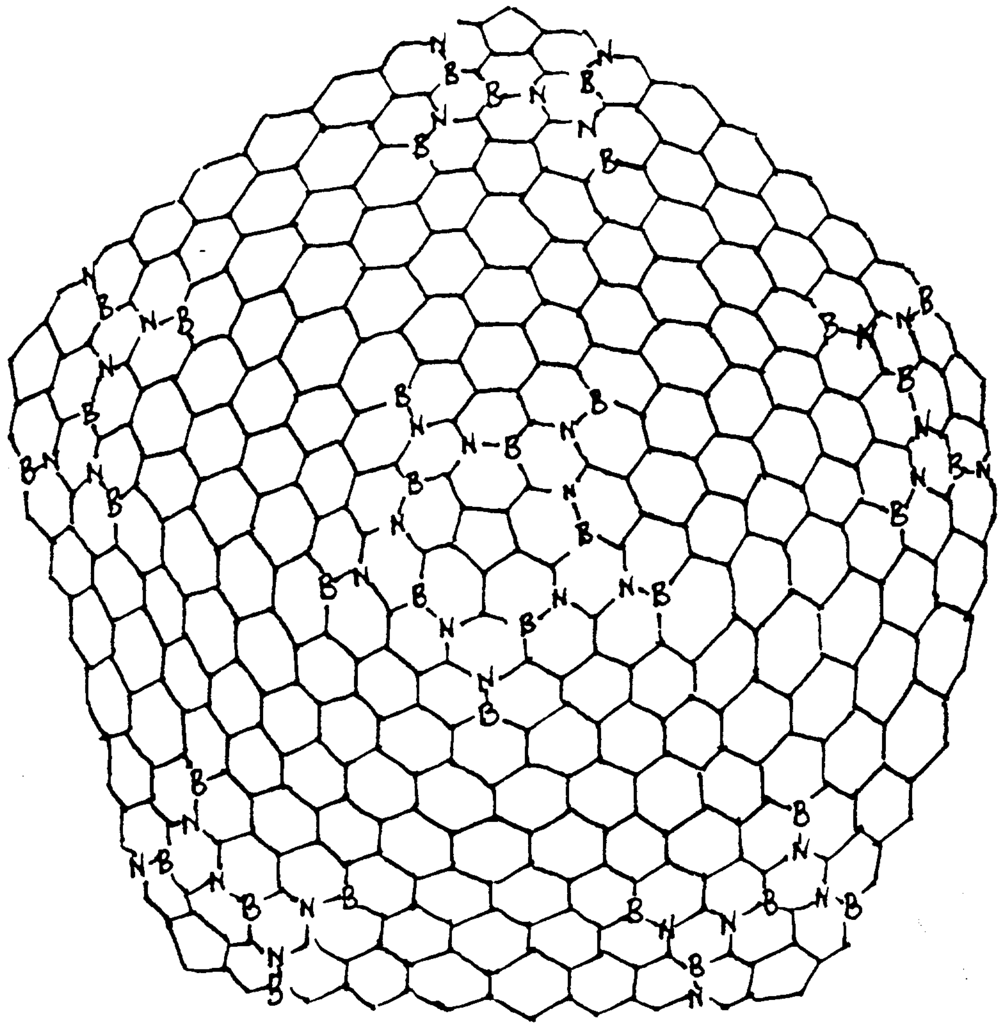

Selen gesättigtem Stickstoff behandelt und dann unter Stickstoff auf Raumtemperatur abgekühlt. Die derart auf Glas aufgetragenen Spherenfilme besitzen eine Reihe interessanter Eigenschaften. Sie lassen sich anodisch oxidieren und kathodisch reduzieren und dadurch als reversibel aufladbare Batterie einsetzen. Die Filme adsorbieren Schwermetalle, insbesondere Silberionen. Bei Bestrahlung mit Sonnenlicht steigt die elektrische Leitfähigkeit der Filme. Die Filme lassen sich auch chemisch oxidieren und danach wiederum reversibel chemisch reduzieren. Durch das Auflösen der Quarzglasträger mit Flußsäure lassen sich auch freie Spherenfilme erhalten. Die erhaltenen Spherenfilme sind von hoher Regelmäßigkeit; sie haben die Eigenschaften zweidimensionaler Einkristalle.

Bei den erhaltenen Spherenfolien handelt es sich ausweislich der Elementaranalyse um Spheren, die sich durch die Summenformel $C_{1260}B_{60}N_{60}Se_{60}$ kennzeichnen lassen. Die ikosaedersymmetrischen Spheren enthalten je Molekül 12 Porphin-5-1/1/1/1/1-5Se-Funktionen, die jeweils die Ecken des ikosaedrischen Moleküls besetzen, wie in dem Formelausschnitt 39) gezeigt wird. Die erhaltenen Spheren sind Fragmente der dem ikosaedersymmetrischen Fullerenen $C_{1620}$ entsprechenden CBN-Heterofullerene.

39)

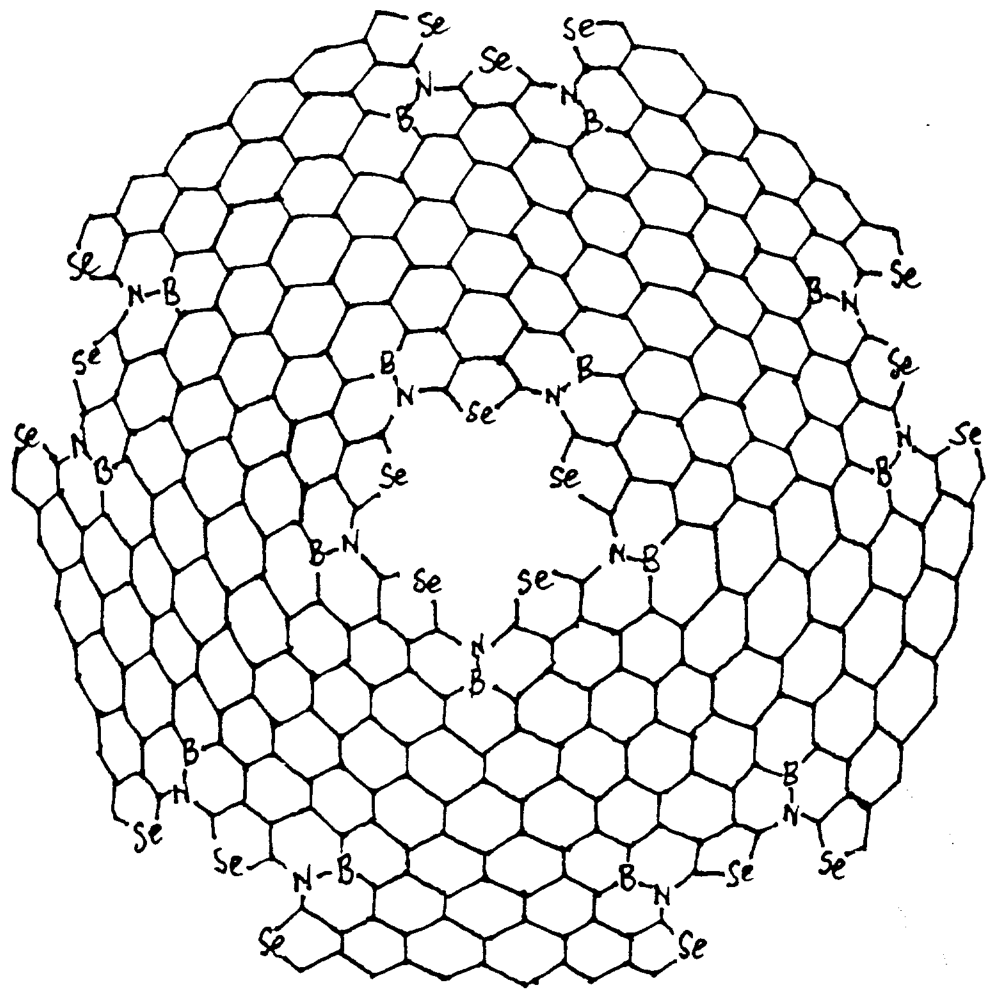

Durch Oxidation, die entsprechend Beispiel 3 geschehen kann, mit 2,3-Dichlor-5,6-dicyan-1,4-benzochinon und Umsetzen mit konzentrierter Schwefelsäure können die neutralen Spheren zu Spherenpolykationen umgewandelt werden, die je Spherus mindestens eine Porphin-5-1/1/1/1/1-3Se2Se$^{\oplus}$-Funktion höchstens aber 12 Porphin-5-1/1/1/1/1-3Se2Se$^{\oplus}$-Funktionen enthalten können, wie sie in der Formel 40) dargestellt sind.

40)

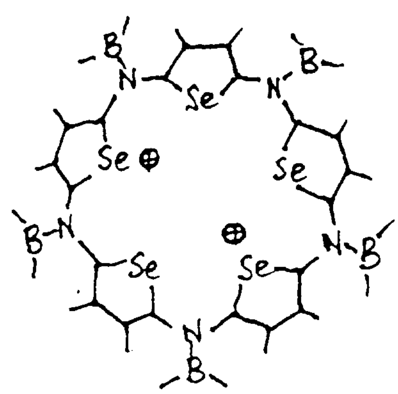

Beispiel 6

1 g der CBN-Heterofullerene 41), die eine Elementarzusammensetzung entsprechend Summenformel $C_{252}N_{24}B_{16}$ haben, und die entsprechend der Methode dargestellt werden, die in der DOS 41 28 357.0 angegeben wird (in dieser Veröffentlichung wird für diese Verbindung zwar die gleiche Formel 41) angegeben, jedoch eine andere Elementarzusammensetzung, nämlich $C_{260}N_{24}B_8$. Nach den vorliegenden Untersuchungen trifft jedoch für diese Heterofullerene die Zusammensetzung $C_{252}N_{24}B_{16}$ zu. Bezüglich der Elementarzusammensetzung dieser Verbindung irrt daher die DOS 41 28 357.0), werden unter

41)

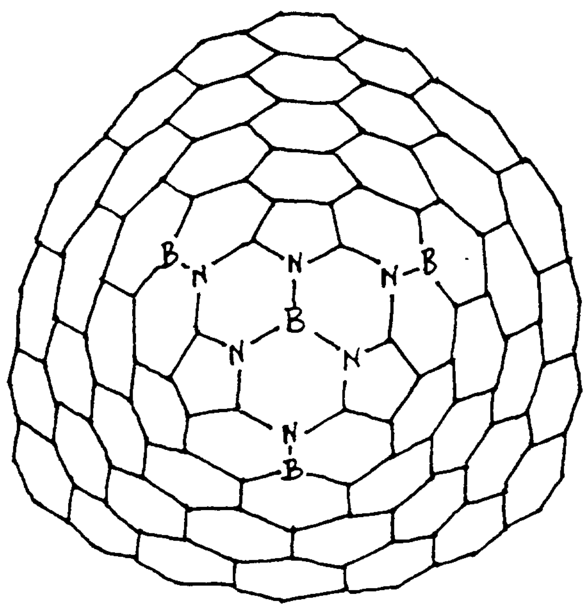

Ultraschalleinwirkung in n-Hexan suspendiert, die n-Hexan-Suspension mit Aerosil eingedickt, und die entstandene Paste auf einem Blech in dünner Schicht eintrocknen lassen, danach stückig gebrochen und gemäß Beispiel 1 oxidiert. Dann wird unter Stickstoffdurchströmung auf 950 °C erwärmt, bei dieser Temperatur und Stickstoffstrom 30 min lang gehalten und danach auf 130 °C abgekühlt. Bei dieser Temperatur wird über Nacht an der Luft stehen gelassen und danach abgekühlt. Nach dem Auflösen des Kieselsäureträgers in Flußsäure, Methanolwäsche des festen Rückstandes, Trocknen bis zur Gewichtskonstanz verbleibt ein schwarzbraunes Pulver, bei dem es sich entsprechend der Elementaranalyse um Spheren der Summenformel $C_{252}B_{12}N_{12}H_{12}$ handelt. Die tetraedersymmetrischen Spheren enthalten je Molekül 4 Porphin-3-1/1/1-3O-Funktionen, die die Ecken des tetraedersymmetrischen Moleküls besetzen, wie die Formeln 42) und 43) ausschnitthaft zeigen.

42) + 43)

42)

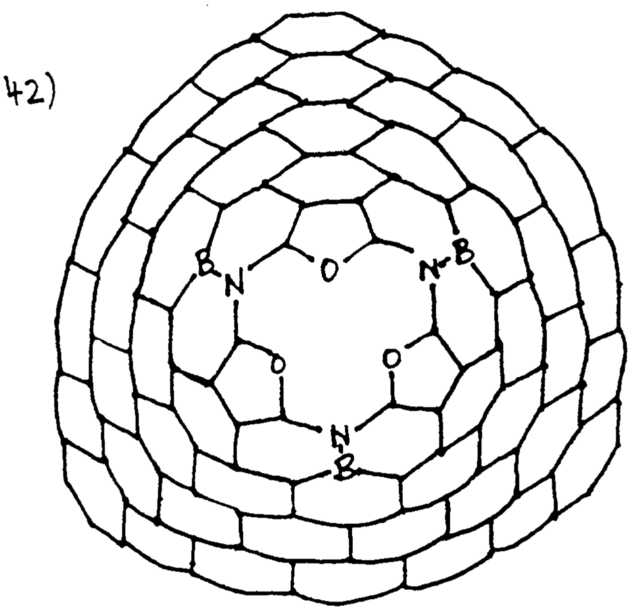

43)

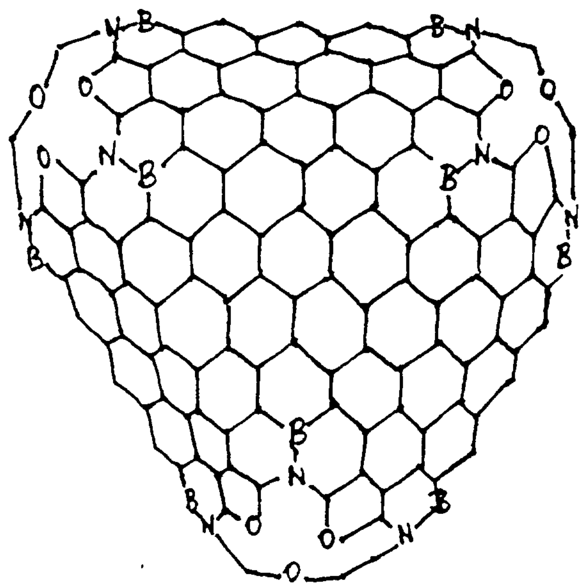

Entsprechend den vorgenannten Beispielen können auch diese Spheren zu Spherenpolykationen oxidiert werden, die mindestens eine höchstens vier Porphin-3-$OO^{\oplus}_2$-Funktionen 44) enthalten.

44)

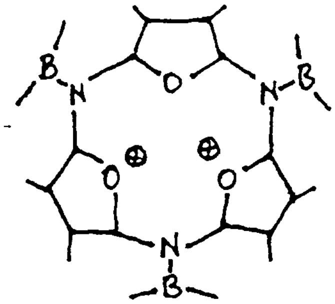

Die entsprechend Formeln 42) bzw. 43) erhaltenen Spheren werden nochmals auf Aerosil aufgetragen, unter Stickstoff auf 950 °C erwärmt und bei dieser Temperatur 10 min lang mit Wasserdampf behandelt und danach wieder auf 130 °C abgekühlt und bei dieser Temperatur über Nacht an der Luft stehen gelassen. Nach der Elementaranalyse der in der gleichen Weise isolierten Spheren haben diese eine Summenformel von $C_{204}O_{24}$. Danach enthalten die Spheren je Molekül vier Porphin-3-3/3/3-3O-Funktionen.

Die derart erhaltenen Spheren werden nochmals auf Aerosil aufgetragen und in unterschiedlicher Weise weiterbehandelt:

a) mit einem Gemisch aus 2 Vol% Blausäure, 1 Vol% Methan und 85 Vol% Stickstoff 10 min lang bei 900 °C behandelt;

b) mit Stickstoff 20 min bei 900 °C, anschließender Abkühlung auf 500 °C und Behandlung 5 min lang mit Methanplasma aus der Glimmentladung;

c) mit Ammoniakplasma, das durch Glimmentladung erzeugt wurde, bei 500 °C 10 min lang umgesetzt;

d) mit einem Gemisch aus 1 Vol% Benzol und 99 Vol% Stickstoff 15 min lang bei 900 °C behandelt.

Nach den jeweiligen Behandlungen werden die Spheren auf Raumtemperatur abgekühlt und wie oben ohne weitere Vorkehrungen aufgearbeitet.

Die nach a) behandelten Spheren enthalten je nach Molekül vier Porphin-3-3/3/3-2NH1N-Funktionen entsprechend Formel 44.3). Die nach b) behandelten Spheren enthalten je Molekül vier Porphin-3-3/3/3-2$CH_2$1CH-Funktionen entsprechend Formel 44.4). Die nach c) behandelten Spheren enthalten je nach Molekül vier Porphin-3-3/3/3-2NH1N-Funktionen entsprechend Formel 44.5). Die nach d) behandelten Spheren enthalten je nach Molekül vier Porphin-3-3/3/3-stämmige zylindrische Fortsätze aus Kohlenstoffhexagonnetzwerk mit 3 Kohlenstoffheptagonen entsprechend Formelausschnitt 44.6).

44.1) + 44.2)

44.1)

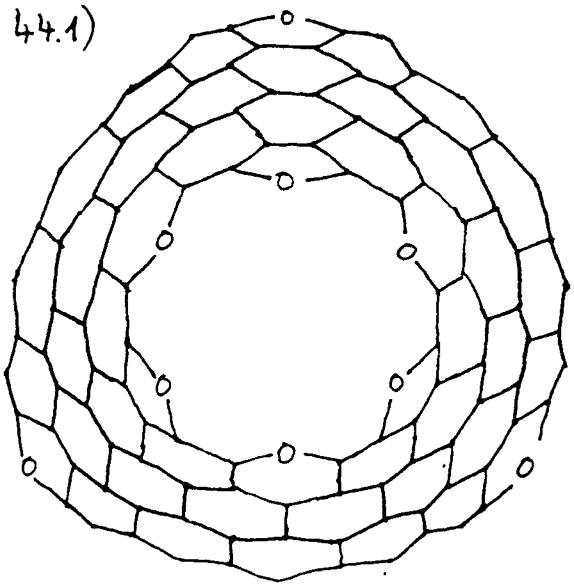

44.2)

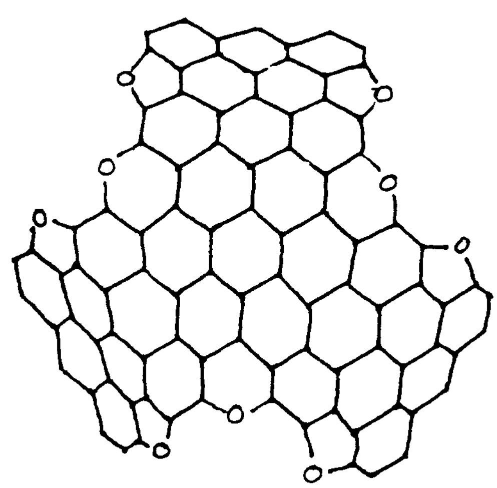

44.3) + 44.4) + 44.5) + 44.6)

44.3)

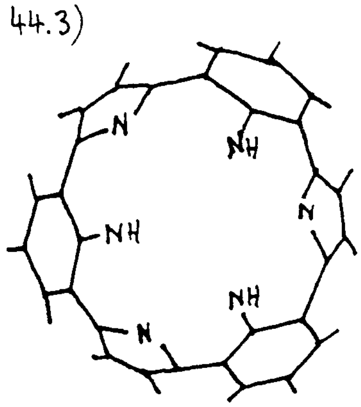

44.4)

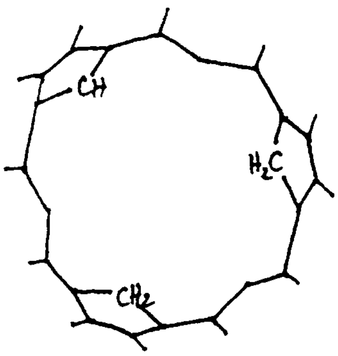

44.5)

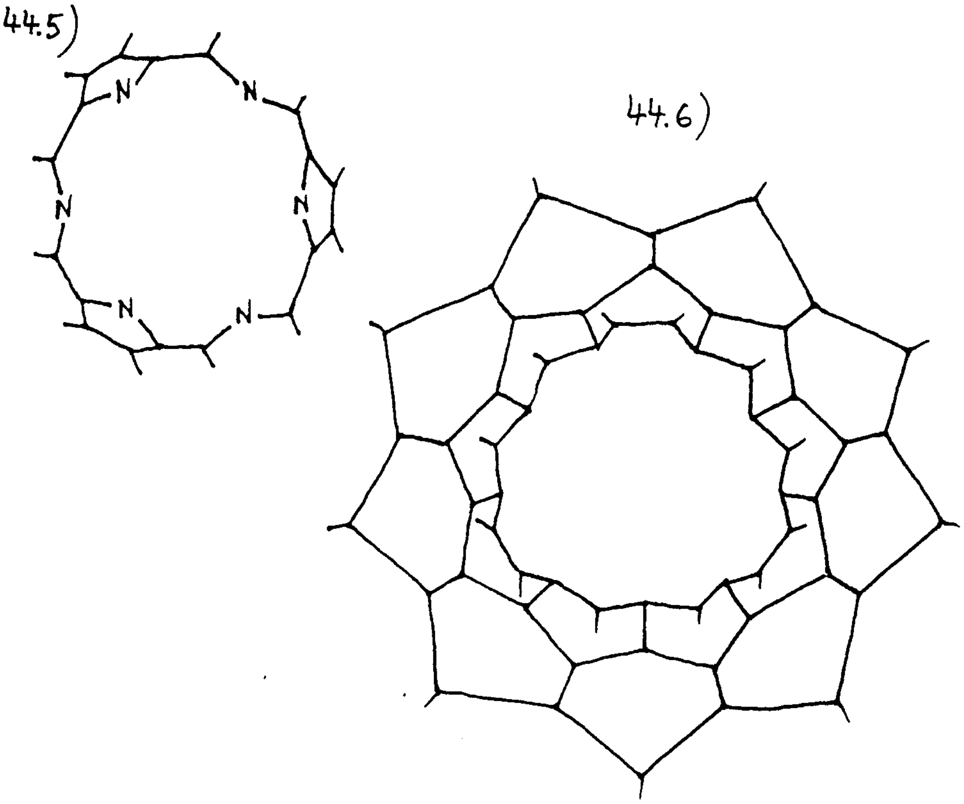

44.6)

Beispiel 7

Die entsprechend Beispiel 3 erhaltenen neutralen Spheren mit Porphin-4-1/1/1/1-4O-Funktionen, die noch auf dem Kieselsäureträger fixiert sind, werden bei 500 °C einer Atmosphäre aus 95 Vol% Stickstoff

und 5 Vol% Ammoniak ausgesetzt. Anschließend werden die Spheren auf 200 °C abgekühlt und danach bei dieser Temperatur einer Atmosphäre aus 90 Vol% Stickstoff und 10 Vol% Sauerstoff während einer Zeitdauer von 15 min ausgesetzt. Die weitere Behandlung des abgekühlten Reaktionsproduktes wird dann gemäß Beispiel 2 fortgesetzt. Als Reaktionsprodukt werden Spheren erhalten, wie sie in den Formeln 29) und 30) ausschnitthaft beschrieben werden. Danach handelt es sich um oktaedersymmetrische Spheren mit der Summenformel $C_{288}N_{24}Cu_6$. Dabei enthält jeder Spherus 6 Porphin-4-1/1/1/1-4NCu-Funktionen, wobei jedes Cu-Atom die Ecke eines Oktaeders einnimmt.

Beispiel 8

Die entsprechend Beispiel 3 erhaltenen neutralen Spheren, die noch auf dem Kieselsäureträger fixiert sind, werden bei 500 °C einer Atmosphäre aus 90 Vol% Stickstoff und 10 Vol% Ammoniak ausgesetzt. Diese Behandlung wird 20 min lang fortgeführt. Danach werden die abgekühlten Reaktionsprodukte mit Aceton versetzt, wobei etwa 200 ml Aceton zur Anwendung gebracht werden. Das Gemisch wird danach bei Zimmertemperatur mit 20 g 2,3-Dichlor-5,6-dicyan-1,4-benzochinon versetzt und dann bei Zimmertemperatur 2 h lang gerührt. Danach wird mit Aceton gewaschen bis der Rückstand chinon-/hydrochinonfrei ist. Danach wird mit Wasser acetonfrei gewaschen, die Kieselsäureträger mit Flußsäure aufgelöst, und der Rückstand im Vakuum bei Zimmertemperatur getrocknet. Bei dem erhaltenen schwarzen Pulver handelt es sich um Spheren mit der Summenformel $C_{288}N_{24}$. Die Spheren sind vom oktaedersymmetrischen Typ. Dabei enthält jeder Spherus 6 Porphin-4-1/1/1/1-4N-Funktionen. Jede nimmt dabei eine Oktaederecke im Spherus ein. Die Porphin-4-1/1/1/1-4N-Funktion ist in der Formel 45) dargestellt. Durch Behandlung mit Hydrochinon lassen sich die Spheren durch Umwandlung der Porphin-4-1/1/1/1-4N-Funktionen in die korrespondierenden Porphin-4-1/1/1/1-2N2NH-Funktionen reduzieren. Letztere sind in der Formel 46) dargestellt. Die reduzierten Spheren haben die Summenformel $C_{288}H_{12}N_{24}$.

45) + 46

45)

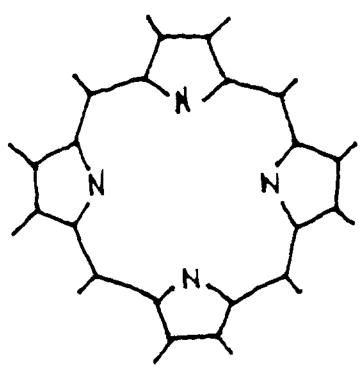

46)

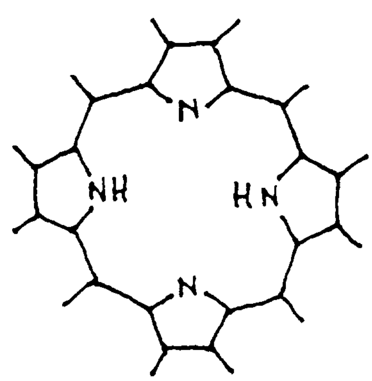

Beispiel 9

In Anlehnung an die Vorschrift zur Herstellung des ikosaedersymmetrischen Spherus $C_{240}N_{120}U_{12}O_{24}$, dessen Molekülstruktur in den Formeln 47) und 48) ausschnitthaft gezeigt wird, die in der DOS 4114536 A1 mitgeteilt wird, werden 25 g wasserfreies Uranylchlorid in 100 ml Pyridin und 150 ml Dimethylformamid im Rührautoklaven auf 200 °C und einen Druck von 200 bar gebracht. Dazu wird eine Lösung von 10 g Hexacyanobenzol in 100 ml Pyridin und 100 ml Dimethylformamid langsam zugegeben. Nach 3 h Reaktionszeit wird absitzen gelassen und danach unter Druck von dem abgesetzten Rückstand dekantiert und danach die fluiden Phasen abgezogen. Die fluiden Phasen werden abgekühlt und entspannt und danach mit Methylalkohol versetzt, bis die Fällung der Spheren vollständig ist. Anschließend wird filtriert und der Filterrückstand getrocknet.

47) + 48)

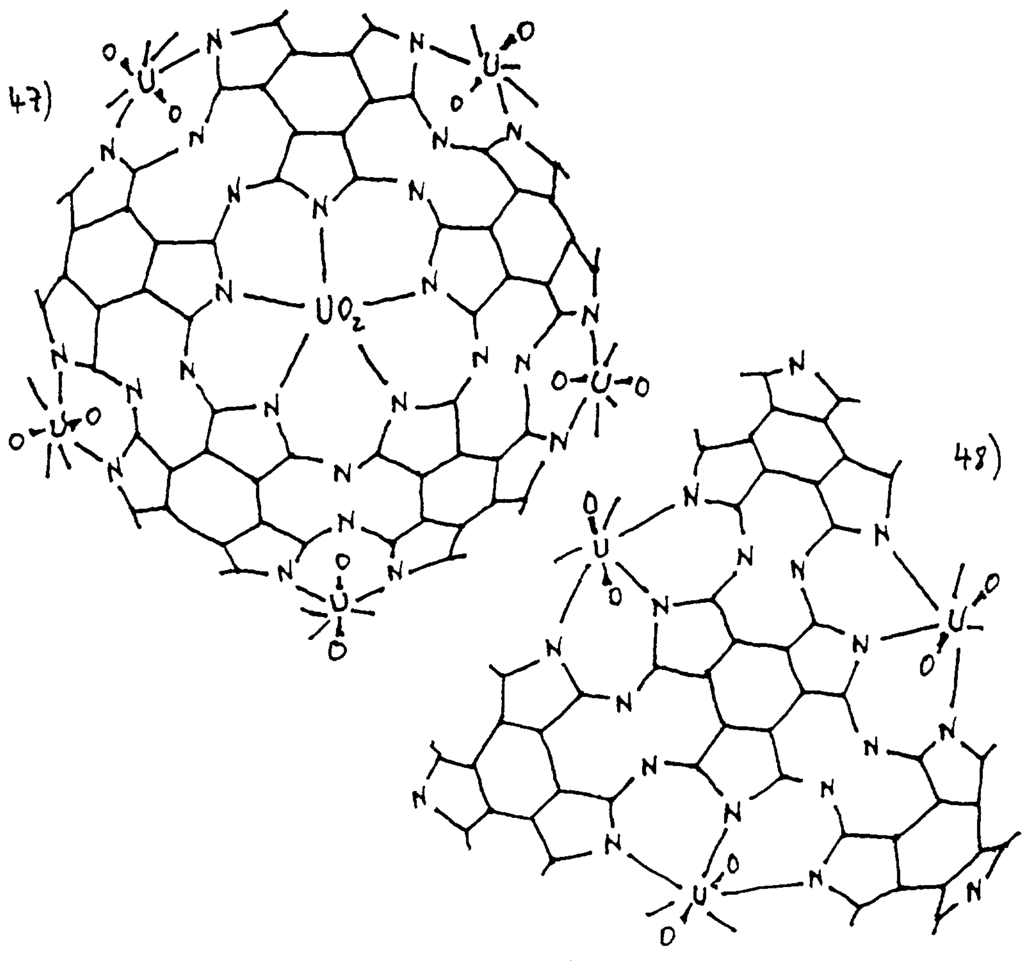

47)

48)

Die derart erhaltenen Spheren sind Fragmentderivate des ikosaedersymmetrischen Fullerens $C_{540}$ und nicht, wie in der DOS 4114536 A1 irrtümlich angegeben ist, des ikosaedersymmetrischen Fullerens $C_{960}$!
10 g der so gewonnenen Spheren werden mit 150 ml Benzol in einem Rührautoklaven auf 350 °C erwärmt und durch Aufpressen von Helium auf einen Druck von 350 bar gebracht. Unter diesen Bedingungen wird 30 min belassen, bis sich eine homogene Mischung/Lösung gebildet hat. Diese homogene fluide Phase wird danach mit einer Durchflußrate von 1 ml/h mittels einer Düse in einen Heliumstrom hinein entspannt, der bei einem absoluten Druck von 150 mbar gehalten wird. Der danach mittels statischem Mischer homogenisierte beladene Heliumstrom wird in einer Durchflußrate von 1 l/h durch einen Hochfrequenzofen hindurchgeleitet, wie er in der Zeitschrift Angewandte Chemie Jahrgang 1992, Nr. 2, Seiten 240 bis 241

beschrieben wird. Die Temperatur in dem Ofen wird dabei bei 2.700 °C gehalten. Die Reaktionsprodukte, nämlich Ruß und Fullerene, werden bei 0 °C zur Abscheidung der kleineren - Fullerenmoleküle mit Toluol extrahiert. Der ungelöste Extraktionsrückstand wird anschließend zusammen mit 100 ml Toluol bei 200 °C und 200 bar im Rührautoklaven 20 min lang behandelt und anschließend die überstehende fluide Phase rasch entspannt und abgekühlt durch Versprühen in einen Rührkolben, der zu etwa 1/5 mit einem Gemisch aus 400 ml Wasser, 1 g Perfluoroctansäure und 40 g pyrogener Kieselsäure gefüllt ist. Danach wird die strukturviskose Masse entsprechend Beispiel 2 weiterbehandelt. Zum Unterschied zu Beispiel 2 wird anstelle von Kupfer-II-Chlorid mit der gleichen Menge Uranylchlorid umgesetzt. Die Sublimation des entsprechend Beispiel 2 erhaltenen Rückstandes bei Temperaturen über 500 °C unter Argon ergibt den schwarzblauen Uranyl-Spheren-Komplex, der mit den Formeln 49) und 50) ausschnitthaft beschrieben wird. Danach besteht der Spherenkomplex, dem die Summenformel $C_{300}N_{60}U_{12}O_{24}$ zukommt, aus einem Arrangement von 12 Porphin-5-1/1/1/1/1-5N1$UO_2$-Funktionen, die symmetrisch in das Hexagonnetzwerk aus dreifach koordiniertem Kohlenstoff eingebettet sind. Der Spherus ist ikosaedersymmetrisch, wobei die Porphin-5-Funktionen jeweils die Ecken des Ikosaeders einnehmen, so daß jede Uranylfunktion eine Ikosaederecke bildet.

49) + 50)

49)

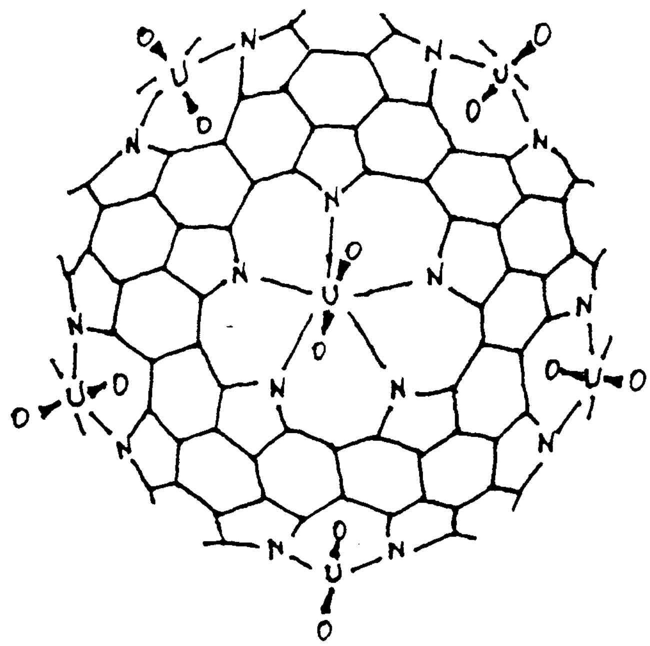

50)

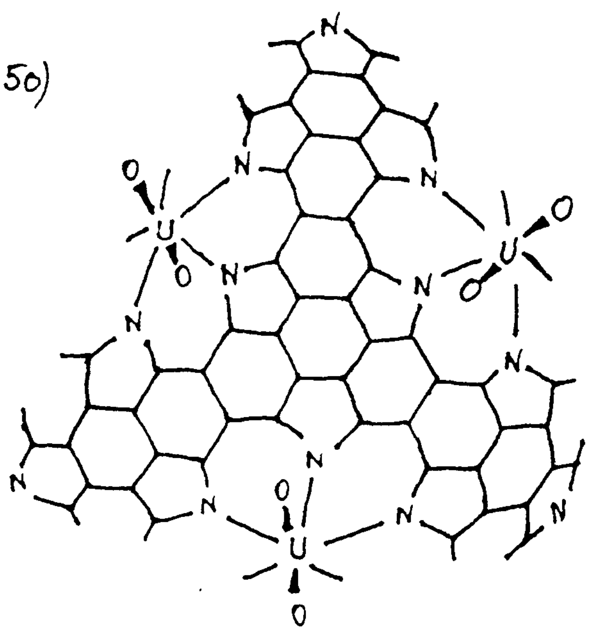

Beispiel 10

Entsprechend der in der DOS 4114536 gegebenen Vorschrift (Seiten 51-53) werden Spheren mit der Summenformel $C_{600}H_{180}N_{120}U_{12}O_{24}$ hergestellt, die als Fragmente des ikosaedersymmetrischen Fullerens $C_{980}$ angesehen werden können und die durch die in dem Formelausschnitt 51) gezeigte Struktur

gekennzeichnet sind. Die Spheren werden auf Kieselgel aufgezogen und unter Hindurchleiten von Stickstoff auf 450 °C erwärmt. Dann wird dem Stickstoff 5 Vol% Boran und 5 Vol% Methan beigemischt. Boran und Methan werden zuvor durch Glimmentladung angeregt. Nach 20 min. der Behandlung werden die spherenbeladenen Kieselgelteilchen abgekühlt, in Flußsäure aufgelöst, filtriert, der Filterrückstand mit Methanol gewaschen und getrocknet. Die nunmehr gewonnenen Spheren haben eine Struktur, die durch Formelausschnitt 52) gekennzeichnet ist. Sie haben die Summenformel $C_{620}H_{24}B_{60}N_{120}$. Durch eine 20 min. andauernde Wasserdampfbehandlung mit einem Gasgemisch aus 25 Vol% $H_20$ und 75 Vol% $N_2$ bei 950 °C der auf Kieselgel aufgezogenen Spheren und Luftbehandlung der danach auf 110 °C abgekühlten Spheren über Nacht werden die Öffnungen der Spheren erweitert. Nach der Separierung der Spheren durch Auflösen des Trägers, Wäsche und Trocknen des Rückstandes werden Spheren erhalten, die durch die Struktur gemäß Formelausschnitt 53) gekennzeichnet sind. Die Struktur 53) zeigt eine der 12 Öffnungen und ihre Umgebung. Die Öffnungen haben demnach die Struktur Porphin-5-3/3/3/3/3-50. Demnach haben die Spheren die Summenformel $C_{380}O_{120}$. Die Spheren lassen sich in der Gegenwart von Anionen leicht oxidieren und nehmen dann Strukturen gemäß Formelausschnitt 54) an.

51)

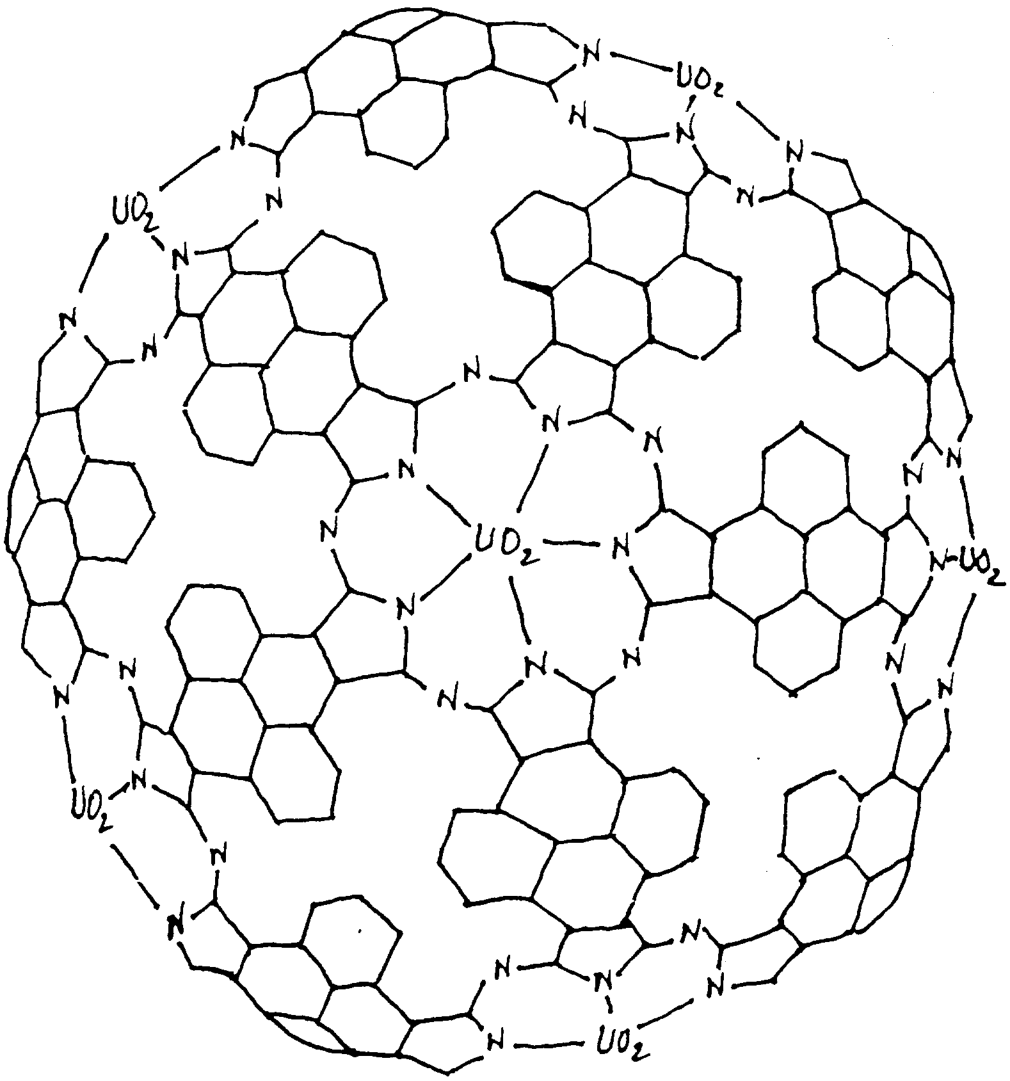

52)

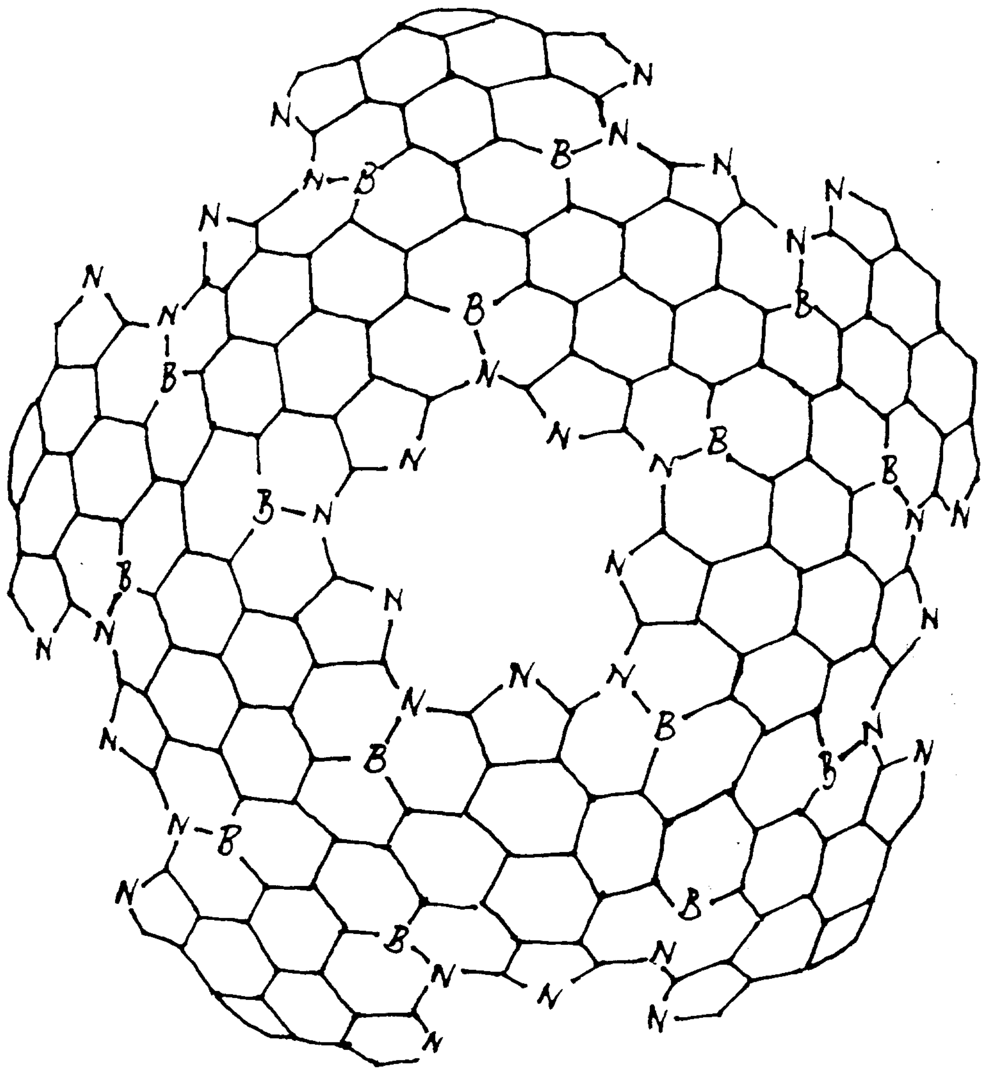

53) + 54)

53)

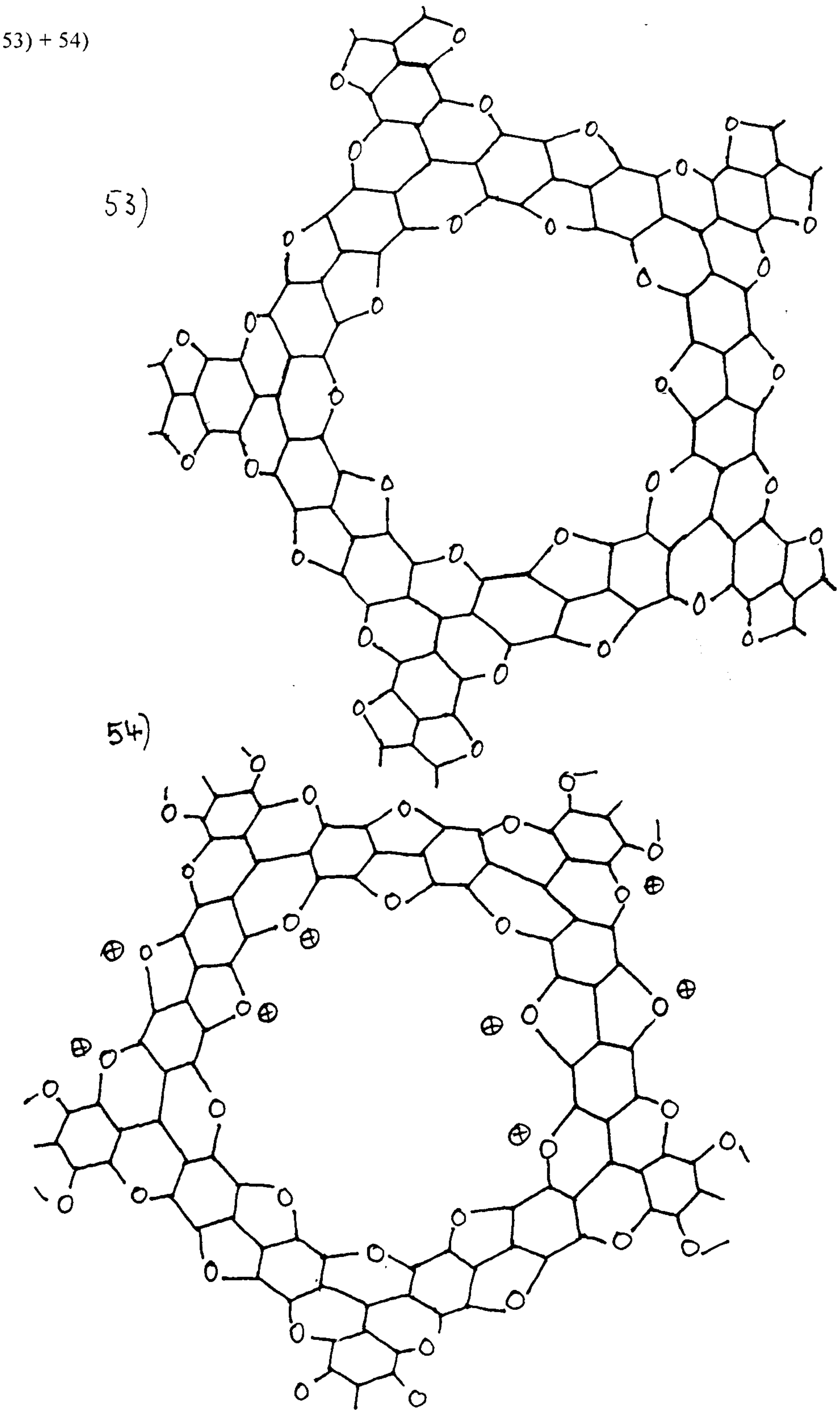

54)

## Patentansprüche

1. Spheren genannte Fulleren- und Heterofullerenfragmente, die dadurch gekennzeichnet sind, daß sie in ihrem Molekül mittels Hexagonnetzwerk anelliert verbundene Funktionen triangularer Symmetrie und/oder quadratischer Symmetrie und/oder pentagonaler Symmetrie entsprechend Formel 55) enthalten, worin den Symbolen die folgende Bedeutung zukommt:

R bedeutet Hexagonnetzwerk aus Kohlenstoff oder aus Kohlenstoff, Bor und Stickstoff oder aus Kohlenstoff und Stickstoff, das auch lückenlos mit Hexagonnetzwerk auffüllbare Ausnehmungen enthalten kann, und das $n_1$ plus $n_2$ plus n Funktionen Ö enthält.

Ö bedeutet eine Funktion in dem Hexagonnetzwerk des Spherus, die nicht lückenlos mit gleichseitigem Kohlenstoff-, Bor- und/oder Stickstoffhexagonnetzwerk ausgefüllt werden kann und die gegebenenfalls an den Hetero-und/ oder CH-Funktionen X und/oder an den Heterofunktionen Y koordiniert eine komplexierte Funktion enthalten kann;

X bedeutet O, $O^{\oplus}$, S, $S^{\oplus}$, Se, $Se^{\oplus}$, NH, N, $CH_2$, CH, CO als Brückenfunktion oder zwei getrennte Funktionen H H oder H O oder H OH oder OH OH oder eine $C_3$-Gruppe, die als Heptagon Bestandteil anellierten Hexagonnetzwerkes ist, wobei in diesem Fall Y ebenfalls anelliertes Hexagonnetzwerk bedeutet, das mit X anelliert ist;

Y hat die in den Formeln 56) bis 61) beschriebene Bedeutung, kann aber auch anelliertes Hexagonnetzwerk bedeuten, das auch mit X anelliert verbunden sein kann;

55)

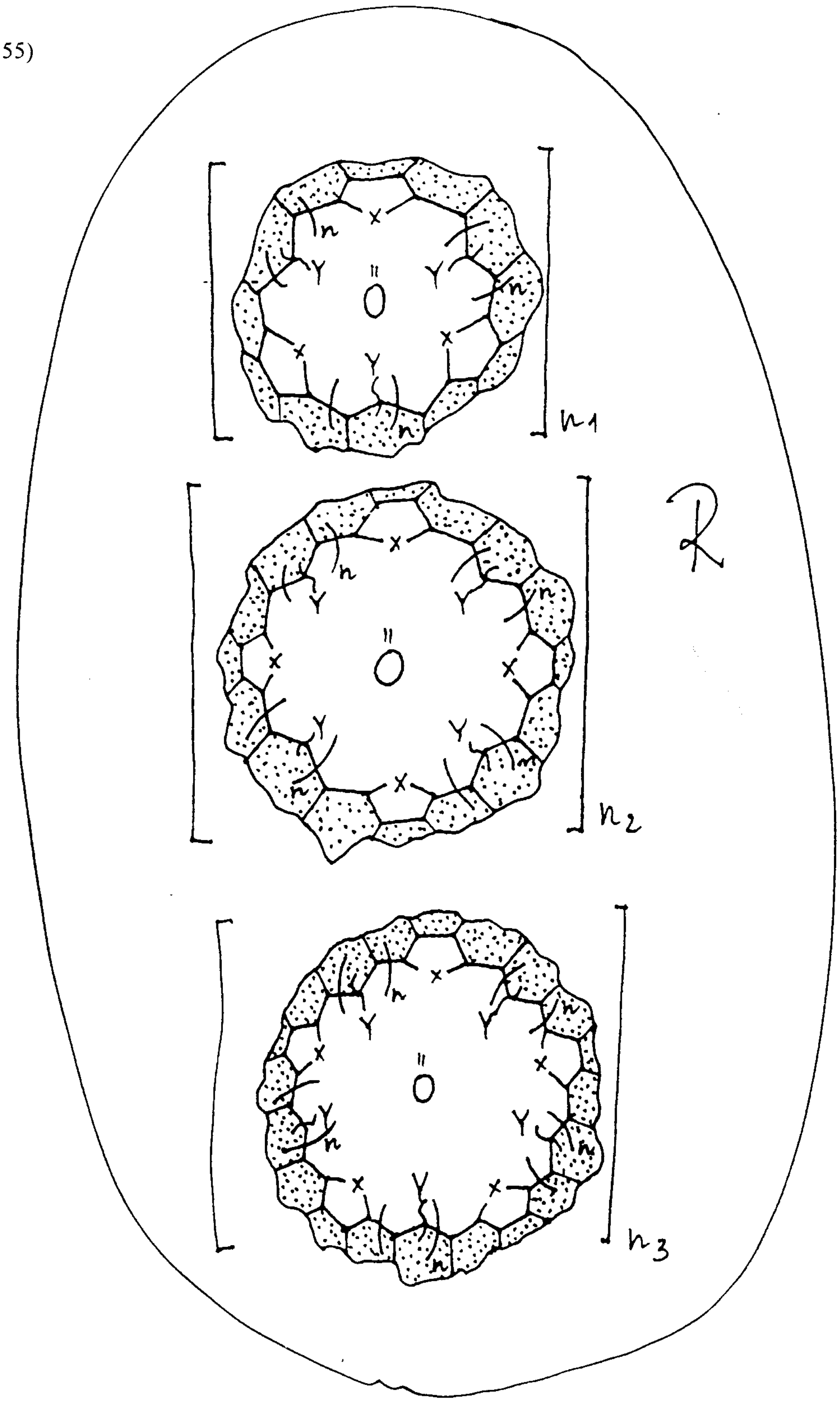

56) - 61)

56) 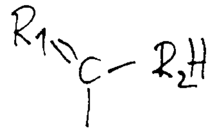

$R_1$ = O, Se, S, NH
$R_2$ = O, Se, S, NH

57) 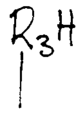

$R_3$ = O, S, Se, NH

58) 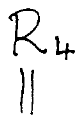

$R_4$ = O, S, Se, NH

59) 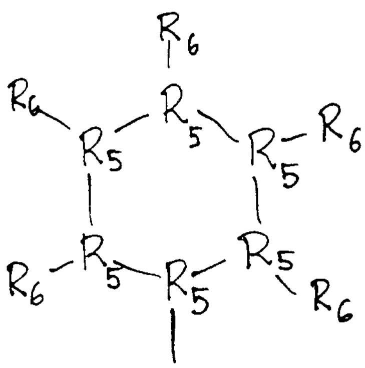

$R_5$ = C, N, B
$R_6$ = anelliertes Hexagonnetzwerk

60) 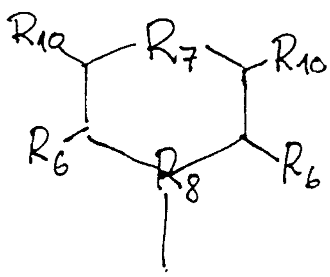

$R_7$ = NH, O, S, Se
$R_8$ = C, N, B
$R_{10}$ = H, =O

61) 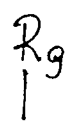

$R_9$ = H

| | |
|---|---|
| n | bedeutet Null oder eine ganze positive Zahl; |
| $n_1$ | bedeutet Null oder eine ganze positive Zahl kleiner gleich Vier; |
| $n_2$ | bedeutet Null oder eine ganze positive Zahl kleiner gleich Sechs; |
| $n_3$ | bedeutet Null oder eine ganze positive Zahl kleiner gleich Zwölf; |

und wobei die folgenden Konstellationen bei überwiegend 3-fach koordiniertem C und/oder B und/oder N ausgenommen sind:

- X gleich N oder NH bei n gleich Null und $n_1$ gleich 4 und $n_2$ plus $n_3$ gleich Null bei CBN-Heterofullerenen oder deren Fragmenten beschränkt auf die Spheren mit Tetraedersymmetrie gemäß den Fullerenen $C_{76}$, $C_{160}$, $C_{268}$, $C_{68}$, $C_{112}$, $c_{164}$, $C_{224}$ und $C_{292}$;
- X gleich N oder NH bei n gleich Null und $n_2$ gleich 6 und $n_1$ plus $n_3$ gleich Null beschränkt auf Spheren mit Oktaedersymmetrie gemäß den Fullerenen $C_{204}$, $C_{372}$, $C_{588}$, $C_{188}$, $c_{276}$, $C_{380}$, $C_{500}$ und $C_{636}$;
- X gleich N oder NH bei n gleich Null und $n_3$ gleich 12 und $n_1$ plus $n_2$ gleich Null beschränkt auf Spheren mit Ikosaedersymmetrie gemäß den Fullerenen $C_{540}$, $C_{960}$, $C_{1500}$, $C_{500}$, $C_{720}$, $C_{980}$, $C_{1280}$, $C_{1620}$.

**2.** Verfahren zur Herstellung von Spheren nach Anspruch 1, dadurch gekennzeichnet, daß diese aus Fullerenen und/oder Heterofullerenen und/oder Spheren hergestellt werden durch einen oder mehrere der Verfahrensschritte Oxidation, Konditionierung und Substitution.

**3.** Verfahren nach Anspruch 2, dadurch gekennzeichnet, daß die Oxidation durch sauerstoffhaltige Oxidationsmittel in fluiden Phasen verursacht wird.

**4.** Verfahren nach Anspruch 2 und 3, dadurch gekennzeichnet, daß zur Konditionierung Inertgase, stickstoffhaltige Gase, sauerstoffhaltige Gase, schwefelhaltige Gase, selenhaltige Gase, kohlenstoffhaltige Gase, borhaltige Gase, wasserstoffhaltige Gase oder Gemische davon angewendet werden.

**5.** Verfahren nach Anspruch 2 bis 4, dadurch gekennzeichnet, daß zur Substitution stickstoffhaltige Gase, schwefelhaltige Gase selenhaltige Gase, kohlenstoffhaltige Gase oder Gemische davon angewendet werden.

**6.** Verfahren nach Anspruch 2, dadurch gekennzeichnet, daß die Substitution als elektrophile oder nukleophile Substitution in der Gegenwart flüssiger, gasförmiger überkritisch fluider Phasen oder Gemischen davon vorgenommen wird.

**7.** Verfahren zur Anreicherung der für die Herstellung der Spheren nach Anspruch 1 geeigneten Fullerene oder Heterofullerene oder Spheren mit ausgewählten Symmetrieeigenschaften und/oder Molekülabmessungen aus Rohprodukten oder Gemischen, die eine Vielzahl unterschiedlicher Fullerene, Heterofullerene oder Spheren enthalten können, unter Anwendung von Spheren nach Anspruch 1 oder geeigneten sonstigen Spheren als Matrizen zur Herstellung von Hohlformen auf den Oberflächen von Partikeln oder anderen Formen, an denen die gewünschten Spheren oder Fullerene oder Heterofullerene chromatographisch angereichert werden können.

**8.** Verfahren zur Herstellung von zweidimensional und gegebenenfalls dreidimensional hochgeordneten einkristallinen Spherenfilmen aus Spheren oder Fullerenen oder Heterofullerenen mit Symmetrieeigenschaften und Abmessungen entsprechend Spheren nach Anspruch 1 durch Auftrag der molekulardispers auf polaren Flüssigkeiten, Wasser oder wäßrigen Lösungen gespreiteten Spheren, Fullerene oder Heteroffullerene oder deren Gemischen auf glatte Oberflächen.

**9.** Verfahren zur Durchführung von chemischen Reaktionen an Fullerenen, Heterofullerenen, Spheren oder Gemischen davon, dadurch gekennzeichnet, daß die Fullerene, Heterofullerene, Spheren oder deren Gemische in der adsorbierten Phase zur Reaktion gebracht werden.

**10.** Kommunizierende Spherenpolymere nach Anspruch 1 mit Verbindungselementen zwischen den Spheren aus Hexagonnetzwerk rohrförmiger Topologie derart, daß die von den miteinander verbundenen Spheren umschlossenen Hohlräume miteinander kommunizieren.